# EUROPEAN PATENT SPECIFICATION

(11) **EP 2 354 248 B1**
(45) Date of publication and mention of the grant of the patent: **03.01.2018**
(21) Application number: 10174462.1
(22) Date of filing: 23.06.2004
(51) Int. Cl.: C12Q 1/68, C07H 21/00

(54) **Methods for analyses of colorectal cell proliferative disorders**
Verfahren zur Analyse von Störungen der Proliferation kolorektaler Zellen
Procédés d'analyse des troubles de prolifération de cellules colorectales

(30) Priority: 23.06.2003 US 602494; 23.06.2003 US 603138; 06.05.2004 EP 04090175; 27.02.2004 EP 04090072; 03.10.2003 US 679062
(43) Date of publication of application: 10.08.2011
(62) Divisional of application: 04777046.6
(73) Proprietor: Epigenomics AG, 10829 Berlin (DE)
(72) Inventor: Lofton-Day, Cathy, Seattle, WA 98103 (US); Model, Fabian, 12683 Berlin (DE); Sledziewski, Andrew, Shoreline, WA 98177 (US); Rujan, Tamas, 79104 Freiburg (DE); Lewin, Jörn, 10115 Berlin (DE); Distler, Jürgen, 12163 Berlin (DE)
(74) Representative: Zwicker, Jörk

(56) References cited:
- WO-A-02/24056
- WO-A-03/014388
- DATABASE Geneseq [Online] 12 July 2002 (2002-07-12), "Oligonucleotide for detecting cytosine methylation SEQ ID NO 4217.", XP002638000, retrieved from EBI accession no. GSN:ABQ17626 Database accession no. ABQ17626 -& WO 02/18632 A2 (EPIGENOMICS AG [DE]; OLEK ALEXANDER [DE]; PIEPENBROCK CHRISTIAN [DE];) 7 March 2002 (2002-03-07)
- DATABASE Geneseq [Online] 16 February 2001 (2001-02-16), "Hammerhead ribozyme substrate no.2726", XP002638234, retrieved from EBI accession no. GSN:AAF05507 Database accession no. AAF05507
- TOYOTA M ET AL: "CpG island methylator phenotype in colorectal cancer", PROCEEDINGS OF THE NATIONAL ACADEMY OF SCIENCES OF USA, NATIONAL ACADEMY OF SCIENCE. WASHINGTON, US, vol. 96, July 1999 (1999-07), pages 8681-8686, XP002307651, ISSN: 0027-8424
- RASHID ASIF ET AL: "CpG island methylation in colorectal adenomas", AMERICAN JOURNAL OF PATHOLOGY, vol. 159, no. 3, September 2001 (2001-09), pages 1129-1135, XP002328021, ISSN: 0002-9440
- VAN RIJNSOEVER M ET AL: "Characterisation of colorectal cancers showing hypermethylation at multiple CpG islands.", GUT, vol. 51, no. 6, December 2002 (2002-12), pages 797-802, XP002328020, ISSN: 0017-5749
- RAKOWICZ-SZULCZYNSKA E ET AL: "Gamma-interferon-induced nerve growth factor receptors in colorectal carcinoma cell lines", GROWTH FACTORS, HARWOOD ACADEMIC PUBLISHERS GMBH, XX, vol. 6, no. 4, 1 January 1992 (1992-01-01) , pages 337-346, XP009148469, ISSN: 0897-7194
- LOFTON-DAY CATHERINE ET AL: "DNA methylation biomarkers for blood-based colorectal cancer screening", CLINICAL CHEMISTRY, AMERICAN ASSOCIATION FOR CLINICAL CHEMISTRY, WASHINGTON, DC, vol. 54, no. 2, 1 February 2008 (2008-02-01), pages 414-423, XP009107287, ISSN: 0009-9147, DOI: DOI:10.1373/CLINCHEM.2007.095992

### Sequence listing

<110> Epigenomics AG; Lofton-Day, Cathy; Model, Fabian; Sledziewski,
   Andrew; Rujan, Tamas; Lewin, Jorn; Distler, Juergen
<120> Methods and nucleic acids for the analysis of colon cell proliferative disorders
<160> 14624
<210> 1 to <210> 6
   000
<210> 7
   <211> 3952
   <212> DNA
   <213> Homo Sapiens
<400> 7
   ggccgcagcc ggagaggaac aggaaccgca gtggggacgc cctggtcccc gggcccacag      60
   catccgggac ggacgcgcag tagcgcgggc cgggaactgg gtaccagggc gggatgggtg     120
   agaggctcta agggacaagg cagggagaag cgcagcgggg tgcggggaac cgcacgccct     180
   ccctttgcct ctgcttccca ccccgaggcg gcagggcggg cgggcgcggt tccgggggtg     240
   ggcgggctgg gcggggcgga ggcggggccg cagcactggc ttcacccagc ctctcccgcc     300
   cgcagccaga gcgagccgag ccgcggccag ctccggcggg cagggggggc gctggagcgc      360
   agcgcagcgc agccccatca gtccgcaaag cggaccgagc tggaagtcga gcgctgccgc     420
   gggaggcggg cgatgggggc aggtgccacc ggccgcgcca tggacgggcc gcgcctgctg      480
   ctgttgctgc ttctgggggt gagtgttagc cggagggggc ccgctccctt tcccgggatc      540
   agaactccga gaagagccgg gcgccgccac caaggaaaca gaacagagca ttggggtccc     600
   agatactgag ggtgggtggt gggaaaggac ctctgatgcc gggaccacga aggagggtct     660
   agggttcccg gagcgcagag gcgactctcc agggtggaga tgagggcaag accggagcac     720
   ggatgccggt cctcaggtac cgcagggggc ggtgggggag ctgggagggg tctttcaaga     780
   gggggcatgg ggctctccga tgcccaggtt cttcggaaga ggacactcga atgccgggat     840
   cccgaaggga ctttcccctc agcatctcgg tctctggaga gtcgtgggac agaaccaagg     900
   cgagagaagg agggggaact ggacggggac tagagatgag aggggcgagc tgggctgggg      960
   cggggagccc gggacgacgg gatggaacaa tggaggaggt cggagggact ggaggcagag    1020
   gggacccggc aaggacaatg gggaccggcc agacaatggg ggggaggggc gtcggaggga    1080
   gaggttgggg agcgggagag aggttgagaa gtgggttagg gagggcagaa gaggggcgac    1140
   ggcgagggcg gcagagagat ggaagcagaa ggaaaggaga cagagcgggg cgcggaccct    1200
   gaagtggtcg gggtcccgcc tgcccgctga gggacagtga gacgagaaga gagagcagag    1260
   gcttggacat ttccagcgcc tgtccccgcg ctcctgctcg cggcctctcc cagtcccgga    1320
   attggggctg cggatctaag gctgggaggg gagtgcccac ttcgccgggg cgaacccgct    1380
   cccggggttc ccccacggcc ggcgggctcc tccgacgcgc gtgttctcac ggcttgaagc    1440
   agcgcagcgg atcgcgaggg acggagcggg tgtgccccta cccgggctgg cggtggctac    1500
   gtctcggggt gcagagcgcc ggggcgcact aggcggggac ccttcctggc cgcgcgcagc    1560
   tctggctacg caggcgggag gcggcaggca gggggcgctg cggggcgagg caagtgcagt    1620
   ccgccgctgc ctgcctgcga gtctggggct gcgcgccgct ccccacctag ctgcgcgacc    1680
   cgagcgccgc gcccaccgcg ctccgtgggc tcccgcgcct cgaagggtcg ggatccggtc    1740
   tgagcctctc ctggctctcg gtgcaagccg ctgggcacct aacgagacgg gggcgcccgg    1800
   acgcctcgga ttctgtagcg cggtatcccg ggccaccgac tcggcaaagg tcggcgggtc    1860
   ctccgggtgg tccgacgaga ttgtcagatg tggcctgctt tatggccatg aaccgaatgc    1920
   cctggaaggt ctggcaacgg aggaggcgtc ttcactctcc ccgccagcat ctttcatcag    1980
   agcactgcgc cccaaattcc ggctttacag atacggagac tgaggctctg agagtttaaa    2040
   cgacttggcc aagatctcac agcaagttgg gccagagtcg tggctagact ccccgacctg    2100
   attcccagct cggtactccc ccaccttttc agggggcagc cgctgtccca gctcggggtc      2160
   acggctgcgt ttcccagcag ggcggggggg gttgacactg tccccagagt ccgtcggcgg     2220
   tagagcctgc gggtgaggtg ggcagagatt ctgtccgggt gcgggcccca gcccgaaagc    2280
   tcagccccag ttccctggag ctgtcaggac ctgtggctgc gtccggggat ttgggagcga    2340
   ccgcgggcac gtctgctgac taacgccgct ggttagagac gctgctcaga cgcgggtggg    2400
   cgagcgtaga ccaggagagc ggggaggagg gaccggatcc cagaggggca acgacttggg    2460
   ctccgggtct gggcaagagc gagggtcccc gcgcgaagac ccaaggaagg aggggagctg    2520
   ggcgtggagg acctgggcgc ttgggaaggt gtaccccgac aggaacctgc cgtctgcagc    2580
   ccctgcggct cccgcctccg cctcggcgcg cggctgctcg gtctgggatg gctgtgcgcg    2640
   gtccgagttt cttcctgggc cgcggtgaga gagagagggc tgaaaccaga gcgcgtcccg    2700
   gcgggtcggc tggcgggccg cgccggtaat ggaggcactt tgtcattcag acgtctgtaa    2760
   ccagagccgc cgggctggct aatgcgccta atagggatgg aacgagggca gcaaatgggc    2820
   gtgcgtgagc ggccgggctg agctgggtgg atggtggatg gggaggcagc tccgcggggg    2880
   acgggcccct accgcactgt tccagcccag tctggctcaa gcgcctcgct tcttccctgg    2940
   gggaccgcgt ggggaggcgg gaataatcga tgtgctgagt tcctactaag tgccttgcac    3000
   ttccctggta gtttctcttg aattatttaa tccttataag gtcctagcaa ggtgaattta    3060
   gttattccca cttcgcagtt gaggaaacag aaccttctag actttcaggc ccgagaccag    3120
   gctcccccag tgagtcagtg gtggcgtctt tgttggaacc caggatgctt ggcctgggat    3180
   gttctctttc ctacatatgc caggctccct cccgccccct accttctaga tcatggaagg    3240
   agtcgtggct ctgagagatt aaatcgtgcc ctatgccttt ggccccacac atttagatct    3300
   gagccccttc tccacacgca gaggccctgc cccctctcaa gtctctatgc tacattatct    3360
   tgggtaatta gctcctttcc tcccaactcc tcccctatcc aaagggagtg aggggtatgt    3420
   gccagtgcct cccctgggtt tgcatcgacc tcaaaaggcc ctccaggtgt ttgaaaatct    3480
   ctcccatccc atggcggtga ccacggcccc attcacttaa gagctctttg ctgaagctgt    3540
   tggaatggcc agggctgatt atactgaaac attcgtatgg tttcaaatac tttcaactgt    3600
   ttcgctgttt ctctcttttc tctaattaaa aaaaatgaaa attcttaaaa gtcaattcat    3660
   ctaaggatcc ttccacctta gttccttgta agccttgaaa tggccaaaac aaaaagatta    3720
   caagcgttct taaaaatgag atgaaatcat tctgtcaaat gtgaaatgac taagtgtaac    3780
   acatacaaat ttcagggaaa tttatctgtc atccctataa attaatcctt ttggatctga    3840
   ttttggagca attagcctga atgttagtca cccctcccta cttggctaaa ctctcccctc    3900
   ctgcctgggg tctccttaag atgagatgct tctttgataa ctaacattta tt            3952
<210> 8 to <210> 315
   000
<210> 316
   <211> 3952
   <212> DNA
   <213> Artificial Sequence
<220>
   <223> chemically treated genomic DNA (Homo sapiens)
<400> 316
   ggtcgtagtc ggagaggaat aggaatcgta gtggggacgt tttggttttc gggtttatag      60
   tattcgggac ggacgcgtag tagcgcgggt cgggaattgg gtattagggc gggatgggtg     120
   agaggtttta agggataagg tagggagaag cgtagcgggg tgcggggaat cgtacgtttt     180
   ttttttgttt ttgtttttta tttcgaggcg gtagggcggg cgggcgcggt ttcgggggtg     240
   ggcgggttgg gcggggcgga ggcggggtcg tagtattggt tttatttagt ttttttcgtt     300
   cgtagttaga gcgagtcgag tcgcggttag tttcggcggg tagggggggc gttggagcgt     360
   agcgtagcgt agttttatta gttcgtaaag cggatcgagt tggaagtcga gcgttgtcgc     420
   gggaggcggg cgatgggggt aggtgttatc ggtcgcgtta tggacgggtc gcgtttgttg     480
   ttgttgttgt ttttgggggt gagtgttagt cggagggggt tcgttttttt tttcgggatt     540
   agaatttcga gaagagtcgg gcgtcgttat taaggaaata gaatagagta ttggggtttt     600
   agatattgag ggtgggtggt gggaaaggat ttttgatgtc gggattacga aggagggttt     660
   agggttttcg gagcgtagag gcgatttttt agggtggaga tgagggtaag atcggagtac     720
   ggatgtcggt ttttaggtat cgtagggggc ggtgggggag ttgggagggg ttttttaaga     780
   gggggtatgg ggtttttcga tgtttaggtt tttcggaaga ggatattcga atgtcgggat     840
   ttcgaaggga tttttttttt agtatttcgg tttttggaga gtcgtgggat agaattaagg     900
   cgagagaagg agggggaatt ggacggggat tagagatgag aggggcgagt tgggttgggg     960
   cggggagttc gggacgacgg gatggaataa tggaggaggt cggagggatt ggaggtagag    1020
   gggattcggt aaggataatg gggatcggtt agataatggg ggggaggggc gtcggaggga    1080
   gaggttgggg agcgggagag aggttgagaa gtgggttagg gagggtagaa gaggggcgac    1140
   ggcgagggcg gtagagagat ggaagtagaa ggaaaggaga tagagcgggg cgcggatttt    1200
   gaagtggtcg gggtttcgtt tgttcgttga gggatagtga gacgagaaga gagagtagag    1260
   gtttggatat ttttagcgtt tgttttcgcg tttttgttcg cggttttttt tagtttcgga    1320
   attggggttg cggatttaag gttgggaggg gagtgtttat ttcgtcgggg cgaattcgtt    1380
   ttcggggttt ttttacggtc ggcgggtttt ttcgacgcgc gtgtttttac ggtttgaagt    1440
   agcgtagcgg atcgcgaggg acggagcggg tgtgttttta ttcgggttgg cggtggttac    1500
   gtttcggggt gtagagcgtc ggggcgtatt aggcggggat tttttttggt cgcgcgtagt    1560
   tttggttacg taggcgggag gcggtaggta gggggcgttg cggggcgagg taagtgtagt    1620
   tcgtcgttgt ttgtttgcga gtttggggtt gcgcgtcgtt ttttatttag ttgcgcgatt    1680
   cgagcgtcgc gtttatcgcg tttcgtgggt tttcgcgttt cgaagggtcg ggattcggtt    1740
   tgagtttttt ttggttttcg gtgtaagtcg ttgggtattt aacgagacgg gggcgttcgg    1800
   acgtttcgga ttttgtagcg cggtatttcg ggttatcgat tcggtaaagg tcggcgggtt    1860
   tttcgggtgg ttcgacgaga ttgttagatg tggtttgttt tatggttatg aatcgaatgt    1920
   tttggaaggt ttggtaacgg aggaggcgtt tttatttttt tcgttagtat tttttattag    1980
   agtattgcgt tttaaatttc ggttttatag atacggagat tgaggttttg agagtttaaa    2040
   cgatttggtt aagattttat agtaagttgg gttagagtcg tggttagatt tttcgatttg    2100
   atttttagtt cggtattttt ttattttttt agggggtagt cgttgtttta gttcggggtt    2160
   acggttgcgt tttttagtag ggcggggggg gttgatattg tttttagagt tcgtcggcgg    2220
   tagagtttgc gggtgaggtg ggtagagatt ttgttcgggt gcgggtttta gttcgaaagt    2280
   ttagttttag ttttttggag ttgttaggat ttgtggttgc gttcggggat ttgggagcga    2340
   tcgcgggtac gtttgttgat taacgtcgtt ggttagagac gttgtttaga cgcgggtggg    2400
   cgagcgtaga ttaggagagc ggggaggagg gatcggattt tagaggggta acgatttggg    2460
   tttcgggttt gggtaagagc gagggttttc gcgcgaagat ttaaggaagg aggggagttg    2520
   ggcgtggagg atttgggcgt ttgggaaggt gtatttcgat aggaatttgt cgtttgtagt    2580
   ttttgcggtt ttcgttttcg tttcggcgcg cggttgttcg gtttgggatg gttgtgcgcg    2640
   gttcgagttt ttttttgggt cgcggtgaga gagagagggt tgaaattaga gcgcgtttcg    2700
   gcgggtcggt tggcgggtcg cgtcggtaat ggaggtattt tgttatttag acgtttgtaa    2760
   ttagagtcgt cgggttggtt aatgcgttta atagggatgg aacgagggta gtaaatgggc    2820
   gtgcgtgagc ggtcgggttg agttgggtgg atggtggatg gggaggtagt ttcgcggggg    2880
   acgggttttt atcgtattgt tttagtttag tttggtttaa gcgtttcgtt ttttttttgg   2940
   gggatcgcgt ggggaggcgg gaataatcga tgtgttgagt ttttattaag tgttttgtat    3000
   ttttttggta gttttttttg aattatttaa tttttataag gttttagtaa ggtgaattta  3060
   gttattttta tttcgtagtt gaggaaatag aattttttag atttttaggt tcgagattag   3120
   gtttttttag tgagttagtg gtggcgtttt tgttggaatt taggatgttt ggtttgggat    3180
   gttttttttt ttatatatgt taggtttttt ttcgtttttt attttttaga ttatggaagg   3240
   agtcgtggtt ttgagagatt aaatcgtgtt ttatgttttt ggttttatat atttagattt    3300
   gagttttttt tttatacgta gaggttttgt ttttttttaa gtttttatgt tatattattt   3360
   tgggtaatta gttttttttt ttttaatttt ttttttattt aaagggagtg aggggtatgt    3420
   gttagtgttt tttttgggtt tgtatcgatt ttaaaaggtt ttttaggtgt ttgaaaattt    3480
   tttttatttt atggcggtga ttacggtttt atttatttaa gagttttttg ttgaagttgt   3540
   tggaatggtt agggttgatt atattgaaat attcgtatgg ttttaaatat ttttaattgt    3600
   ttcgttgttt tttttttttt tttaattaaa aaaaatgaaa atttttaaaa gttaatttat    3660
   ttaaggattt ttttatttta gttttttgta agttttgaaa tggttaaaat aaaaagatta  3720
   taagcgtttt taaaaatgag atgaaattat tttgttaaat gtgaaatgat taagtgtaat    3780
   atatataaat tttagggaaa tttatttgtt atttttataa attaattttt ttggatttga    3840
   ttttggagta attagtttga atgttagtta ttttttttta tttggttaaa tttttttttt   3900
   ttgtttgggg tttttttaag atgagatgtt tttttgataa ttaatattta tt           3952
<210> 317
   <211> 3952
   <212> DNA
   <213> Artificial Sequence
<220>
   <223> chemically treated genomic DNA (Homo sapiens)
<400> 317
   aataaatgtt agttattaaa gaagtatttt attttaagga gattttaggt aggaggggag      60
   agtttagtta agtagggagg ggtgattaat atttaggtta attgttttaa aattagattt     120
   aaaaggatta atttataggg atgatagata aatttttttg aaatttgtat gtgttatatt     180
   tagttatttt atatttgata gaatgatttt attttatttt taagaacgtt tgtaattttt     240
   ttgttttggt tattttaagg tttataagga attaaggtgg aaggattttt agatgaattg     300
   atttttaaga atttttattt tttttaatta gagaaaagag agaaatagcg aaatagttga     360
   aagtatttga aattatacga atgttttagt ataattagtt ttggttattt taatagtttt     420
   agtaaagagt ttttaagtga atggggtcgt ggttatcgtt atgggatggg agagattttt     480
   aaatatttgg agggtttttt gaggtcgatg taaatttagg ggaggtattg gtatatattt     540
   tttatttttt ttggataggg gaggagttgg gaggaaagga gttaattatt taagataatg     600
   tagtatagag atttgagagg gggtagggtt tttgcgtgtg gagaaggggt ttagatttaa     660
   atgtgtgggg ttaaaggtat agggtacgat ttaatttttt agagttacga ttttttttat     720
   gatttagaag gtagggggcg ggagggagtt tggtatatgt aggaaagaga atattttagg     780
   ttaagtattt tgggttttaa taaagacgtt attattgatt tattggggga gtttggtttc     840
   gggtttgaaa gtttagaagg ttttgttttt ttaattgcga agtgggaata attaaattta     900
   ttttgttagg attttataag gattaaataa tttaagagaa attattaggg aagtgtaagg     960
   tatttagtag gaatttagta tatcgattat tttcgttttt ttacgcggtt ttttagggaa    1020
   gaagcgaggc gtttgagtta gattgggttg gaatagtgcg gtaggggttc gtttttcgcg    1080
   gagttgtttt tttatttatt atttatttag tttagttcgg tcgtttacgt acgtttattt    1140
   gttgttttcg ttttattttt attaggcgta ttagttagtt cggcggtttt ggttatagac    1200
   gtttgaatga taaagtgttt ttattatcgg cgcggttcgt tagtcgattc gtcgggacgc    1260
   gttttggttt tagttttttt ttttttatcg cggtttagga agaaattcgg atcgcgtata    1320
   gttattttag atcgagtagt cgcgcgtcga ggcggaggcg ggagtcgtag gggttgtaga    1380
   cggtaggttt ttgtcggggt atattttttt aagcgtttag gttttttacg tttagttttt    1440
   tttttttttt gggttttcgc gcggggattt tcgtttttgt ttagattcgg agtttaagtc    1500
   gttgtttttt tgggattcgg tttttttttt tcgttttttt ggtttacgtt cgtttattcg    1560
   cgtttgagta gcgtttttaa ttagcggcgt tagttagtag acgtgttcgc ggtcgttttt    1620
   aaattttcgg acgtagttat aggttttgat agttttaggg aattggggtt gagttttcgg    1680
   gttggggttc gtattcggat agaatttttg tttattttat tcgtaggttt tatcgtcgac    1740
   ggattttggg gatagtgtta atttttttcg ttttgttggg aaacgtagtc gtgatttcga    1800
   gttgggatag cggttgtttt ttgaaaaggt gggggagtat cgagttggga attaggtcgg    1860
   ggagtttagt tacgattttg gtttaatttg ttgtgagatt ttggttaagt cgtttaaatt    1920
   tttagagttt tagttttcgt atttgtaaag tcggaatttg gggcgtagtg ttttgatgaa    1980
   agatgttggc ggggagagtg aagacgtttt tttcgttgtt agatttttta gggtattcgg    2040
   tttatggtta taaagtaggt tatatttgat aatttcgtcg gattattcgg aggattcgtc    2100
   gatttttgtc gagtcggtgg ttcgggatat cgcgttatag aattcgaggc gttcgggcgt    2160
   tttcgtttcg ttaggtgttt agcggtttgt atcgagagtt aggagaggtt tagatcggat    2220
   ttcgattttt cgaggcgcgg gagtttacgg agcgcggtgg gcgcggcgtt cgggtcgcgt    2280
   agttaggtgg ggagcggcgc gtagttttag attcgtaggt aggtagcggc ggattgtatt    2340
   tgtttcgttt cgtagcgttt tttgtttgtc gtttttcgtt tgcgtagtta gagttgcgcg    2400
   cggttaggaa gggttttcgt ttagtgcgtt tcggcgtttt gtatttcgag acgtagttat    2460
   cgttagttcg ggtaggggta tattcgtttc gtttttcgcg attcgttgcg ttgttttaag    2520
   tcgtgagaat acgcgcgtcg gaggagttcg tcggtcgtgg gggaatttcg ggagcgggtt    2580
   cgtttcggcg aagtgggtat ttttttttta gttttagatt cgtagtttta atttcgggat    2640
   tgggagaggt cgcgagtagg agcgcgggga taggcgttgg aaatgtttaa gtttttgttt    2700
   ttttttttcg ttttattgtt ttttagcggg taggcgggat ttcgattatt ttagggttcg    2760
   cgtttcgttt tgtttttttt ttttttgttt ttattttttt gtcgttttcg tcgtcgtttt    2820
   ttttttgttt tttttaattt attttttaat ttttttttcg ttttttaatt ttttttttcg    2880
   acgttttttt tttttattgt ttggtcggtt tttattgttt ttgtcgggtt ttttttgttt    2940
   ttagtttttt cgattttttt tattgtttta tttcgtcgtt tcgggttttt cgttttagtt    3000
   tagttcgttt tttttatttt tagttttcgt ttagtttttt tttttttttt cgttttggtt    3060
   ttgttttacg attttttaga gatcgagatg ttgaggggaa agttttttcg ggatttcggt    3120
   attcgagtgt tttttttcga agaatttggg tatcggagag ttttatgttt ttttttgaaa    3180
   gatttttttt agttttttta tcgttttttg cggtatttga ggatcggtat tcgtgtttcg    3240
   gttttgtttt tatttttatt ttggagagtc gtttttgcgt ttcgggaatt ttagattttt    3300
   tttcgtggtt tcggtattag aggttttttt ttattattta tttttagtat ttgggatttt    3360
   aatgttttgt tttgtttttt tggtggcggc gttcggtttt tttcggagtt ttgatttcgg    3420
   gaaagggagc gggttttttt cggttaatat ttatttttag aagtagtaat agtagtaggc    3480
   gcggttcgtt tatggcgcgg tcggtggtat ttgtttttat cgttcgtttt tcgcggtagc    3540
   gttcgatttt tagttcggtt cgttttgcgg attgatgggg ttgcgttgcg ttgcgtttta    3600
   gcgttttttt tgttcgtcgg agttggtcgc ggttcggttc gttttggttg cgggcgggag    3660
   aggttgggtg aagttagtgt tgcggtttcg ttttcgtttc gtttagttcg tttattttcg    3720
   gaatcgcgtt cgttcgtttt gtcgtttcgg ggtgggaagt agaggtaaag ggagggcgtg    3780
   cggtttttcg tatttcgttg cgtttttttt tgttttgttt tttagagttt tttatttatt    3840
   tcgttttggt atttagtttt cggttcgcgt tattgcgcgt tcgtttcgga tgttgtgggt    3900
   tcggggatta gggcgttttt attgcggttt ttgttttttt tcggttgcgg tt            3952
<210> 318 to <210> 431
   000
<210> 432
   <211> 3952
   <212> DNA
   <213> Artificial Sequence
<220>
   <223> chemically treated genomic DNA (Homo sapiens)
<400> 432
   ggttgtagtt ggagaggaat aggaattgta gtggggatgt tttggttttt gggtttatag      60
   tatttgggat ggatgtgtag tagtgtgggt tgggaattgg gtattagggt gggatgggtg     120
   agaggtttta agggataagg tagggagaag tgtagtgggg tgtggggaat tgtatgtttt     180
   ttttttgttt ttgtttttta ttttgaggtg gtagggtggg tgggtgtggt tttgggggtg     240
   ggtgggttgg gtggggtgga ggtggggttg tagtattggt tttatttagt tttttttgtt     300
   tgtagttaga gtgagttgag ttgtggttag ttttggtggg tagggggggt gttggagtgt      360
   agtgtagtgt agttttatta gtttgtaaag tggattgagt tggaagttga gtgttgttgt      420
   gggaggtggg tgatgggggt aggtgttatt ggttgtgtta tggatgggtt gtgtttgttg      480
   ttgttgttgt ttttgggggt gagtgttagt tggagggggt ttgttttttt ttttgggatt      540
   agaattttga gaagagttgg gtgttgttat taaggaaata gaatagagta ttggggtttt      600
   agatattgag ggtgggtggt gggaaaggat ttttgatgtt gggattatga aggagggttt      660
   agggtttttg gagtgtagag gtgatttttt agggtggaga tgagggtaag attggagtat     720
   ggatgttggt ttttaggtat tgtagggggt ggtgggggag ttgggagggg ttttttaaga      780
   gggggtatgg ggttttttga tgtttaggtt ttttggaaga ggatatttga atgttgggat     840
   tttgaaggga tttttttttt agtattttgg tttttggaga gttgtgggat agaattaagg      900
   tgagagaagg agggggaatt ggatggggat tagagatgag aggggtgagt tgggttgggg      960
   tggggagttt gggatgatgg gatggaataa tggaggaggt tggagggatt ggaggtagag     1020
   gggatttggt aaggataatg gggattggtt agataatggg ggggaggggt gttggaggga     1080
   gaggttgggg agtgggagag aggttgagaa gtgggttagg gagggtagaa gaggggtgat     1140
   ggtgagggtg gtagagagat ggaagtagaa ggaaaggaga tagagtgggg tgtggatttt     1200
   gaagtggttg gggttttgtt tgtttgttga gggatagtga gatgagaaga gagagtagag     1260
   gtttggatat ttttagtgtt tgtttttgtg tttttgtttg tggttttttt tagttttgga     1320
   attggggttg tggatttaag gttgggaggg gagtgtttat tttgttgggg tgaatttgtt     1380
   tttggggttt ttttatggtt ggtgggtttt tttgatgtgt gtgtttttat ggtttgaagt     1440
   agtgtagtgg attgtgaggg atggagtggg tgtgttttta tttgggttgg tggtggttat     1500
   gttttggggt gtagagtgtt ggggtgtatt aggtggggat tttttttggt tgtgtgtagt      1560
   tttggttatg taggtgggag gtggtaggta gggggtgttg tggggtgagg taagtgtagt      1620
   ttgttgttgt ttgtttgtga gtttggggtt gtgtgttgtt ttttatttag ttgtgtgatt     1680
   tgagtgttgt gtttattgtg ttttgtgggt ttttgtgttt tgaagggttg ggatttggtt     1740
   tgagtttttt ttggtttttg gtgtaagttg ttgggtattt aatgagatgg gggtgtttgg    1800
   atgttttgga ttttgtagtg tggtattttg ggttattgat ttggtaaagg ttggtgggtt     1860
   ttttgggtgg tttgatgaga ttgttagatg tggtttgttt tatggttatg aattgaatgt     1920
   tttggaaggt ttggtaatgg aggaggtgtt tttatttttt ttgttagtat tttttattag    1980
   agtattgtgt tttaaatttt ggttttatag atatggagat tgaggttttg agagtttaaa     2040
   tgatttggtt aagattttat agtaagttgg gttagagttg tggttagatt ttttgatttg     2100
   atttttagtt tggtattttt ttattttttt agggggtagt tgttgtttta gtttggggtt     2160
   atggttgtgt tttttagtag ggtggggggg gttgatattg tttttagagt ttgttggtgg     2220
   tagagtttgt gggtgaggtg ggtagagatt ttgtttgggt gtgggtttta gtttgaaagt     2280
   ttagttttag ttttttggag ttgttaggat ttgtggttgt gtttggggat ttgggagtga    2340
   ttgtgggtat gtttgttgat taatgttgtt ggttagagat gttgtttaga tgtgggtggg     2400
   tgagtgtaga ttaggagagt ggggaggagg gattggattt tagaggggta atgatttggg     2460
   ttttgggttt gggtaagagt gagggttttt gtgtgaagat ttaaggaagg aggggagttg     2520
   ggtgtggagg atttgggtgt ttgggaaggt gtattttgat aggaatttgt tgtttgtagt     2580
   ttttgtggtt tttgtttttg ttttggtgtg tggttgtttg gtttgggatg gttgtgtgtg     2640
   gtttgagttt ttttttgggt tgtggtgaga gagagagggt tgaaattaga gtgtgttttg     2700
   gtgggttggt tggtgggttg tgttggtaat ggaggtattt tgttatttag atgtttgtaa     2760
   ttagagttgt tgggttggtt aatgtgttta atagggatgg aatgagggta gtaaatgggt     2820
   gtgtgtgagt ggttgggttg agttgggtgg atggtggatg gggaggtagt tttgtggggg     2880
   atgggttttt attgtattgt tttagtttag tttggtttaa gtgttttgtt ttttttttgg     2940
   gggattgtgt ggggaggtgg gaataattga tgtgttgagt ttttattaag tgttttgtat     3000
   ttttttggta gttttttttg aattatttaa tttttataag gttttagtaa ggtgaattta     3060
   gttattttta ttttgtagtt gaggaaatag aattttttag atttttaggt ttgagattag     3120
   gtttttttag tgagttagtg gtggtgtttt tgttggaatt taggatgttt ggtttgggat    3180
   gttttttttt ttatatatgt taggtttttt tttgtttttt attttttaga ttatggaagg    3240
   agttgtggtt ttgagagatt aaattgtgtt ttatgttttt ggttttatat atttagattt     3300
   gagttttttt tttatatgta gaggttttgt ttttttttaa gtttttatgt tatattattt     3360
   tgggtaatta gttttttttt ttttaatttt ttttttattt aaagggagtg aggggtatgt    3420
   gttagtgttt tttttgggtt tgtattgatt ttaaaaggtt ttttaggtgt ttgaaaattt     3480
   tttttatttt atggtggtga ttatggtttt atttatttaa gagttttttg ttgaagttgt    3540
   tggaatggtt agggttgatt atattgaaat atttgtatgg ttttaaatat ttttaattgt     3600
   tttgttgttt tttttttttt tttaattaaa aaaaatgaaa atttttaaaa gttaatttat     3660
   ttaaggattt ttttatttta gttttttgta agttttgaaa tggttaaaat aaaaagatta     3720
   taagtgtttt taaaaatgag atgaaattat tttgttaaat gtgaaatgat taagtgtaat     3780
   atatataaat tttagggaaa tttatttgtt atttttataa attaattttt ttggatttga     3840
   ttttggagta attagtttga atgttagtta ttttttttta tttggttaaa tttttttttt    3900
   ttgtttgggg tttttttaag atgagatgtt tttttgataa ttaatattta tt             3952
<210> 433
   <211> 3952
   <212> DNA
   <213> Artificial Sequence
<220>
   <223> chemically treated genomic DNA (Homo sapiens)
<400> 433
   aataaatgtt agttattaaa gaagtatttt attttaagga gattttaggt aggaggggag      60
   agtttagtta agtagggagg ggtgattaat atttaggtta attgttttaa aattagattt     120
   aaaaggatta atttataggg atgatagata aatttttttg aaatttgtat gtgttatatt     180
   tagttatttt atatttgata gaatgatttt attttatttt taagaatgtt tgtaattttt     240
   ttgttttggt tattttaagg tttataagga attaaggtgg aaggattttt agatgaattg     300
   atttttaaga atttttattt tttttaatta gagaaaagag agaaatagtg aaatagttga     360
   aagtatttga aattatatga atgttttagt ataattagtt ttggttattt taatagtttt     420
   agtaaagagt ttttaagtga atggggttgt ggttattgtt atgggatggg agagattttt     480
   aaatatttgg agggtttttt gaggttgatg taaatttagg ggaggtattg gtatatattt     540
   tttatttttt ttggataggg gaggagttgg gaggaaagga gttaattatt taagataatg     600
   tagtatagag atttgagagg gggtagggtt tttgtgtgtg gagaaggggt ttagatttaa     660
   atgtgtgggg ttaaaggtat agggtatgat ttaatttttt agagttatga ttttttttat     720
   gatttagaag gtagggggtg ggagggagtt tggtatatgt aggaaagaga atattttagg     780
   ttaagtattt tgggttttaa taaagatgtt attattgatt tattggggga gtttggtttt     840
   gggtttgaaa gtttagaagg ttttgttttt ttaattgtga agtgggaata attaaattta     900
   ttttgttagg attttataag gattaaataa tttaagagaa attattaggg aagtgtaagg     960
   tatttagtag gaatttagta tattgattat ttttgttttt ttatgtggtt ttttagggaa    1020
   gaagtgaggt gtttgagtta gattgggttg gaatagtgtg gtaggggttt gttttttgtg    1080
   gagttgtttt tttatttatt atttatttag tttagtttgg ttgtttatgt atgtttattt    1140
   gttgtttttg ttttattttt attaggtgta ttagttagtt tggtggtttt ggttatagat    1200
   gtttgaatga taaagtgttt ttattattgg tgtggtttgt tagttgattt gttgggatgt    1260
   gttttggttt tagttttttt ttttttattg tggtttagga agaaatttgg attgtgtata    1320
   gttattttag attgagtagt tgtgtgttga ggtggaggtg ggagttgtag gggttgtaga    1380
   tggtaggttt ttgttggggt atattttttt aagtgtttag gttttttatg tttagttttt    1440
   tttttttttt gggtttttgt gtggggattt ttgtttttgt ttagatttgg agtttaagtt    1500
   gttgtttttt tgggatttgg tttttttttt ttgttttttt ggtttatgtt tgtttatttg    1560
   tgtttgagta gtgtttttaa ttagtggtgt tagttagtag atgtgtttgt ggttgttttt    1620
   aaatttttgg atgtagttat aggttttgat agttttaggg aattggggtt gagtttttgg    1680
   gttggggttt gtatttggat agaatttttg tttattttat ttgtaggttt tattgttgat    1740
   ggattttggg gatagtgtta attttttttg ttttgttggg aaatgtagtt gtgattttga    1800
   gttgggatag tggttgtttt ttgaaaaggt gggggagtat tgagttggga attaggttgg    1860
   ggagtttagt tatgattttg gtttaatttg ttgtgagatt ttggttaagt tgtttaaatt    1920
   tttagagttt tagtttttgt atttgtaaag ttggaatttg gggtgtagtg ttttgatgaa    1980
   agatgttggt ggggagagtg aagatgtttt ttttgttgtt agatttttta gggtatttgg    2040
   tttatggtta taaagtaggt tatatttgat aattttgttg gattatttgg aggatttgtt    2100
   gatttttgtt gagttggtgg tttgggatat tgtgttatag aatttgaggt gtttgggtgt    2160
   ttttgttttg ttaggtgttt agtggtttgt attgagagtt aggagaggtt tagattggat    2220
   tttgattttt tgaggtgtgg gagtttatgg agtgtggtgg gtgtggtgtt tgggttgtgt    2280
   agttaggtgg ggagtggtgt gtagttttag atttgtaggt aggtagtggt ggattgtatt    2340
   tgttttgttt tgtagtgttt tttgtttgtt gttttttgtt tgtgtagtta gagttgtgtg    2400
   tggttaggaa gggtttttgt ttagtgtgtt ttggtgtttt gtattttgag atgtagttat    2460
   tgttagtttg ggtaggggta tatttgtttt gttttttgtg atttgttgtg ttgttttaag    2520
   ttgtgagaat atgtgtgttg gaggagtttg ttggttgtgg gggaattttg ggagtgggtt    2580
   tgttttggtg aagtgggtat ttttttttta gttttagatt tgtagtttta attttgggat    2640
   tgggagaggt tgtgagtagg agtgtgggga taggtgttgg aaatgtttaa gtttttgttt    2700
   tttttttttg ttttattgtt ttttagtggg taggtgggat tttgattatt ttagggtttg    2760
   tgttttgttt tgtttttttt ttttttgttt ttattttttt gttgtttttg ttgttgtttt    2820
   ttttttgttt tttttaattt attttttaat tttttttttg ttttttaatt tttttttttg    2880
   atgttttttt tttttattgt ttggttggtt tttattgttt ttgttgggtt ttttttgttt    2940
   ttagtttttt tgattttttt tattgtttta ttttgttgtt ttgggttttt tgttttagtt    3000
   tagtttgttt tttttatttt tagtttttgt ttagtttttt tttttttttt tgttttggtt    3060
   ttgttttatg attttttaga gattgagatg ttgaggggaa agtttttttg ggattttggt    3120
   atttgagtgt ttttttttga agaatttggg tattggagag ttttatgttt ttttttgaaa    3180
   gatttttttt agttttttta ttgttttttg tggtatttga ggattggtat ttgtgttttg    3240
   gttttgtttt tatttttatt ttggagagtt gtttttgtgt tttgggaatt ttagattttt    3300
   ttttgtggtt ttggtattag aggttttttt ttattattta tttttagtat ttgggatttt    3360
   aatgttttgt tttgtttttt tggtggtggt gtttggtttt ttttggagtt ttgattttgg    3420
   gaaagggagt gggttttttt tggttaatat ttatttttag aagtagtaat agtagtaggt    3480
   gtggtttgtt tatggtgtgg ttggtggtat ttgtttttat tgtttgtttt ttgtggtagt     3540
   gtttgatttt tagtttggtt tgttttgtgg attgatgggg ttgtgttgtg ttgtgtttta     3600
   gtgttttttt tgtttgttgg agttggttgt ggtttggttt gttttggttg tgggtgggag     3660
   aggttgggtg aagttagtgt tgtggttttg tttttgtttt gtttagtttg tttatttttg     3720
   gaattgtgtt tgtttgtttt gttgttttgg ggtgggaagt agaggtaaag ggagggtgtg     3780
   tggttttttg tattttgttg tgtttttttt tgttttgttt tttagagttt tttatttatt    3840
   ttgttttggt atttagtttt tggtttgtgt tattgtgtgt ttgttttgga tgttgtgggt     3900
   ttggggatta gggtgttttt attgtggttt ttgttttttt ttggttgtgg tt             3952
<210> 434 to <210> 599
   000
<210> 600
   <211> 22
   <212> DNA
   <213> Artificial Sequence
<220>
   <223> Detection primer for
<400> 600
   ggggttgata ttgtttttag ag 22
<210> 601
   <211> 19
   <212> DNA
   <213> Artificial Sequence
<220>
   <223> Detection primer for
<400> 601
   cccctccttc cttaaatct 19
<210> 602 to <210> 769
   000
<210> 770
   <211> 16
   <212> DNA
   <213> Artificial Sequence
<220>
   <223>
<400> 770
   ttcgtcggcg gtagag 16
<210> 771
   <211> 16
   <212> DNA
   <213> Artificial Sequence
<220>
   <223>
<400> 771
   tagagtttgt tggtgg 16
<210> 772
   <211> 16
   <212> DNA
   <213> Artificial Sequence
<220>
   <223>
<400> 772
   gatcgcgggt acgttt 16
<210> 773
   <211> 17
   <212> DNA
   <213> Artificial Sequence
<220>
   <223>
<400> 773
   attgtgggta tgtttgt 17
<210> 774
   <211> 16
   <212> DNA
   <213> Artificial Sequence
<220>
   <223>
<400> 774
   ttaacgtcgt tggtta 16
<210> 775
   <211> 17
   <212> DNA
   <213> Artificial Sequence
<220>
   <223>
<400> 775
   tgattaatgt tgttggt 17
<210> 776
   <211> 16
   <212> DNA
   <213> Artificial Sequence
<220>
   <223>
<400> 776
   ttcgcgcgaa gattta 16
<210> 777
   <211> 17
   <212> DNA
   <213> Artificial Sequence
<220>
   <223>
<400> 777
   gtttttgtgt gaagatt 17
<210> 778 to <210> 1306
   000
<210> 1307
   <211> 21
   <212> DNA
   <213> Artificial Sequence
<220> bisulfite treated
   <223> nucleic acid for analysis of methylation status of SEQ ID NO: 7
<400> 1307
   GAGAGAGAGG GTTGAAATTA G 21
<210> 1308
   <211> 16
   <212> DNA
   <213> Artificial Sequence
<220> bisulfite treated
   <223> nucleic acid for analysis of methylation status of SEQ ID NO: 7
<400> 1308
   CCATTACCGA CGCGAC 16
<210> 1309
   <211> 19
   <212> DNA
   <213> Artificial Sequence
<220> bisulfite treated
   <223> nucleic acid for analysis of methylation status of SEQ ID NO: 7
<400> 1309
   CTCTAATTTC AACCCTCTC 19
<210> 1310
   <211> 20
   <212> DNA
   <213> Artificial Sequence
<220> bisulfite treated
   <223> nucleic acid for analysis of methylation status of SEQ ID NO: 7
<400> 1310
   CCTCCATTAC CAACACAACC 20
<210> 1311
   <211> 20
   <212> DNA
   <213> Artificial Sequence
<220> bisulfite treated
   <223> nucleic acid for analysis of methylation status of SEQ ID NO: 7
<400> 1311
   AGAGAGAGGG TTGAAATTAG 20
<210> 1312
   <211> 23
   <212> DNA
   <213> Artificial Sequence
<220> bisulfite treated
   <223> nucleic acid for analysis of methylation status of SEQ ID NO: 7
<400> 1312
   CTAAATAACA AAATACCTCC ATT 23
<210> 1313
   <211> 23
   <212> DNA
   <213> Artificial Sequence
<220> bisulfite treated
   <223> nucleic acid for analysis of methylation status of SEQ ID NO: 7
<400> 1313
   AAATAACAAA ATACCTCCAT TAC 23
<210> 1314
   <211> 22
   <212> DNA
   <213> Artificial Sequence
<220> bisulfite treated
   <223> nucleic acid for analysis of methylation status of SEQ ID NO: 7
<400> 1314
   TAAATAACAA AATACCTCCA TT 22
<210> 1315
   <211> 20
   <212> DNA
   <213> Artificial Sequence
<220> bisulfite treated
   <223> nucleic acid for analysis of methylation status of SEQ ID NO: 7
<400> 1315
   ACCTCCATTA CCAACACAAC 20
<210> 1316
   <211> 20
   <212> DNA
   <213> Artificial Sequence
<220> bisulfite treated
   <223> nucleic acid for analysis of methylation status of SEQ ID NO: 7
<400> 1316
   CTCCATTACC AACACAACCC 20
<210> 1317
   <211> 21
   <212> DNA
   <213> Artificial Sequence
<220> bisulfite treated
   <223> nucleic acid for analysis of methylation status of SEQ ID NO: 7
<400> 1317
   TACCTCCATT ACCAACACAA C 21
<210> 1318
   <211> 21
   <212> DNA
   <213> Artificial Sequence
<220> bisulfite treated
   <223> nucleic acid for analysis of methylation status of SEQ ID NO: 7
<400> 1318
   CCTCCATTAC CAACACAACC C 21
<210> 1319
   <211> 21
   <212> DNA
   <213> Artificial Sequence
<220> bisulfite treated
   <223> nucleic acid for analysis of methylation status of SEQ ID NO: 7
<400> 1319
   ACCTCCATTA CCAACACAAC C 21
<210> 1320
   <211> 22
   <212> DNA
   <213> Artificial Sequence
<220> bisulfite treated
   <223> nucleic acid for analysis of methylation status of SEQ ID NO: 7
<400> 1320
   ACCTCCATTA CCAACACAAC CC 22
<210> 1321
   <211> 22
   <212> DNA
   <213> Artificial Sequence
<220> bisulfite treated
   <223> nucleic acid for analysis of methylation status of SEQ ID NO: 7
<400> 1321
   TACCTCCATT ACCAACACAA CC 22
<210> 1322
   <211> 23
   <212> DNA
   <213> Artificial Sequence
<220> bisulfite treated
   <223> nucleic acid for analysis of methylation status of SEQ ID NO: 7
<400> 1322
   ATACCTCCAT TACCAACACA ACC 23
<210> 1323
   <211> 22
   <212> DNA
   <213> Artificial Sequence
<220> bisulfite treated
   <223> nucleic acid for analysis of methylation status of SEQ ID NO: 7
<400> 1323
   AAATAACAAA ATACCTCCAT TA 22
<210> 1324
   <211> 23
   <212> DNA
   <213> Artificial Sequence
<220> bisulfite treated
   <223> nucleic acid for analysis of methylation status of SEQ ID NO: 7
<400> 1324
   TACCTCCATT ACCAACACAA CCC 23
<210> 1325
   <211> 24
   <212> DNA
   <213> Artificial Sequence
<220> bisulfite treated
   <223> nucleic acid for analysis of methylation status of SEQ ID NO: 7
<400> 1325
   AATACCTCCA TTACCAACAC AACC 24
<210> 1326
   <211> 21
   <212> DNA
   <213> Artificial Sequence
<220> bisulfite treated
   <223> nucleic acid for analysis of methylation status of SEQ ID NO: 7
<400> 1326
   AAATAACAAA ATACCTCCAT T 21
<210> 1327
   <211> 24
   <212> DNA
   <213> Artificial Sequence
<220> bisulfite treated
   <223> nucleic acid for analysis of methylation status of SEQ ID NO: 7
<400> 1327
   ATACCTCCAT TACCAACACA ACCC 24
<210> 1328
   <211> 25
   <212> DNA
   <213> Artificial Sequence
<220> bisulfite treated
   <223> nucleic acid for analysis of methylation status of SEQ ID NO: 7
<400> 1328
   AATACCTCCA TTACCAACAC AACCC 25
<210> 1329
   <211> 22
   <212> DNA
   <213> Artificial Sequence
<220> bisulfite treated
   <223> nucleic acid for analysis of methylation status of SEQ ID NO: 7
<400> 1329
   TCTAAATAAC AAAATACCTC CA 22
<210> 1330
   <211> 26
   <212> DNA
   <213> Artificial Sequence
<220> bisulfite treated
   <223> nucleic acid for analysis of methylation status of SEQ ID NO: 7
<400> 1330
   AAATACCTCC ATTACCAACA CAACCC 26
<210> 1331
   <211> 22
   <212> DNA
   <213> Artificial Sequence
<220> bisulfite treated
   <223> nucleic acid for analysis of methylation status of SEQ ID NO: 7
<400> 1331
   CTAAATAACA AAATACCTCC AT 22
<210> 1332
   <211> 27
   <212> DNA
   <213> Artificial Sequence
<220> bisulfite treated
   <223> nucleic acid for analysis of methylation status of SEQ ID NO: 7
<400> 1332
   AAAATACCTC CATTACCAAC ACAACCC 27
<210> 1333
   <211> 25
   <212> DNA
   <213> Artificial Sequence
<220> bisulfite treated
   <223> nucleic acid for analysis of methylation status of SEQ ID NO: 7
<400> 1333
   TCTAAATAAC AAAATACCTC CATTA 25
<210> 1334
   <211> 21
   <212> DNA
   <213> Artificial Sequence
<220> bisulfite treated
   <223> nucleic acid for analysis of methylation status of SEQ ID NO: 7
<400> 1334
   CCATTACCAA CACAACCCAC C 21
<210> 1335
   <211> 24
   <212> DNA
   <213> Artificial Sequence
<220> bisulfite treated
   <223> nucleic acid for analysis of methylation status of SEQ ID NO: 7
<400> 1335
   CTAAATAACA AAATACCTCC ATTA 24
<210> 1336
   <211> 24
   <212> DNA
   <213> Artificial Sequence
<220> bisulfite treated
   <223> nucleic acid for analysis of methylation status of SEQ ID NO: 7
<400> 1336
   TAAATAACAA AATACCTCCA TTAC 24
<210> 1337
   <211> 22
   <212> DNA
   <213> Artificial Sequence
<220> bisulfite treated
   <223> nucleic acid for analysis of methylation status of SEQ ID NO: 7
<400> 1337
   TCCATTACCA ACACAACCCA CC 22
<210> 1338
   <211> 23
   <212> DNA
   <213> Artificial Sequence
<220> bisulfite treated
   <223> nucleic acid for analysis of methylation status of SEQ ID NO: 7
<400> 1338
   CTCCATTACC AACACAACCC ACC 23
<210> 1338 to <210> 4648
   000
<210> 4649
   <211> 22
   <212> DNA
   <213> Artificial Sequence
<220> bisulfite treated
   <223> nucleic acid for analysis of methylation status of SEQ ID NO: 7
<400> 4649
   TTTCGATAGG AATTTGTCGT TT 22
<210> 4650
   <211> 21
   <212> DNA
   <213> Artificial Sequence
<220> bisulfite treated
   <223> nucleic acid for analysis of methylation status of SEQ ID NO: 7
<400> 4650
   GAAACGCGCT CTAATTTCAA C 21
<210> 4651
   <211> 29
   <212> DNA
   <213> Artificial Sequence
<220> bisulfite treated
   <223> nucleic acid for analysis of methylation status of SEQ ID NO: 7
<400> 4651
   TTGTTCGGTT TGGGATGGTT GTGCGCGGT 29
<210> 4652
   <211> 22
   <212> DNA
   <213> Artificial Sequence
<220> bisulfite treated
   <223> nucleic acid for analysis of methylation status of SEQ ID NO: 7
<400> 4652
   CGTTTAATAG GGATGGAACG AG 22
<210> 4653 to <12005>
   000
<210> 12006
   <211> 20
   <212> DNA
   <213> Artificial Sequence
<220> bisulfite treated
   <223> nucleic acid for analysis of methylation status of SEQ ID NO: 7
<400> 12006
   GTTTTCGGTG TAAGTCGTTG 20
<210> 12007
   <211> 22
   <212> DNA
   <213> Artificial Sequence
<220> bisulfite treated
   <223> nucleic acid for analysis of methylation status of SEQ ID NO: 7
<400> 12007
   ACATCTAACA ATCTCGTCGA AC 22
<210> 12008
   <211> 16
   <212> DNA
   <213> Artificial Sequence
<220> bisulfite treated
   <223> nucleic acid for analysis of methylation status of SEQ ID NO: 7
<400> 12008
   AACGAGACGG GGGCGT 16
<210> 12009
   <211> 27
   <212> DNA
   <213> Artificial Sequence
<220> bisulfite treated
   <223> nucleic acid for analysis of methylation status of SEQ ID NO: 7
<400> 12009
   TTCGGATTTT GTAGCGCGGT ATTTCGG 27
<210> 12010
   <211> 21
   <212> DNA
   <213> Artificial Sequence
<220> bisulfite treated
   <223> nucleic acid for analysis of methylation status of SEQ ID NO: 7
<400> 12010
   CATCTAACAA TCTCGTCGAA C 21
<210> 12011
   <211> 19
   <212> DNA
   <213> Artificial Sequence
<220> bisulfite treated
   <223> nucleic acid for analysis of methylation status of SEQ ID NO: 7
<400> 12011
   ATTCGAGCGT AGCGTTTAT 19
<210> 12012
   <211> 22
   <212> DNA
   <213> Artificial Sequence
<220> bisulfite treated
   <223> nucleic acid for analysis of methylation status of SEQ ID NO: 7
<400> 12012
   CGTTAAATAC CCAACGACTT AC 22
<210> 12013
   <211> 17
   <212> DNA
   <213> Artificial Sequence
<220> bisulfite treated
   <223> nucleic acid for analysis of methylation status of SEQ ID NO: 7
<400> 12013
   GGTTTTCGCG TTTCGAA 17
<210> 12014
   <211> 17
   <212> DNA
   <213> Artificial Sequence
<220> bisulfite treated
   <223> nucleic acid for analysis of methylation status of SEQ ID NO: 7
<400> 12014
   GTCGGGATTC GGTTTGA 17
<210> 12015
   <211> 19
   <212> DNA
   <213> Artificial Sequence
<220> bisulfite treated
   <223> nucleic acid for analysis of methylation status of SEQ ID NO: 7
<400> 12015
   GGTTTTCGGT GTAAGTCGT 19
<210> 12016
   <211> 19
   <212> DNA
   <213> Artificial Sequence
<220> bisulfite treated
   <223> nucleic acid for analysis of methylation status of SEQ ID NO: 7
<400> 12016
   TTTTCGGTGT AAGTCGTTG 19
<210> 12017
   <211> 19
   <212> DNA
   <213> Artificial Sequence
<220> bisulfite treated
   <223> nucleic acid for analysis of methylation status of SEQ ID NO: 7
<400> 12017
   ACGTTTCGGA TTTTGTAGC 19
<210> 12018
   <211> 18
   <212> DNA
   <213> Artificial Sequence
<220> bisulfite treated
   <223> nucleic acid for analysis of methylation status of SEQ ID NO: 7
<400> 12018
   TTCGGGTTAT CGATTCGG 18
<210> 12019
   <211> 16
   <212> DNA
   <213> Artificial Sequence
<220> bisulfite treated
   <223> nucleic acid for analysis of methylation status of SEQ ID NO: 7
<400> 12019
   AAAGGTCGGC GGGTTT 16
<210> 12020
   <211> 21
   <212> DNA
   <213> Artificial Sequence
<220> bisulfite treated
   <223> nucleic acid for analysis of methylation status of SEQ ID NO: 7
<400> 12020
   ATCTAACAAT CTCGTCGAAC C 21
<210> 12021
   <211> 18
   <212> DNA
   <213> Artificial Sequence
<220> bisulfite treated
   <223> nucleic acid for analysis of methylation status of SEQ ID NO: 7
<400> 12021
   TCGAGCGTAG CGTTTATC 18
<210> 12022
   <211> 18
   <212> DNA
   <213> Artificial Sequence
<220> bisulfite treated
   <223> nucleic acid for analysis of methylation status of SEQ ID NO: 7
<400> 12022
   TAGCGTTTAT CGCGTTTC 18
<210> 12023
   <211> 19
   <212> DNA
   <213> Artificial Sequence
<220> bisulfite treated
   <223> nucleic acid for analysis of methylation status of SEQ ID NO: 7
<400> 12023
   GTAGCGTTTA TCGCGTTTC 19
<210> 12024
   <211> 20
   <212> DNA
   <213> Artificial Sequence
<220> bisulfite treated
   <223> nucleic acid for analysis of methylation status of SEQ ID NO: 7
<400> 12024
   GATTCGAGCG TAGCGTTTAT 20
<210> 12025
   <211> 18
   <212> DNA
   <213> Artificial Sequence
<220> bisulfite treated
   <223> nucleic acid for analysis of methylation status of SEQ ID NO: 7
<400> 12025
   TTCGAGCGTA GCGTTTAT 18
<210> 12026
   <211> 19
   <212> DNA
   <213> Artificial Sequence
<220> bisulfite treated
   <223> nucleic acid for analysis of methylation status of SEQ ID NO: 7
<400> 12026
   GGACGTTTCG GATTTTGTA 19
<210> 12027
   <211> 22
   <212> DNA
   <213> Artificial Sequence
<220> bisulfite treated
   <223> nucleic acid for analysis of methylation status of SEQ ID NO: 7
<400> 12027
   AAACCTATAA CTACGTCCGA AA 22
<210> 12028
   <211> 22
   <212> DNA
   <213> Artificial Sequence
<220> bisulfite treated
   <223> nucleic acid for analysis of methylation status of SEQ ID NO: 7
<400> 12028
   TTTTGGTTTA CGTTCGTTTA TT 22
<210> 12029
   <211> 17
   <212> DNA
   <213> Artificial Sequence
<220> bisulfite treated
   <223> nucleic acid for analysis of methylation status of SEQ ID NO: 7
<400> 12029
   GTTCGCGGTC GTTTTTA 17
<210> 12030
   <211> 18
   <212> DNA
   <213> Artificial Sequence
<220> bisulfite treated
   <223> nucleic acid for analysis of methylation status of SEQ ID NO: 7
<400> 12030
   AGCGTTTTTA ATTAGCGG 18
<210> 12031
   <211> 22
   <212> DNA
   <213> Artificial Sequence
<220> bisulfite treated
   <223> nucleic acid for analysis of methylation status of SEQ ID NO: 7
<400> 12031
   AAAACCTATA ACTACGTCCG AA 22
<210> 12032
   <211> 22
   <212> DNA
   <213> Artificial Sequence
<220> bisulfite treated
   <223> nucleic acid for analysis of methylation status of SEQ ID NO: 7
<400> 12032
   TTTGGTTTAC GTTCGTTTAT TC 22
<210> 12033
   <211> 21
   <212> DNA
   <213> Artificial Sequence
<220> bisulfite treated
   <223> nucleic acid for analysis of methylation status of SEQ ID NO: 7
<400> 12033
   TTGGTTTACG TTCGTTTATT C 21
<210> 12034
   <211> 22
   <212> DNA
   <213> Artificial Sequence
<220> bisulfite treated
   <223> nucleic acid for analysis of methylation status of SEQ ID NO: 7
<400> 12034
   AACACGTCTA CTAACTAACG CC 22
<210> 12035
   <211> 21
   <212> DNA
   <213> Artificial Sequence
<220> bisulfite treated
   <223> nucleic acid for analysis of methylation status of SEQ ID NO: 7
<400> 12035
   TTTCGTTTTT GTTTAGATTC G 21
<210> 12036
   <211> 19
   <212> DNA
   <213> Artificial Sequence
<220> bisulfite treated
   <223> nucleic acid for analysis of methylation status of SEQ ID NO: 7
<400> 12036
   TACGTTCGTT TATTCGCGT 19
<210> 12037
   <211> 20
   <212> DNA
   <213> Artificial Sequence
<220> bisulfite treated
   <223> nucleic acid for analysis of methylation status of SEQ ID NO: 7
<400> 12037
   AAGTCGTTGT TTTTTTGGGA 20
<210> 12038
   <211> 21
   <212> DNA
   <213> Artificial Sequence
<220> bisulfite treated
   <223> nucleic acid for analysis of methylation status of SEQ ID NO: 7
<400> 12038
   TTTGGTTTAC GTTCGTTTAT T 21
<210> 12039
   <211> 21
   <212> DNA
   <213> Artificial Sequence
<220> bisulfite treated
   <223> nucleic acid for analysis of methylation status of SEQ ID NO: 7
<400> 12039
   CGTTTGAGTA GCGTTTTTAA T 21
<210> 12040
   <211> 17
   <212> DNA
   <213> Artificial Sequence
<220> bisulfite treated
   <223> nucleic acid for analysis of methylation status of SEQ ID NO: 7
<400> 12040
   TTCGCGGTCG TTTTTAA 17
<210> 12041
   <211> 19
   <212> DNA
   <213> Artificial Sequence
<220> bisulfite treated
   <223> nucleic acid for analysis of methylation status of SEQ ID NO: 7
<400> 12041
   GGCGTTAGTT AGTAGACGT 19
<210> 12042
   <211> 22
   <212> DNA
   <213> Artificial Sequence
<220> bisulfite treated
   <223> nucleic acid for analysis of methylation status of SEQ ID NO: 7
<400> 12042
   TCAAAACCTA TAACTACGTC CG 22
<210> 12043
   <211> 22
   <212> DNA
   <213> Artificial Sequence
<220> bisulfite treated
   <223> nucleic acid for analysis of methylation status of SEQ ID NO: 7
<400> 12043
   ACCTATAACT ACGTCCGAAA AT 22
<210> 12044
   <211> 20
   <212> DNA
   <213> Artificial Sequence
<220> bisulfite treated
   <223> nucleic acid for analysis of methylation status of SEQ ID NO: 7
<400> 12044
   GTTTACGTTC GTTTATTCGC 20
<210> 12045
   <211> 22
   <212> DNA
   <213> Artificial Sequence
<220> bisulfite treated
   <223> nucleic acid for analysis of methylation status of SEQ ID NO: 7
<400> 12045
   TAACTCTCGA TACAAACCGC TA 22
<210> 12046
   <211> 22
   <212> DNA
   <213> Artificial Sequence
<220> bisulfite treated
   <223> nucleic acid for analysis of methylation status of SEQ ID NO: 7
<400> 12046
   ATATTTGATA ATTTCGTCGG AT 22
<210> 12047
   <211> 24
   <212> DNA
   <213> Artificial Sequence
<220> bisulfite treated
   <223> nucleic acid for analysis of methylation status of SEQ ID NO: 7
<400> 12047
   ATTGTGTTAT AGAATTTGAG GTGT 24
<210> 12048
   <211> 18
   <212> DNA
   <213> Artificial Sequence
<220> bisulfite treated
   <223> nucleic acid for analysis of methylation status of SEQ ID NO: 7
<400> 12048
   GGAGGATTCG TCGATTTT 18
<210> 12049
   <211> 21
   <212> DNA
   <213> Artificial Sequence
<220> bisulfite treated
   <223> nucleic acid for analysis of methylation status of SEQ ID NO: 7
<400> 12049
   CAAAACCTAT AACTACGTCC G 21
<210> 12050
   <211> 23
   <212> DNA
   <213> Artificial Sequence
<220> bisulfite treated
   <223> nucleic acid for analysis of methylation status of SEQ ID NO: 7
<400> 12050
   AACCTATAAC TACGTCCGAA AAT 23
<210> 12051
   <211> 20
   <212> DNA
   <213> Artificial Sequence
<220> bisulfite treated
   <223> nucleic acid for analysis of methylation status of SEQ ID NO: 7
<400> 12051
   ACTCTCGATA CAAACCGCTA 20
<210> 12052
   <211> 22
   <212> DNA
   <213> Artificial Sequence
<220> bisulfite treated
   <223> nucleic acid for analysis of methylation status of SEQ ID NO: 7
<400> 12052
   GCGTTTGAGT AGCGTTTTTA AT 22
<210> 12053
   <211> 17
   <212> DNA
   <213> Artificial Sequence
<220> bisulfite treated
   <223> nucleic acid for analysis of methylation status of SEQ ID NO: 7
<400> 12053
   AGCGGCGTTA GTTAGTA 17
<210> 12054
   <211> 22
   <212> DNA
   <213> Artificial Sequence
<220> bisulfite treated
   <223> nucleic acid for analysis of methylation status of SEQ ID NO: 7
<400> 12054
   AACCTATAAC TACGTCCGAA AA 22
<210> 12055
   <211> 21
   <212> DNA
   <213> Artificial Sequence
<220> bisulfite treated
   <223> nucleic acid for analysis of methylation status of SEQ ID NO: 7
<400> 12055
   GTTTATTCGC GTTTGAGTAG C 21
<210> 12056
   <211> 22
   <212> DNA
   <213> Artificial Sequence
<220> bisulfite treated
   <223> nucleic acid for analysis of methylation status of SEQ ID NO: 7
<400> 12056
   TTATATTTGA TAATTTCGTC GG 22
<210> 12057
   <211> 20
   <212> DNA
   <213> Artificial Sequence
<220> bisulfite treated
   <223> nucleic acid for analysis of methylation status of SEQ ID NO: 7
<400> 12057
   TTTATTCGCG TTTGAGTAGC 20
<210> 12058
   <211> 23
   <212> DNA
   <213> Artificial Sequence
<220> bisulfite treated
   <223> nucleic acid for analysis of methylation status of SEQ ID NO: 7
<400> 12058
   GTTATATTTG ATAATTTCGT CGG 23
<210> 12059
   <211> 20
   <212> DNA
   <213> Artificial Sequence
<220> bisulfite treated
   <223> nucleic acid for analysis of methylation status of SEQ ID NO: 7
<400> 12059
   TCGTTTTTGT TTAGATTCGG 20
<210> 12060
   <211> 23
   <212> DNA
   <213> Artificial Sequence
<220> bisulfite treated
   <223> nucleic acid for analysis of methylation status of SEQ ID NO: 7
<400> 12060
   TATATTTGAT AATTTCGTCG GAT 23
<210> 12061
   <211> 21
   <212> DNA
   <213> Artificial Sequence
<220> bisulfite treated
   <223> nucleic acid for analysis of methylation status of SEQ ID NO: 7
<400> 12061
   CGTTTTTGTT TAGATTCGGA G 21
<210> 12062
   <211> 21
   <212> DNA
   <213> Artificial Sequence
<220> bisulfite treated
   <223> nucleic acid for analysis of methylation status of SEQ ID NO: 7
<400> 12062
   AACTCTCGAT ACAAACCGCT A 21
<210> 12063
   <211> 21
   <212> DNA
   <213> Artificial Sequence
<220> bisulfite treated
   <223> nucleic acid for analysis of methylation status of SEQ ID NO: 7
<400> 12063
   CTAACTCTCG ATACAAACCG C 21
<210> 12064
   <211> 21
   <212> DNA
   <213> Artificial Sequence
<220> bisulfite treated
   <223> nucleic acid for analysis of methylation status of SEQ ID NO: 7
<400> 12064
   ACACGTCTAC TAACTAACGC C 21
<210> 12065
   <211> 20
   <212> DNA
   <213> Artificial Sequence
<220> bisulfite treated
   <223> nucleic acid for analysis of methylation status of SEQ ID NO: 7
<400> 12065
   GGTTTACGTT CGTTTATTCG 20
<210> 12066
   <211> 23
   <212> DNA
   <213> Artificial Sequence
<220> bisulfite treated
   <223> nucleic acid for analysis of methylation status of SEQ ID NO: 7
<400> 12066
   ATCAAAACCT ATAACTACGT CCG 23
<210> 12067
   <211> 21
   <212> DNA
   <213> Artificial Sequence
<220> bisulfite treated
   <223> nucleic acid for analysis of methylation status of SEQ ID NO: 7
<400> 12067
   AACCTATAAC TACGTCCGAA A 21
<210> 12068
   <211> 20
   <212> DNA
   <213> Artificial Sequence
<220> bisulfite treated
   <223> nucleic acid for analysis of methylation status of SEQ ID NO: 7
<400> 12068
   CGATACAAAC CGCTAAACAC 20
<210> 12069
   <211> 21
   <212> DNA
   <213> Artificial Sequence
<220> bisulfite treated
   <223> nucleic acid for analysis of methylation status of SEQ ID NO: 7
<400> 12069
   TTCGTTTTTG TTTAGATTCG G 21
<210> 12070
   <211> 23
   <212> DNA
   <213> Artificial Sequence
<220> bisulfite treated
   <223> nucleic acid for analysis of methylation status of SEQ ID NO: 7
<400> 12070
   AAAACCTATA ACTACGTCCG AAA 23
<210> 12071
   <211> 20
   <212> DNA
   <213> Artificial Sequence
<220> bisulfite treated
   <223> nucleic acid for analysis of methylation status of SEQ ID NO: 7
<400> 12071
   CGTTTATTCG CGTTTGAGTA 20
<210> 12072
   <211> 23
   <212> DNA
   <213> Artificial Sequence
<220> bisulfite treated
   <223> nucleic acid for analysis of methylation status of SEQ ID NO: 7
<400> 12072
   TCTAAAATAA CTATACGCGA TCC 23
<210> 12073
   <211> 20
   <212> DNA
   <213> Artificial Sequence
<220> bisulfite treated
   <223> nucleic acid for analysis of methylation status of SEQ ID NO: 7
<400> 12073
   CGGTTTTGGT TATAGACGTT 20
<210> 12074
   <211> 18
   <212> DNA
   <213> Artificial Sequence
<220> bisulfite treated
   <223> nucleic acid for analysis of methylation status of SEQ ID NO: 7
<400> 12074
   GTTCGTTAGT CGATTCGT 18
<210> 12075
   <211> 17
   <212> DNA
   <213> Artificial Sequence
<220> bisulfite treated
   <223> nucleic acid for analysis of methylation status of SEQ ID NO: 7
<400> 12075
   ATCGCGGTTA GGAAGAA 17
<210> 12076
   <211> 20
   <212> DNA
   <213> Artificial Sequence
<220> bisulfite treated
   <223> nucleic acid for analysis of methylation status of SEQ ID NO: 7
<400> 12076
   CGACGATAAA ACCTACGAAT 20
<210> 12077
   <211> 22
   <212> DNA
   <213> Artificial Sequence
<220> bisulfite treated
   <223> nucleic acid for analysis of methylation status of SEQ ID NO: 7
<400> 12077
   GTTAGTTAGT AGACGTGTTC GC 22
<210> 12078
   <211> 23
   <212> DNA
   <213> Artificial Sequence
<220> bisulfite treated
   <223> nucleic acid for analysis of methylation status of SEQ ID NO: 7
<400> 12078
   GTCGTTTTTA AATTTTCGGA CGT 23
<210> 12079
   <211> 17
   <212> DNA
   <213> Artificial Sequence
<220> bisulfite treated
   <223> nucleic acid for analysis of methylation status of SEQ ID NO: 7
<400> 12079
   AGGGAATTGG GGTTGAG 17
<210> 12080
   <211> 19
   <212> DNA
   <213> Artificial Sequence
<220> bisulfite treated
   <223> nucleic acid for analysis of methylation status of SEQ ID NO: 7
<400> 12080
   TTATTCGCGT TTGAGTAGC 19
<210> 12081
   <211> 21
   <212> DNA
   <213> Artificial Sequence
<220> bisulfite treated
   <223> nucleic acid for analysis of methylation status of SEQ ID NO: 7
<400> 12081
   TATTTGATAA TTTCGTCGGA T 21
<210> 12082
   <211> 20
   <212> DNA
   <213> Artificial Sequence
<220> bisulfite treated
   <223> nucleic acid for analysis of methylation status of SEQ ID NO: 7
<400> 12082
   CGCGAACACG TCTACTAACT 20
<210> 12083
   <211> 21
   <212> DNA
   <213> Artificial Sequence
<220> bisulfite treated
   <223> nucleic acid for analysis of methylation status of SEQ ID NO: 7
<400> 12083
   AGCGTTTTTA ATTAGCGGCG T 21
<210> 12084
   <211> 19
   <212> DNA
   <213> Artificial Sequence
<220> bisulfite treated
   <223> nucleic acid for analysis of methylation status of SEQ ID NO: 7
<400> 12084
   TTTACGTTCG TTTATTCGC 19
<210> 12085
   <211> 19
   <212> DNA
   <213> Artificial Sequence
<220> bisulfite treated
   <223> nucleic acid for analysis of methylation status of SEQ ID NO: 7
<400> 12085
   AACCGCTAAA CACCTAACG 19
<210> 12086
   <211> 22
   <212> DNA
   <213> Artificial Sequence
<220> bisulfite treated
   <223> nucleic acid for analysis of methylation status of SEQ ID NO: 7
<400> 12086
   CGCGAACACG TCTACTAACT AA 22
<210> 12087
   <211> 21
   <212> DNA
   <213> Artificial Sequence
<220> bisulfite treated
   <223> nucleic acid for analysis of methylation status of SEQ ID NO: 7
<400> 12087
   ATTCGGAGTT TAAGTCGTTG T 21
<210> 12088
   <211> 20
   <212> DNA
   <213> Artificial Sequence
<220> bisulfite treated
   <223> nucleic acid for analysis of methylation status of SEQ ID NO: 7
<400> 12088
   TGGTTTACGT TCGTTTATTC 20
<210> 12089
   <211> 21
   <212> DNA
   <213> Artificial Sequence
<220> bisulfite treated
   <223> nucleic acid for analysis of methylation status of SEQ ID NO: 7
<400> 12089
   CCTATAACTA CGTCCGAAAA T 21
<210> 12090
   <211> 20
   <212> DNA
   <213> Artificial Sequence
<220> bisulfite treated
   <223> nucleic acid for analysis of methylation status of SEQ ID NO: 7
<400> 12090
   ATTTCTTCCT AACCGCGATA 20
<210> 12091
   <211> 22
   <212> DNA
   <213> Artificial Sequence
<220> bisulfite treated
   <223> nucleic acid for analysis of methylation status of SEQ ID NO: 7
<400> 12091
   TTAGGCGTAT TAGTTAGTTC GG 22
<210> 12092
   <211> 24
   <212> DNA
   <213> Artificial Sequence
<220> bisulfite treated
   <223> nucleic acid for analysis of methylation status of SEQ ID NO: 7
<400> 12092
   GTTTGAATGA TAAAGTGTTT TTAT 24
<210> 12093
   <211> 22
   <212> DNA
   <213> Artificial Sequence
<220> bisulfite treated
   <223> nucleic acid for analysis of methylation status of SEQ ID NO: 7
<400> 12093
   CTCGATACAA ACCGCTAAAC AC 22
<210> 12094
   <211> 21
   <212> DNA
   <213> Artificial Sequence
<220> bisulfite treated
   <223> nucleic acid for analysis of methylation status of SEQ ID NO: 7
<400> 12094
   TCGATACAAA CCGCTAAACA C 21
<210> 12095
   <211> 19
   <212> DNA
   <213> Artificial Sequence
<220> bisulfite treated
   <223> nucleic acid for analysis of methylation status of SEQ ID NO: 7
<400> 12095
   CTTCCTAACC GCGATAAAA 19
<210> 12096
   <211> 23
   <212> DNA
   <213> Artificial Sequence
<220> bisulfite treated
   <223> nucleic acid for analysis of methylation status of SEQ ID NO: 7
<400> 12096
   GAACACGTCT ACTAACTAAC GCC 23
<210> 12097
   <211> 19
   <212> DNA
   <213> Artificial Sequence
<220> bisulfite treated
   <223> nucleic acid for analysis of methylation status of SEQ ID NO: 7
<400> 12097
   CTCTCGATAC AAACCGCTA 19
<210> 12098
   <211> 24
   <212> DNA
   <213> Artificial Sequence
<220> bisulfite treated
   <223> nucleic acid for analysis of methylation status of SEQ ID NO: 7
<400> 12098
   TATCAAAACC TATAACTACG TCCG 24
<210> 12099
   <211> 20
   <212> DNA
   <213> Artificial Sequence
<220> bisulfite treated
   <223> nucleic acid for analysis of methylation status of SEQ ID NO: 7
<400> 12099
   TTCGGAGTTT AAGTCGTTGT 20
<210> 12100
   <211> 23
   <212> DNA
   <213> Artificial Sequence
<220> bisulfite treated
   <223> nucleic acid for analysis of methylation status of SEQ ID NO: 7
<400> 12100
   TTTTGGTTTA CGTTCGTTTA TTC 23
<210> 12101
   <211> 20
   <212> DNA
   <213> Artificial Sequence
<220> bisulfite treated
   <223> nucleic acid for analysis of methylation status of SEQ ID NO: 7
<400> 12101
   AAACCGCTAA ACACCTAACG 20
<210> 12102
   <211> 22
   <212> DNA
   <213> Artificial Sequence
<220> bisulfite treated
   <223> nucleic acid for analysis of methylation status of SEQ ID NO: 7
<400> 12102
   TCGTTTATTC GCGTTTGAGT AG 22
<210> 12103
   <211> 21
   <212> DNA
   <213> Artificial Sequence
<220> bisulfite treated
   <223> nucleic acid for analysis of methylation status of SEQ ID NO: 7
<400> 12103
   CGACAAAAAC CTACCGTCTA C 21
<210> 12104
   <211> 20
   <212> DNA
   <213> Artificial Sequence
<220> bisulfite treated
   <223> nucleic acid for analysis of methylation status of SEQ ID NO: 7
<400> 12104
   TCGGATCGCG TATAGTTATT 20
<210> 12105
   <211> 17
   <212> DNA
   <213> Artificial Sequence
<220> bisulfite treated
   <223> nucleic acid for analysis of methylation status of SEQ ID NO: 7
<400> 12105
   ATCGAGTAGT CGCGCGT 17
<210> 12106
   <211> 16
   <212> DNA
   <213> Artificial Sequence
<220> bisulfite treated
   <223> nucleic acid for analysis of methylation status of SEQ ID NO: 7
<400> 12106
   GGCGGGAGTC GTAGGG 16
<210> 12107
   <211> 21
   <212> DNA
   <213> Artificial Sequence
<220> bisulfite treated
   <223> nucleic acid for analysis of methylation status of SEQ ID NO: 7
<400> 12107
   AATTTCTTCC TAACCGCGAT A 21
<210> 12108
   <211> 22
   <212> DNA
   <213> Artificial Sequence
<220> bisulfite treated
   <223> nucleic acid for analysis of methylation status of SEQ ID NO: 7
<400> 12108
   CTAAAATAAC TATACGCGAT CC 22
<210> 12109
   <211> 21
   <212> DNA
   <213> Artificial Sequence
<220> bisulfite treated
   <223> nucleic acid for analysis of methylation status of SEQ ID NO: 7
<400> 12109
   TCGTTTATTC GCGTTTGAGT A 21
<210> 12110
   <211> 21
   <212> DNA
   <213> Artificial Sequence
<220> bisulfite treated
   <223> nucleic acid for analysis of methylation status of SEQ ID NO: 7
<400> 12110
   CGGAGTTTAA GTCGTTGTTT T 21
<210> 12111
   <211> 19
   <212> DNA
   <213> Artificial Sequence
<220> bisulfite treated
   <223> nucleic acid for analysis of methylation status of SEQ ID NO: 7
<400> 12111
   TTCCTAACCG CGATAAAAA 19
<210> 12112
   <211> 19
   <212> DNA
   <213> Artificial Sequence
<220> bisulfite treated
   <223> nucleic acid for analysis of methylation status of SEQ ID NO: 7
<400> 12112
   CGATCCGAAT TTCTTCCTA 19
<210> 12113
   <211> 21
   <212> DNA
   <213> Artificial Sequence
<220> bisulfite treated
   <223> nucleic acid for analysis of methylation status of SEQ ID NO: 7
<400> 12113
   GTATTAGTTA GTTCGGCGGT T 21
<210> 12114
   <211> 21
   <212> DNA
   <213> Artificial Sequence
<220> bisulfite treated
   <223> nucleic acid for analysis of methylation status of SEQ ID NO: 7
<400> 12114
   CGAATCTAAA CAAAAACGAA A 21
<210> 12115
   <211> 19
   <212> DNA
   <213> Artificial Sequence
<220> bisulfite treated
   <223> nucleic acid for analysis of methylation status of SEQ ID NO: 7
<400> 12115
   GTCGTAGGGG TTGTAGACG 19
<210> 12116
   <211> 16
   <212> DNA
   <213> Artificial Sequence
<220> bisulfite treated
   <223> nucleic acid for analysis of methylation status of SEQ ID NO: 7
<400> 12116
   GGTTTTCGCG CGGGGA 16
<210> 12117
   <211> 21
   <212> DNA
   <213> Artificial Sequence
<220> bisulfite treated
   <223> nucleic acid for analysis of methylation status of SEQ ID NO: 7
<400> 12117
   TGTCGGGGTA TATTTTTTTA A 21
<210> 12118
   <211> 19
   <212> DNA
   <213> Artificial Sequence
<220> bisulfite treated
   <223> nucleic acid for analysis of methylation status of SEQ ID NO: 7
<400> 12118
   TCTTCCTAAC CGCGATAAA 19
<210> 12119
   <211> 19
   <212> DNA
   <213> Artificial Sequence
<220> bisulfite treated
   <223> nucleic acid for analysis of methylation status of SEQ ID NO: 7
<400> 12119
   TTTCTTCCTA ACCGCGATA 19
<210> 12120
   <211> 24
   <212> DNA
   <213> Artificial Sequence
<220> bisulfite treated
   <223> nucleic acid for analysis of methylation status of SEQ ID NO: 7
<400> 12120
   ATCTAAAATA ACTATACGCG ATCC 24
<210> 12121
   <211> 21
   <212> DNA
   <213> Artificial Sequence
<220> bisulfite treated
   <223> nucleic acid for analysis of methylation status of SEQ ID NO: 7
<400> 12121
   GGTTTACGTT CGTTTATTCG C 21
<210> 12122
   <211> 20
   <212> DNA
   <213> Artificial Sequence
<220> bisulfite treated
   <223> nucleic acid for analysis of methylation status of SEQ ID NO: 7
<400> 12122
   CGGATCGCGT ATAGTTATTT 20
<210> 12123
   <211> 22
   <212> DNA
   <213> Artificial Sequence
<220> bisulfite treated
   <223> nucleic acid for analysis of methylation status of SEQ ID NO: 7
<400> 12123
   GATTCGGAGT TTAAGTCGTT GT 22
<210> 12124
   <211> 22
   <212> DNA
   <213> Artificial Sequence
<220> bisulfite treated
   <223> nucleic acid for analysis of methylation status of SEQ ID NO: 7
<400> 12124
   TTCGTTTATT CGCGTTTGAG TA 22
<210> 12125
   <211> 20
   <212> DNA
   <213> Artificial Sequence
<220> bisulfite treated
   <223> nucleic acid for analysis of methylation status of SEQ ID NO: 7
<400> 12125
   AGTCGTAGGG GTTGTAGACG 20
<210> 12126
   <211> 19
   <212> DNA
   <213> Artificial Sequence
<220> bisulfite treated
   <223> nucleic acid for analysis of methylation status of SEQ ID NO: 7
<400> 12126
   AGGTTTTTGT CGGGGTATA 19
<210> 12127
   <211> 21
   <212> DNA
   <213> Artificial Sequence
<220> bisulfite treated
   <223> nucleic acid for analysis of methylation status of SEQ ID NO: 7
<400> 12127
   TGGTTTACGT TCGTTTATTC G 21
<210> 12128
   <211> 21
   <212> DNA
   <213> Artificial Sequence
<220> bisulfite treated
   <223> nucleic acid for analysis of methylation status of SEQ ID NO: 7
<400> 12128
   GCGAACACGT CTACTAACTA A 21
<210> 12129
   <211> 19
   <212> DNA
   <213> Artificial Sequence
<220> bisulfite treated
   <223> nucleic acid for analysis of methylation status of SEQ ID NO: 7
<400> 12129
   GTTCGTTTAT TCGCGTTTG 19
<210> 12130 to <210> 14553
   000
<210> 14554
   <211> 20
   <212> DNA
   <213> Artificial Sequence
<220>
   <223> chemically treated genomic DNA (Homo sapiens)
<400> 14554
   tgagagagag agggttgaaa 20
<210> 14555
   <211> 24
   <212> DNA
   <213> Artificial Sequence
<220>
   <223> chemically treated genomic DNA (Homo sapiens)
<400> 14555
   tctaaataac aaaatacctc catt 24
<210> 14556
   <211> 16
   <212> DNA
   <213> Artificial Sequence
<220>
   <223> chemically treated genomic DNA (Homo sapiens)
<400> 14556
   cgacccgcca accgac 16
<210> 14557
   <211> 15
   <212> DNA
   <213> Artificial Sequence
<220>
   <223> chemically treated genomic DNA (Homo sapiens)
<400> 14557
   cgccgaaacg cgctc 15
<210> 14558
   <211> 27
   <212> DNA
   <213> Artificial Sequence
<220>
   <223> chemically treated genomic DNA (Homo sapiens)
<400> 14558
   ccattaccaa cacaacccac caaccaa 27
<210> 14559 to <210> 14614
   000
<210> 14615
   <211> 16
   <212> DNA
   <213> Artificial Sequence
<220>
   <223> chemically treated genomic DNA (Homo sapiens)
<400> 14615
   ggtttgggat ggttgt 16
<210> 14616
   <211> 24
   <212> DNA
   <213> Artificial Sequence
<220>
   <223> chemically treated genomic DNA (Homo sapiens)
<400> 14616
   tctaaataac aaaatacctc catt 24
<210> 14617
   <211> 27
   <212> DNA
   <213> Artificial Sequence
<220>
   <223> chemically treated genomic DNA (Homo sapiens)
<400> 14617
   ccattaccaa cacaacccac caaccaa 27
<210> 14618
   <211> 20
   <212> DNA
   <213> Artificial Sequence
<220>
   <223> chemically treated genomic DNA (Homo sapiens)
<400> 14618
   ggtcgcggtg agagagagag 20
<210> 14619
   <211> 21
   <212> DNA
   <213> Artificial Sequence
<220>
   <223> chemically treated genomic DNA (Homo sapiens)
<400> 14619
   tgaaattaga gcgcgtttcg g 21
<210> 14620 to <210> 14624
   000

## Description

### FIELD OF THE INVENTION

The present invention relates to methods using genomic DNA sequences that exhibit altered CPG methylation patterns in disease states relative to normal. Particular embodiments of this disclosure methods, nucleic acids, nucleic acid arrays and kits useful for detecting, or for detecting and differentiating between or among colorectal cell proliferative disorders.

### BACKGROUND

The etiology of pathogenic states is known to involve modified methylation patterns of individual genes or of the genome. 5-methylcytosine, in the context of CpG dinucleotide sequences, is the most frequent covalently modified base in the DNA of eukaryotic cells, and plays a role in the regulation of transcription, genetic imprinting, and tumorigenesis. The identification and quantification of 5-methylcytosine sites in a specific specimen, or between or among a plurality of specimens, is thus of considerable interest, not only in research, but particularly for the molecular diagnoses of various diseases.

*Correlation of aberrant DNA methylation with cancer.* Aberrant DNA methylation within CpG 'islands' is characterized by *hyper-* or *hypomethylation* of CpG dinucleotide sequences leading to abrogation or overexpression of a broad spectrum of genes, and is among the earliest and most common alterations found in, and correlated with human malignancies. Additionally, abnormal methylation has been shown to occur in CpG-rich regulatory elements in intronic and coding parts of genes for certain tumors. In colon cancer, for example, aberrant DNA methylation constitutes one of the most prominent alterations and inactivates many tumor suppressor genes such as p14ARF, p16INK4a, THBS1, MINT2, and MINT31 and DNA mismatch repair genes such as hMLH1.

In contrast to the specific hypermethylation of tumor suppressor genes, an overall hypomethylation of DNA can be observed in tumor cells. This decrease in global methylation can be detected early, far before the development of frank tumor formation. A correlation between hypomethylation and increased gene expression has been determined for many oncogenes.

In the United States the annual incidence of colorectal cancer is approximately 150,000, with 56,600 individuals dying form colorectal cancer each year. The lifetime risk of colorectal cancer in the general population is about 5 to 6 percent. Despite intensive efforts in recent years in screening and early detection of colon cancer, until today most cases are diagnosed in an advanced stage with regional or distant metastasis. While the therapeutic options include surgery and adjuvant or palliative chemotherapy, most patients die from progression of their cancer within a few months. Identifying the molecular changes that underlie the development of colon cancer may help to develop new monitoring, screening, diagnostic and therapeutic options that could improve the overall poor prognosis of these patients.

The current guidelines for colorectal screening according to the American Cancer Society utilizes one of five different options for screening in average risk individuals 50 years of age or older. These options include 1) fecal occult blood test (FOBT) annually, 2) flexible sigmoidoscopy every five years, 3) annual FPBT plus flexible sigmoidoscopy every five years, 4) double contrast barium enema (DCBE) every five years or 5) colonoscopy every ten years. Even though these testing procedures are well accepted by the medical community, the implementation of widespread screening for colorectal cancer has not been realized. Patient compliance is a major factor for limited use due to the discomfort or inconvenience associated with the procedures. FOBT testing, although a non-invasive procedure, requires dietary and other restrictions 3-5 days prior to testing. Sensitivity levels for this test are also very low for colorectal adenocarcinoma with wide variability depending on the trial. Sensitivity measurements for detection of adenomas is even less since most adenomas do not bleed. In contrast, sensitivity for more invasive procedures such as sigmoidoscopy and colonoscopy are quite high because of direct visualization of the lumen of the colon. No randomized trials have evaluated the efficacy of these techniques, however, using data from case-control studies and data from the National Polyp Study (U.S.) it has been shown that removal of adenomatous polyps results in a 76-90% reduction in CRC incidence. Sigmoidoscopy has the limitation of only visualizing the left side of the colon leaving lesions in the right colon undetected. Both scoping procedures are expensive, require cathartic preparation and have increased risk of morbidity and mortality. Improved tests with increased sensitivity, specificity, ease of use and decreased costs are clearly needed before general widespread screening for colorectal cancer becomes routine.

Early colorectal cancer detection is generally based on the fecal occult blood test (FOBT) performed annually on asymptomatic individuals. Current recommendations adapted by several healthcare organizations, including the American Cancer Society, call for fecal occult blood testing beginning at age 50, repeated annually until such time as the patient would no longer benefit from screening. A positive FOBT leads to colonoscopic examination of the bowel; an expensive and invasive procedure, with a serious complication rate of one per 5,000 examinations. Only 12% of patients with heme positive stool are diagnosed with cancer or large polyps at the time of colonoscopy. A number of studies show that FOBT screening does not improve cancer-related mortality or overall survival. Compliance with occult blood testing has been poor; less than 20 percent of the population is offered or completes FOBT as recommended. If FOBT is properly done, the patient collects a fecal sample from three consecutive bowel movements. Samples are obtained while the patient adheres to dietary guidelines and avoids medications known to induce occult gastrointestinal bleeding. In reality, physicians frequently fail to instruct patients properly, patients frequently fail to adhere to protocol, and some patients find the task of collecting fecal samples difficult or unpleasant, hence compliance with annual occult blood testing is poor. If testing sensitivity and specificity can be improved over current methods, the frequency of testing could be reduced, collection of consecutive samples would be eliminated, dietary and medication schedule modifications would be eliminated, and patient compliance would be enhanced. Compounding the problem of compliance, the sensitivity and specificity of FOBT to detect colon cancer is poor. Poor test specificity leads to unnecessary colonoscopy, adding considerable expense to colon cancer screening.

Specificity of the FOBT has been calculated at best to be 96%, with a sensitivity of 43% (adenomas) and 50% (colorectal carcinoma). Sensitivity can be improved using an immunoassay FOBT such as that produced under the tradename 'InSure™', with an improved sensitivity of 77 % (adenomas) and 88.9% (colorectal carcinoma.

Molecular disease markers offer several advantages over other types of markers, one advantage being that even samples of very small sizes and/or samples whose tissue architecture has not been maintained can be analyzed quite efficiently. Within the last decade a number of genes have been shown to be differentially expressed between normal and colon carcinomas. However, no single or combination of marker has been shown to be sufficient for the diagnosis of colon carcinomas. High-dimensional mRNA based approaches have recently been shown to be able to provide a better means to distinguish between different tumor types and benign and malignant lesions. However its application as a routine diagnostic tool in a clinical environment is impeded by the extreme instability of mRNA, the rapidly occurring expression changes following certain triggers (e.g., sample collection), and, most importantly, the large amount of mRNA needed for analysis (Lipshutz, R. J. et al., Nature Genetics 21:20-24, 1999; Bowtell, D. D. L. Nature genetics suppl. 21:25-32, 1999), which often cannot be obtained from a routine biopsy.

The use of biological markers to further improve sensitivity and specificity of FOBT has been suggested, examples of such tests include the PreGen-Plus™ stool analysis assay available from EXACT Sciences which has a sensitivity of 20% (adenoma) and 52% (colorectal carcinoma) and a specificity of 95% in both cases. This test assays for the presence of 23 DNA mutations associated with the development of colon neoplasms. The use of DNA methylation as colon cancer markers is known. For example Sabbioni et al. (Molecular Diagnosis 7:201-207, 2003) detected hypermethylation of a panel of genes consisiting TPEF, HIC1, DAPK and MGMT in peripheral blood in 98% of colon carcinoma patients. However, this does provide a suitable basis for a commercially marketable test, as the specificity of such a test must also be sufficiently high.

The current model of colorectal pathogenesis favours a stepwise progression of adenomas, which includes the development of dysplasia and finally signs of invasive cancer. The molecular changes underlying this adenoma-carcinoma sequence include genetic and epigenetic alterations of tumor suppressor genes (APC, p53, DCC), the activation of oncogenes (K-ras) and the inactivation of DNA mismatch repair genes. Recently, further molecular changes and genetic defects have been revealed. Thus, activation of the Wnt signalling pathway not only includes mutations of the A PC gene, but may also result from β-catenin mutations. Furthermore, alterations in the TGF-β signalling pathway together with its signal transducers SMAD4 and SMAD2 have been linked to the development of colon cancer.

Despite recent progress in the understanding of the pathogenesis of adenomas and carcinomas of the colon and their genetic and molecular changes, the genetic and epigenetic changes underlying the development of metastasis are less well understood. It is, however, generally well accepted that the process of invasion and proteolysis of the extracellular matrix, as well as infiltration of the vascular basement membrane involve adhesive proteins, such as members of the family of integrin receptors, the cadherins, the immunoglobulin superfamily, the laminin binding protein and the CD44 receptor. Apart from adhesion, the process of metastasis formation also includes the induction and regulation of angiogenesis (VEGF, bFGF), the induction of cell proliferation (EGF, HGF, IGF) and the activation of proteolytic enzymes (MMPs, TIMPs, uPAR), as well as the inhibition of apoptosis (Bcl-2, Bcl-X). More recently other groups have compared the genetic and molecular changes in metastatic lesions to the changes found in primary colorectal cancers. Thus, Kleeff et al. reported the loss of DOC-2, a candidate tumor suppressor gene, both in primary and metastatic colorectal cancer. Furthermore, Zauber et al. reported that in their series of 42 colorectal cancers Ki-ras mutations in the primary cancers were identical in all of the 42 paired primary and synchronous metastatic lesions. Similarly loss of heterozygosity at the APC locus was identical for 39 paired carcinomas and synchronous metastasis. The authors concluded that for Ki-ras and APC genes the genetic changes in metastasis are identical to the primary colorectal cancer. However, other groups have found genetic and molecular changes in metastatic colon cancers, that are not present in the primary cancers. Thus, the development of LOH of chromosome 3p in colorectal metastasis has been reported. In addition, using comparative genomic hybridization several alterations were found in liver metastasis that were unique to metastastic lesions (-9q, -11q, and -17q).

Apart from mutations aberrant methylation of CpG islands has been shown to lead to the transcriptional silencing of certain genes that have been previously linked to the pathogenesis of various cancers. CpG islands are short sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting.

Recently several groups have also analysed the methylation of various genes in colorectal cancer and reported the transcriptional silencing by promoter methylation for p16INK4, p14ARF, p15INK4b, MGMT, hMLH1, GSTP1, DAPK, CDH1, TIMP-3 and APC among others. Thus apart from mutational inactivation of certain genes, the hypermethylation of these genes also contributes significantly to the pathogenesis of this disease.

In recent years several genes that are methylated in colon cancer have been identified by MS-APPCR. This group of genes, among others, includes TPEF/HPP1 which is frequently methylated in colon cancers and which was independently identified by two different groups using the MS-APPCR method (see, e.g., Young J, Biden KG, Simms LA, Huggard P, Karamatic R, Eyre HJ, Sutherland GR, Herath N, Barker M, Anderson GJ, Fitzpatrick DR, Ramm GA, Jass JR, Leggett BA. HPP1: a transmembrane protein-encoding gene commonly methylated in colorectal polyps and cancers. Proc Natl Acad Sci USA 98:265-270, 2001).

*Multifactorial approach.* Cancer diagnostics has traditionally relied upon the detection of single molecular markers (e.g., gene mutations, elevated PSA levels). Unfortunately, cancer is a disease state in which single markers have typically failed to detect or differentiate many forms of the disease. Thus, assays that recognize only a single marker have been shown to be of limited predictive value. A fundamental aspect of this invention is that methylation-based cancer diagnostics and the screening, diagnosis, and therapeutic monitoring of such diseases will provide significant improvements over the state-of-the-art that uses single marker analyses by the use of a selection of multiple markers. The multiplexed analytical approach is particularly well suited for cancer diagnostics since cancer is not a simple disease, this multi-factorial "panel" approach is consistent with the heterogeneous nature of cancer, both cytologically and clinically.

Key to the successful implementation of a panel approach to methylation based diagnostic tests is the design and development of optimized panels of markers that can characterize and distinguish disease states.

*Development of medical tests.* Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, *i.e.,* individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, i.e., individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cancer, often involve a plurality of genes and proteins. Additionally, certain proteins may be elevated for reasons unrelated to a disease state. n example of a test that has high specificity is a gene-based test that can detect a p53 mutation. Specificity is important when the cost or risk associated with further diagnostic procedures or further medical intervention are very high.

*Pronounced need in the art.* If colon cancer screening specificity can be increased, the problem of false positive test results leading to unnecessary colonoscopic examination would be reduced leading to cost savings and improved safety.

In view of the incidence of colon cancer and disadvantages associated with current colorectal cell proliferative disorder screening methods there is a substantial need in the art for improved methods for the early detection of colon cell proliferative disorders, in particular colon cancer, to be used in addition to or as a substitute for currently available tests. The methylation status of certain genes is known to be important in the colon cancer phenotype (Toyota et al. PNAS, vol. 96, 1999, p. 8681-8686; Rashid et al. American Journal of Pathology, vol. 159, no. 3 2011, p. 1129-1135; WO 02/24056; van Rijnsoever et al. Gut, vol. 51, no. 6, 2002, p. 797-802; WO 03/014388), but NGFR was heretofore not known to be hypermethylated in colon proliferative disorders.

### SUMMARYOF THE INVENTION

Various aspects of the present disclosure provide efficient and unique panels of genes, whereby methylation analysis NGFR enables the detection of cell proliferative disorders of the colon with a particularly high sensitivity, specificity and/or predictive value. The inventive colorectal cancer testing methods have particular utility for the screening of at-risk populations. The inventive methods have advantages over prior art methods (including the industry standard FOBT), because of improved sensitivity, specificity and likely patient compliance.

The present invention provides novel methods for detecting or distinguishing between colorectal cell proliferative disorders. Said methods are most preferably utilised for detecting or detecting and distinguishing between one or more of the following: colorectal carcinoma, colon adenoma, inflammatory colon tissue, grade 2 dysplasia colon adenomas less than 1 cm, grade 3 dysplasia colon adenomas larger than 1 cm, normal colon tissue, non-colon normal tissue, body fluids and non-colon cancer tissue.

In one embodiment the invention provides a method for detecting and/or for detecting and distinguishing between or among colorectal cell proliferative disorders in a subject. Said method comprises the following steps: i) contacting NGFR genomic DNA isolated from blood plasma, blood serum, whole blood, isolated blood cells, cells isolated from the blood obtained from the subject with at least one reagent, or series of reagents that distinguishes between methylated and non-methylated CpG dinucleotides within at least one target region of the genomic DNA, wherein the nucleotide sequence of said target region comprises at least one CpG dinucleotide sequence; and ii) detecting, or detecting and distinguishing between or among c olorectal cell proliferative disorders afforded with a sensitivity of greater than or equal to 80% and a specificity of greater than or equal to 80%.

Preferably, the sensitivity is from about 75% to about 96%, or from about 80% to about 90%, or from about 80% to about 85%. Perferably, the specificity is is from about 75% to about 96%, or from about 80% to about 90%, or from about 80% to about 85%.

Current methods used to detect and diagnose colorectal cell proliferative disorders include colonoscopy, sigmoidoscopy, and fecal occult blood colon cancer. In comparison to these methods, the disclosed invention is much less invasive than colonoscopy, and as, if not more sensitive than sigmoidoscopy and FOBT. The development of a body fluid assay represents a clear technical advantage over current methods known in the art in that it is anticipated that patient compliance for a single body fluid based test will be higher than the triplicate analysis of stool currently recommended for FOBT_{.}

A particular embodiment of the disclosed method comprises the use of NGFR as marker for the differentiation, detection and distinguishing of colorectal cell proliferative disorders.

Said use of the gene may be enabled by means of any analysis of the expression of the gene, by means of mRNA expression analysis or protein expression analysis. However, in the most preferred embodiment of the invention, the detection, differentiation and distinguishing of colorectal cell proliferative disorders is enabled by means of analysis of the *methylation status*.

The disclosure provides a method for the analysis of biological samples for features associated with the development of colorectal cell proliferative disorders, of the disclosed method characterised in that nucleic acid, or a fragment thereof, according to SEQ ID NO:7 is contacted with a reagent or series of reagents capable of distinguishing between methylated and non methylated CpG dinucleotides within the genomic sequence, or sequences of interest.

The method has utility for the improved diagnosis, treatment and monitoring of colorectal cell proliferative disorders, more specifically by enabling of the disclosed improved identification of and differentiation between subclasses of said disorder. The method presents improvements over the state of the art in that it enables a highly specific classification of colorectal cell proliferative disorders, thereby allowing for improved and informed treatment of patients.

Preferably, the source of the test sample is selected from the group consisting of cells or cell lines, histological slides, biopsies, paraffin-embedded tissue, bodily fluids, ejaculate, stool, urine, blood, and combinations thereof. Preferably, the source is selected from the group consisting of stool, blood plasma, blood serum, whole blood, isolated blood cells, cells isolated from the blood obtained from the subject.

Specifically, the present disclosure provides a method for detecting colorectal cell proliferative disorders, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within a target sequence of the subject nucleic acid, wherein the target sequence comprises, or hybridizes under stringent conditions to, a sequence comprising at least 16 contiguous nucleotides of SEQ ID NO 7, said contiguous nucleotides comprising at least one CpG dinucleotide sequence; and determining, based at least in part on said distinguishing, the methylation state of at least one target CpG dinucleotide sequence, or an average, or a valuer reflecting an average methylation state of a plurality of target CpG dinucleotide sequences. Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NOS:316, 317, 432 and 433, and contiguous regions thereof corresponding to the target sequence.

Additional embodiments provide a method for the detection of colorectal cell proliferative disorders, comprising: obtaining a biological sample having subject genomic DNA; extracting the genomic D NA; treating the genomic D NA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NOS:316, 317, 432 and 433, and complements thereof, wherein the treated DNA or the fragment thereof is either amplified to produce an amplificate, or is not amplified; and determining, based on a presence or absence of, or on a property of NGFR said amplificate, of the disclosed methylation state of NGFR at least one CpG dinucleotide sequence in a sequence according to SEQ ID NO: 7, or an average, or a value reflecting an average methylation state of a plurality of CpG dinucleotide sequences thereof. Preferably, at least one such hybridizing nucleic acid molecule or peptide nucleic acid molecule is bound to a solid phase. Preferably, determining comprises use of at least one method selected from the group consisting of: hybridizing at least one nucleic acid molecule comprising a contiguous sequence at least 9 nucleotides in length that is complementary to, or hybridizes under moderately stringent or stringent conditions to a sequence selected from the group consisting of SEQ ID NOS:316, 317, 432 and 433, and complements thereof; hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NOS:316, 317, 432 and 433, and complements thereof; hybridizing at least one nucleic acid molecule comprising a contiguous sequence at least 9 nucleotides in length that is complementary to, or hybridizes under moderately stringent or stringent conditions to a sequence selected from the group consisting of SEQ ID NOS:316, 317, 432 and 433, and complements thereof, and extending at least one such hybridized nucleic acid molecule by at least one nucleotide base; and sequencing of the amplificate.

Further embodiments provide a method for the analysis of colorectal cell proliferative disorders, comprising: obtaining a biological sample having subject genomic DNA; extracting of the disclosed genomic DNA; contacting of the disclosed genomic DNA, or a fragment thereof, comprising a sequence according to SEQ ID NO: 7 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, of the disclosed methylation state of at least one CpG dinucleotide sequence of said genomic sequence, or an average, or a value reflecting an average methylation state of a plurality of CpG dinucleotide sequences thereof. Preferably, the digested or undigested genomic DNA is amplified prior to said determining.

Additional embodiments provide the use of a nucleic acid derived from SEQ ID NO:7 in detecting colon cell proliferative disorders.

### BRIEF DESCRIPTION OF THE DRAWINGS

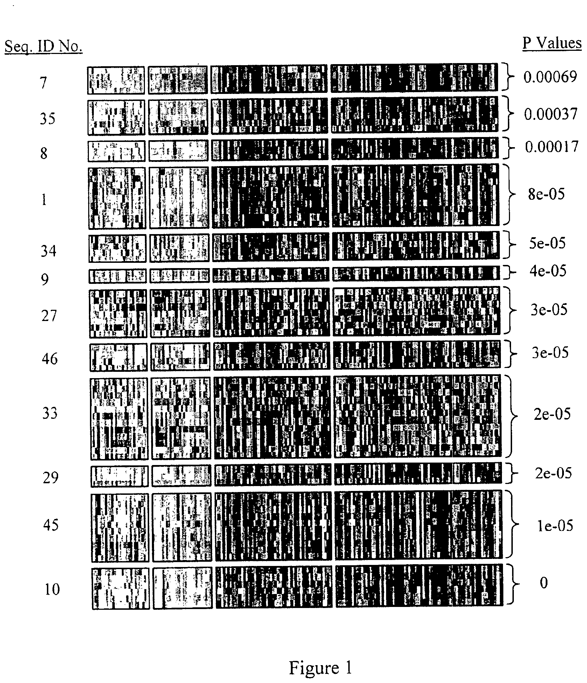

Figures 1, 5, 9, 16, 20, show ranked matrices of data obtained according to EXAMPLES 1, and according to CpG methylation differences between the two classes of tissues, using a suitable algorithm. The figures are shown in greyscale, wherein the most significant CpG positions are at the bottom of the matrix with significance decreasing towards the top. Black indicates total methylation at a given CpG position, white represents no methylation at the particular position, with degrees of methylation represented in grey, from light (low proportion of methylation) to dark (high proportion of methylation). Each row represents one specific CpG position within a gene and each column shows the methylation profile for the different CpGs for one sample. The p-values for the individual CpG positions are shown on the right side. The p-values are the probabilities that the observed distribution occurred by chance in the data set.

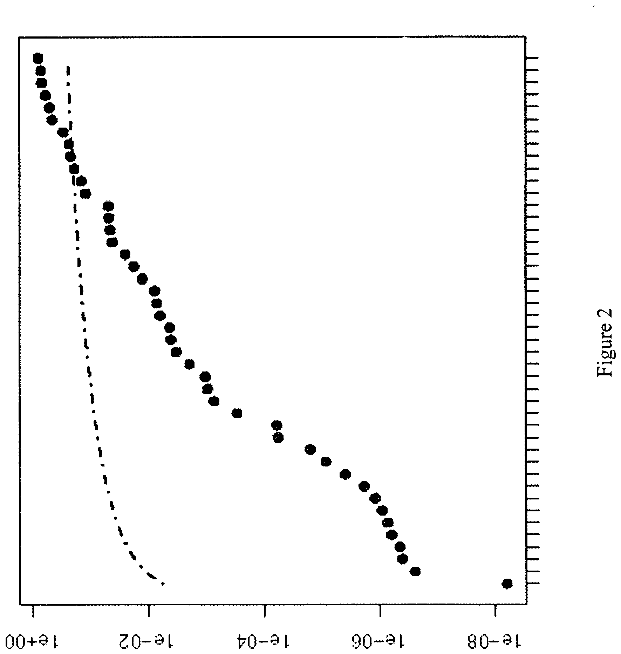

Figures 2, 6, 10, 17, 21, 25, 29 show the uncorrected multivariate LogReg test results for each comparison. Each individual genomic region of interest is represented as a point, the dotted line represents the cut off point for the 25% false discovery rate.

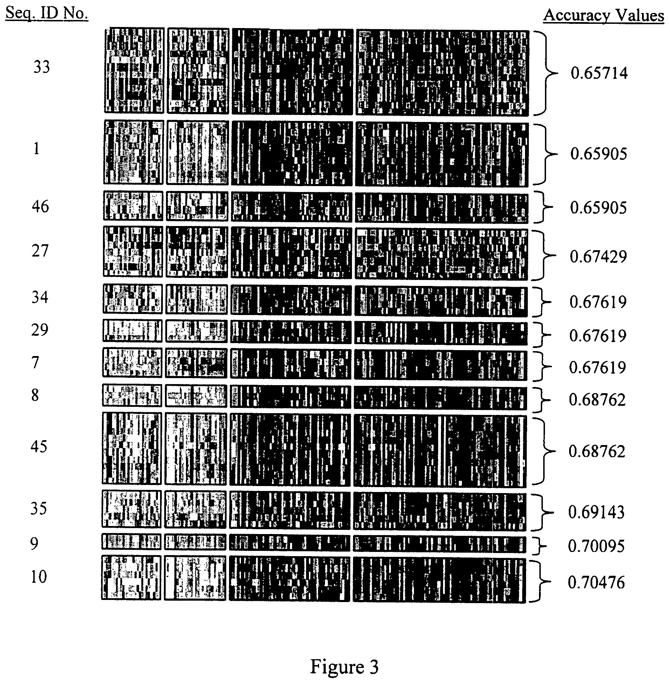

Figures 3, 7, 11, 22, 26, show ranked matrices of data, obtained according to EXAMPLES 1 of the accuracy of the genewise linear support vector machine cross validations between the two classes of tissues, for the best performing markers. The figures are shown in greyscale, wherein the most significant CpG positions are at the bottom of the matrix with significance decreasing towards the top. Black indicates total methylation at a given CpG position, white represents no methylation at the particular position, with degrees of methylation represented in grey, from light (low proportion of methylation) to dark (high proportion of methylation). Each row represents one specific CpG position within a gene and each column shows the methylation profile for the different CpGs for one sample. Accuracy values for each individual genomic region of interest are shown on the right side.

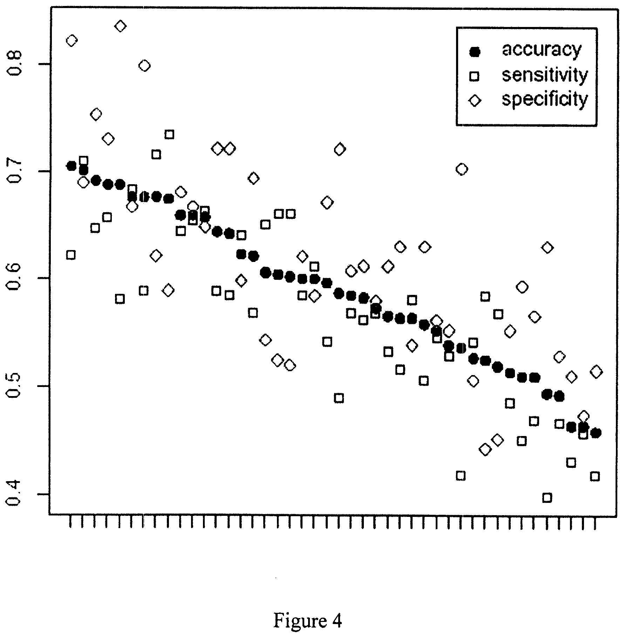

Figures 4, 8, 12, 23, 27, 31 and 35 show the accuracy of the genewise linear support vector machine cross validation for all genomic regions of each classification. The accuracy of each genomic region is represented as black squares, the specificity as unfilled diamonds, and the sensitivity as unfilled squares. The accuracy as measured on the X-axis shows the fraction of correctly classified samples.

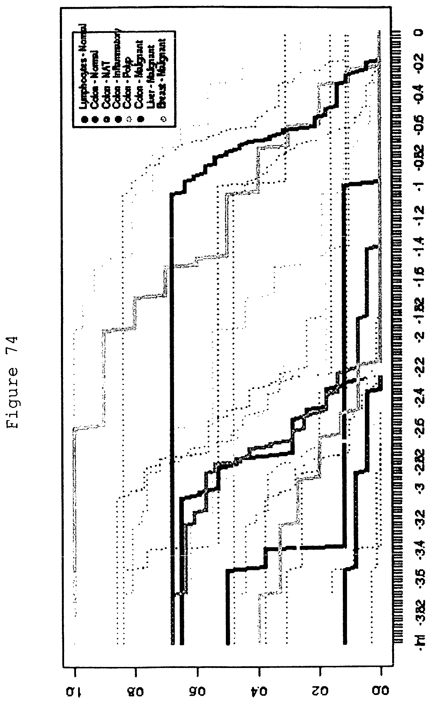

Figures 74 shows of the disclosed PCR amplification of samples according to assay 3 of example 14 respectively. The X-axis shows the cycle number of the polymerase chain reaction, the Y-axis shows the fluorescence intensity over background.

### DETAILED DESCRIPTION OF THE INVENTION

### Definitions:

The term "Observed/Expected Ratio" ("O/E Ratio") refers to the frequency of CpG dinucleotides within a particular DNA sequence, and corresponds to the [number of CpG sites / (number of C bases x number of G bases)] x band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG CpG dinucleotide sequences) within a DNA sequence include "unmethylated," "fully-methylated" and "hemi-methylated."

The term "hemi-methylation" or "hemimethylation" refers to the methylation state of a palindromic CpG methylation site, where only a single cytosine in one of the two CpG dinucleotide sequences of the palindromic CpG methylation site is methylated (*e.g.,* 5'-CC^{M}GG-3' (top strand): 3'-GGCC-5' (bottom strand)).

The term 'AUC' as used herein is an abbreviation for the area under a curve. In particular it refers to the area under a Receiver Operating Characteristic (ROC) curve. The ROC curve is a plot of the true positive rate against the false positive rate for the different possible cutpoints of a diagnostic test. It shows the tradeoff between sensitivity and specificity depending on the selected cutpoint (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of NGFR 0.5; for reference: J.P. Egan. Signal Detection Theory and ROC Analysis, Academic Press, New York, 1975).

The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylations. Further epigenetic parameters include, for example, the acetylation of histones which, however, cannot be directly analyzed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "array SEQ ID NO," "composite array SEQ ID NO," or "composite array sequence" refer to a sequence, hypothetical or otherwise, consisting of a head-to-tail (5' to 3') linear composite of all individual contiguous sequences of a subject array (e.g., a head-to-tail composite of SEQ ID NOS:1-71, in that order).

The terms "array SEQ ID NO node," "composite array SEQ ID NO node," or "composite array sequence node" refer to a *junction* between any two individual contiguous sequences of the "array SEQ ID NO," the "composite array SEQ ID NO," or the "composite array sequence."

In reference to composite array sequences, the phrase "contiguous nucleotides" refers to a contiguous sequence region of any individual contiguous sequence of the composite array, but does not include a region of the composite array sequence that includes a "node," as defined herein above.

### Overview:

Described herein are molecular genetic markers that have novel utility for the analysis of methylation patterns associated with the development of colorectal cell proliferative disorders. Said markers may be used for detecting or distinguishing between colorectal cell proliferative disorders, thereby providing improved means for the classification and treatment of said disorders.

*Bisulfite modification of DNA is an art-recognized tool used to assess CpG methylation-status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, *e.g.,* PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyze individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (*e.g.,* Zeschnigk M, etal., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyze individual cytosine positions, or treated by enzymatic digestion (Xiong & Laird, Nucleic Acids Res., 25:2532-4, 1997). Detection by hybridization has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

The present disclosure provides for the use of the bisulfite technique, in combination with one or more methylation assays, for determination of methylation status of CpG dinuclotide sequences within a sequence according to SEQ ID NO: 7. According to the present disclosure this, determination has diagnostic and prognostic utility.

*Methylation Assay Procedures.* Various methylation assay procedures are known in the art, and can be used in conjunction with the present invention. These assays allow for determination of the methylation state of one or a plurality of CpG dinucleotides (*e.g.,* CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, e.g., the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534,1997).

*COBRA.* COBRA™ analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (*e.g*., as might be found in a typical COBRA™-based kit) for COBRA™ analysis may include, but are not limited to: PCR primers for specific gene (or bisulfite treated DNA sequence or CpG island); restriction enzyme and appropriate buffer; gene-hybridization oligo; control hybridization oligo; kinase labeling kit for oligo probe; and labeled nucleotides. Additionally, bisulfite conversion reagents may include: DNA denaturation buffer; sulfonation buffer; DNA recovery reagents or kits (*e.g*., precipitation, ultrafiltration, affinity column); desulfonation buffer; and DNA recovery components.

Preferably, assays such as "MethyLight™" (a fluorescence-based real-time PCR technique) (Eads et al., Cancer Res. 59:2302-2306, 1999), Ms-SNuPE™ (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

*MethyLight* ™. The MethyLight ™ assay is a high-throughput quantitative methylation assay that utilizes fluorescence-based real-time PCR (TaqMan ™) technology that requires no further manipulations after the PCR step (Eads et al., Cancer Res. 59:2302-2306, 1999). Briefly, the MethyLight ™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight ™ assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight ™ process can by used with a "TaqMan®" probe in the amplification process. For example, double-stranded genomic DNA is treated with sodium bisulfite and subjected to one of two sets of PCR reactions using TaqMan® probes; *e.g.,* with either biased primers and TaqMan® probe, or unbiased primers and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g*., as might be found in a typical MethyLight ™-based kit) for MethyLight ™ analysis may include, but are not limited to: PCR primers for specific gene (or bisulfite treated DNA sequence or CpG island); TaqMan® probes; optimized PCR buffers and deoxynucleotides; and Taq polymerase.

*Ms-SNuPE.* The Ms-SNuPE™ technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (*e.g*., microdissected pathology sections), and it avoids utilization of restriction enzymes for determining the methylation status at CpG sites.

Typical reagents (*e.g*., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (*e.g*., precipitation, ultrafiltration, affinity column); desulfonation buffer; and DNA recovery components.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (*e.g*., as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MCA.* The MCA technique is a method that can be used to screen for altered methylation patterns in genomic DNA, and to isolate specific sequences associated with these changes (Toyota et al., Cancer Res. 59:2307-12, 1999). Briefly, restriction enzymes with different sensitivities to cytosine methylation in their recognition sites are used to digest genomic DNAs from primary tumors, cell lines, and normal tissues prior to arbitrarily primed PCR amplification. Fragments that show differential methylation are cloned and sequenced after resolving the PCR products on high-resolution polyacrylamide gels. The cloned fragments are then used as probes for Southern analysis to confirm differential methylation of these regions. Typical reagents (*e.g.,* as might be found in a typical MCA-based kit) for MCA analysis may include, but are not limited to: PCR primers for arbitrary priming Genomic DNA; PCR buffers and nucleotides, restriction enzymes and appropriate buffers; gene-hybridization oligos or probes; control hybridization oligos or probes.

### Genomic Sequences According to SEQ ID NO:7, and Non-naturally Occurring Treated Variants Thereof According to SEQ ID NOS:316, 317, 432 and 433, were Determined to have Novel Utility for of the disclosed Detection, Classification and/or Treatment of Colorectral Cell Proliferative Disorders.

The disclosure provides a method for detecting and/or for detecting and distinguishing between or among colon cell proliferative disorders in a subject. Said method comprises the following steps: i) contacting genomic DNA isolated from bodily fluids obtained from the subject with at least one reagent, or series of reagents that distinguishes between methylated and non-methylated CpG dinucleotides within at least one target region of the genomic DNA, wherein said contiguous nucleotides comprise at least one CpG dinucleotide sequence; and ii) detecting, or detecting and distinguishing between or among colon cell proliferative disorders afforded with a sensitivity of greater than or equal to 80% and a specificity of greater than or equal to 80%.

Preferably, the sensitivity is from about 75% to about 96%, or from about 80% to about 90%, or from about 80% to about 85%. Perferably, the specificity is is from about 75% to about 96%, or from about 80% to about 90%, or from about 80% to about 85%.

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, *e.g.,* by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. Body fluids are the preferred source of the DNA; particularly preferred are blood plasma, blood serum, whole blood, isolated blood cells and cells isolated from the blood.

The genomic DNA sample is then treated in such a manner that cytosine bases which are unmethylated at the 5'-position are converted to uracil, thymine, or another base which is dissimilar to cytosine in terms of hybridization behavior. This will be understood as 'treatment' herein.

The above described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

The treated DNA is then analysed in order to determine the methylation state of one or more target gene sequences (prior to the treatment) associated with the development of colorectal carcinoma. It is particularly preferred that the target region comprises, or hybridizes under stringent conditions to at least 16 contiguous nucleotides of the NGFR gene. It is further preferred that the sequences of said genes in Table 3 as described in the accompanying sequence listing are analysed. The method of analysis may be selected from those known in the art, including those listed herein. Particularly preferred are MethyLight, MSP and the use of blocking oligonucleotides as will be described herein. It is further preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID NO: (IDBISEQFIRST) to SEQ ID NO: and (IDBISEQLAST) and sequences complementary thereto.

Aberrant methylation, more preferably hypermethylation of DUX4 are associated with the presence of colorectal carcinoma.

Analysis of one or a plurality of the sequences enables for the first time detecting, or detecting and distinguishing between or among colon cell proliferative disorders afforded with a sensitivity of greater than or equal to 80% and a specificity of greater than or equal to 80%. Sensitivity is calculated as: {detected colon neoplasia/all colon neoplasia); and specificity is calculated as (non-detected negatives/total negatives).

Preferably, the sensitivity is from about 75% to about 96%, or from about 80% to about 90%, or from about 80% to about 85%. Perferably, the specificity is is from about 75% to about 96%, or from about 80% to about 90%, or from about 80% to about 85%.

Colon neoplasia can be defined as all colon malignancies or colon malignancies and large (> 1 cm adenomas), or subsets thereof. Negatives can be defined as any disease other than colon malignancy and adenomas, or healthy individuals.

Disclosed is the use of NGFR as markers for the differentiation, detection and distinguishing of colon cell proliferative disorders.

Said use of the gene may be enabled by means of any analysis of the expression of the gene, by means of mRNA expression analysis or protein expression analysis. However, as particularly disclosed herein, the detection, differentiation and distinguishing of colon cell proliferative disorders is enabled by means of analysis of the methylation status of and its promoter or regulatory elements. Methods for the methylation analysis of genes are described herein.

Aberrant levels of mRNA expression NGFR are associated with colorectal carcinoma.

Accordingly, increased or decreased levels of expression of said genes or sequences are associable with the development of colorectal carcinoma and other colorectal cell proliferative disorders.

To detect the presence of mRNA encoding a gene or genomic sequence in a detection system for colon cancer, a sample is obtained from a patient. The sample can be a tissue biopsy sample or a sample of blood, plasma, serum or the like. The sample may be treated to extract the nucleic acids contained therein. The resulting nucleic acid from the sample is subjected to gel electrophoresis or other separation techniques. Detection involves contacting the nucleic acids and in particular the mRNA of the sample with a DNA sequence serving as a probe to form hybrid duplexes. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2 d ed., 1989). Detection of the resulting duplex is usually accomplished by the use of labelled probes. Alternatively, the probe may be unlabeled, but may be detectable by specific binding with a ligand which is labelled, either directly or indirectly. Suitable labels and methods for labelling probes and ligands are known in the art, and include, for example, radioactive labels which may b e incorporated by known methods (*e.g.,* nick translation or kinasing),biotin, fluorescent groups, chemiluminescent groups (*e.g.,* dioxetanes, particularly triggered dioxetanes), enzymes, antibodies, and the like.

In order to increase the sensitivity of the detection in a sample of mRNA transcribed from the gene or genomic sequence, the technique of reverse transcription/polymerisation chain reaction can be used to amplify cDNA transcribed from the mRNA. The method of reverse transcription /PCR is well known in the art (for example, see Watson and Fleming, *supra*).

The reverse transcription /PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end primer. Typically, the primer contains an oligo(dT) sequence. The cDNA thus produced is then amplified using the PCR method and EYA4 specific primers. (Belyavsky et al, Nucl Acid Res 17:2919-2932, 1989; Krug and Berger, Methods in Enzymology, Academic Press,N.Y., Vo1.152, pp. 316-325, 1987).

The present invention may also be described in certain embodiments as the use of a kit for use in detecting a colon cancer disease state through testing of a biological sample. A representative kit may comprise one or more nucleic acid segments that selectively hybridise to the mRNA and a container for each of the one or more nucleic acid segments. In certain embodiments the nucleic acid segments may be combined in a single tube. In further embodiments, the nucleic acid segments may also include a pair of primers for amplifying the target mRNA. Such kits may also include any buffers, solutions, solvents, enzymes, nucleotides, or other components for hybridisation, amplification or detection reactions. Preferred kit components include reagents for reverse transcription-PCR, in situ hybridisation, Northern analysis and/or RPA

The present invention further provides for methods to detect the presence of the polypeptide encoded by NGFR in a sample obtained from a patient.

Aberrant levels of polypeptide expression of the polypeptides encoded by according to Table 3 are associated with colorectal carcinoma.

Accordingly over or under expression of said polypeptides is associable with the development of colorectal carcinoma and other colorectal cell proliferative disorders.

Any method known in the art for detecting proteins can be used. Such methods include, but are not limited to immunodiffusion, immunoelectrophoresis, immunochemical methods, binder-ligand assays, immunohistochemical techniques, agglutination and complement assays. (*e.g.,* see Basic and Clinical Immunology, Sites and Terr, eds., Appleton & Lange, Norwalk, Conn. pp 217-262, 1991). Preferred are binder-ligand immunoassay methods including reacting antibodies with an epitope or epitopes and competitively displacing a labelled protein or derivative thereof.

Certain embodiments of the present disclosure comprise the use of antibodies specific to the polypeptide encoded by NGFR.

Such antibodies may be useful for diagnostic and prognostic applications in detecting the disease state, by comparing a patient's levels of colon disease marker expression to expression of the same markers in normal individuals. In certain embodiments production of monoclonal or polyclonal antibodies can be induced by the use of the coded polypeptide as antigene. Such antibodies may in turn be used to detect expressed proteins as markers for human disease states. The levels of such proteins present in the peripheral blood or tissue sample of a patient may be quantified by conventional methods. Antibody-protein binding may be detected and quantified by a variety of means known in the art such as labelling with fluorescent or radioactive ligands. The invention further comprises the use of kits for performing the above-mentioned procedures, wherein such kits contain antibodies specific for the investigated polypeptides.

Numerous competitive and non-competitive protein binding immunoassays are well known in the art. Antibodies employed in such assays may be unlabeled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like for use in radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

One approach for preparing antibodies to a protein is the selection and preparation of an amino acid sequence of all or part of the protein, chemically synthesising the sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981). Methods for preparation of the polypeptides or epitopes thereof include, but are not limited to chemical synthesis, recombinant DNA techniques or isolation from biological samples.

Particular embodiments of the present invention provide a novel application of the analysis of methylation levels and/or patterns within said sequences that enables a precise detection, characterisation and/or treatment of colorectal cell proliferative disorders. Early detection of colorectal cell proliferative disorders is directly linked with disease prognosis, and the disclosed method thereby enables the physician and patient to make better and more informed treatment decisions.

### FURTHER IMPROVEMENTS

The present disclosure provides novel uses for genomic sequences according to SEQ ID NO: 7. Additional embodiments provide oligonucleotides and/or PNA-oligomers for analysis of cytosine methylation patterns within SEQ ID NO: 7.

The disclosure provides treated nucleic acids, derived from genomic SEQ ID NO: 7, wherein the treatment is suitable to convert at least one unmethylated cytosine base of the genomic DNA sequence to uracil or another base that is detectably dissimilar to cytosine in terms of hybridization. The genomic sequences in question may comprise one, or more, consecutive or random methylated CpG positions. Said treatment preferably comprises use of a reagent selected from the group consisting of bisulfite, hydrogen sulfite, disulfite, and combinations thereof. In a preferred aspect, the objective comprises analysis of a non-naturally occurring modified nucleic acid comprising a sequence of at least 16 contiguous nucleotide bases in length of a sequence selected from the group consisting of SEQ ID NOS:316, 317, 432 and 433, wherein said sequence comprises at least one CpG, TpA or CpA dinucleotide and sequences complementary thereto. The sequences of SEQ ID NOS:316, 317, 432 and 433 provide non-naturally occurring modified versions of the nucleic acid according to SEQ ID NO:7, wherein the modification of each genomic sequence results in the synthesis of a nucleic acid having a sequence that is unique and distinct from said genomic sequence as follows. For each sense strand genomic DNA, e.g., SEQ ID NO:1, four converted versions are disclosed. A first version wherein "C" →"T," but "CpG" remains "CpG" (*i.e.,* corresponds to case where, for the genomic sequence, all "C" residues of CpG dinucleotide sequences are methylated and are thus not converted); a second version discloses the complement of the disclosed genomic DNA sequence (i.e. *antisense* strand), wherein "C" →"T," but "CpG" remains "CpG" (*i.e.,* corresponds to case where, for all "C" residues of CpG dinucleotide sequences are methylated and are thus not converted). The 'upmethylated' converted sequences of SEQ ID NO:7 correspond to SEQ ID NOS:316 and 317. A third chemically converted version of each genomic sequences is provided, wherein "C" →"T" for all "C" residues, including those of "CpG" dinucleotide sequences (*i.e.,* corresponds to case where, for the genomic sequences, all "C" residues of CpG dinucleotide sequences are *un*methylated); a final chemically converted version of each sequence, discloses the complement of the disclosed genomic DNA sequence (i.e. *antisense* strand), wherein "C" →"T" for all "C" residues, including those of "CpG" dinucleotide sequences (*i.e.,* corresponds to case where, for the complement (*antisense* strand) of each genomic sequence, all "C" residues of CpG dinucleotide sequences are *un*methylated). The 'downmethylated' converted sequences of SEQ ID NO:7 correspond to SEQ ID NOS:432 and 433.

In an alternative preferred embodiment, such analysis comprises the use of an oligonucleotide or oligomer for detecting the cytosine methylation state within genomic or treated (chemically modified) DNA, according to SEQ ID NOS:7, 316, 317, 432 and 433. Said oligonucleotide or oligomer comprising a nucleic acid sequence having a length of at least nine (9) nucleotides which hybridizes, under moderately stringent or stringent conditions (as defined herein above), to a treated nucleic acid sequence according to SEQ ID NOS:316, 317, 432 and 433 and/or sequences complementary thereto, or to a genomic sequence according to SEQ ID NOS:7 and/or sequences complementary thereto.

Thus, the present disclosure includes nucleic acid molecules (*e.g.,* oligonucleotides and peptide nucleic acid (PNA) molecules (PNA-oligomers)) that hybridize under moderately stringent and/or stringent hybridization conditions to all or a portion of the sequences SEQ ID NOS:7, 316, 317, 432 and 433 or to the complements thereof. The hybridizing portion of the hybridizing nucleic acids is typically at least 9, 15, 20, 25, 30 or 35 nucleotides in length. However, longer molecules have utility.

Preferably, the hybridizing portion of the inventive hybridizing nucleic acids is at least 95%, or at least 98%, or 100% identical to the sequence, or to a portion thereof of SEQ ID NOS:7, 316, 317, 432 and 433 or to the complements thereof.

Hybridizing nucleic acids of the type described herein can be used, for example, as a primer (*e.g.,* a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO:7 (such as allelic variants and SNPs), rather than identical, it is useful to first establish the lowest temperature at which only homologous hybridization occurs with a particular concentration of salt (*e.g.,* SSC or SSPE). Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Preferably, the set is limited to those oligomers that comprise at least one CpG, TpG or CpA dinucleotide.

Described are, for *each* of SEQ ID NOS:7, 316, 317, 432 and 433 (sense and antisense), multiple consecutively overlapping sets of oligonucleotides or modified oligonucleotides of length X, where, *e.g.,* X= 9, 10, 17, 20, 22, 23, 25, 27, 30 or 35 nucleotides.

The oligonucleotides or oligomers onstitute effective tools useful to ascertain genetic and epigenetic parameters of the genomic sequence corresponding to SEQ ID NO:7. Preferred sets of such oligonucleotides or modified oligonucleotides of length X are those consecutively overlapping sets of oligomers corresponding to SEQ ID NOS:7, 316, 317, 432 and 433 (and to the complements thereof). Preferably, said oligomers comprise at least one CpG, TpG or CpA dinucleotide.

Particularly preferred oligonucleotides or oligomers are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, *e.g*., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers are typically used in 'sets,' which contain at least one oligomer for analysis of each of the CpG dinucleotides of genomic sequences SEQ ID NO:7 and sequences complementary thereto, or to the corresponding CpG, TpG or CpA dinucleotide within a sequence of the treated nucleic acids according to SEQ ID NOS:304 to SEQ ID NO:535 and sequences complementary thereto. However, it is anticipated that for economic or other factors it may be preferable to analyze a limited selection of the CpG dinucleotides within said sequences, and the content of the set of oligonucleotides is altered accordingly.

Therefore, in particular embodiments, the present disclosure provides a set of at least two (2) (oligonucleotides and/or PNA-oligomers useful for detectin the cytosine methylation state in treated genomic DNA (SEQ ID NOS:316, 317, 432 and 433 or in genomic DNA (SEQ ID NO:7 and sequences complementary thereto). These probes enable diagnosis, classification and/or therapy of genetic and epigenetic parameters of colorectal cell proliferative disorders. The set of oligomers may also be used for detectin single nucleotide polymorphisms (SNPs) in treated genomic DNA (SEQ ID NOS:316, 317, 432 and 433 or in genomic DNA (SEQ ID NO:7 and sequences complementary thereto).

In preferred embodiments, at least one, and more preferably all members of a set of oligonucleotides is bound to a solid phase.

In further embodiments, the present disclosure provides a set of at least two (2) oligonucleotides that are used as 'primer' oligonucleotides for amplifying DNA sequences of one of SEQ ID NOS:7, 316, 317, 432 and 433 and sequences complementary thereto, or segments thereof.

It is anticipated that the oligonucleotides may constitute all or part of an "array" or "DNA chip" (*i.e.,* an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labeled probes are often used for the scanning of immobilized D NA arrays. The simple attachment of Cy3 a nd C y5 d yes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091. In such methods, enough labels are generated so that each nucleic acid in the complex mixture (*i.e.,* each analyte) can be uniquely bound by a unique label and thus detected (each label is directly counted, resulting in a digital read-out of each molecular species in the mixture).

It is particularly preferred that the oligomers according to the invention are utilised for at least one of: detection of; detection and differentiation between or among subclasses of; diagnosis of; prognosis of; treatment of; monitoring of; and treatment and monitoring of colorectal cell proliferative disorders. This is enabled by use of said sets for the detection or detection and differentiation of one or more of the following classes of tissues: colorectal carcinoma, colon adenoma, inflammatory colon tissue, grade 2 dysplasia colon adenomas less than 1 cm, grade 3 dysplasia colon adenomas larger than 1 cm, normal colon tissue, non-colon healthy tissue and non-colon cancer tissue.

Particularly preferrred are those sets of oligomer relating to SEQ ID NO: 7 (see Table 2) that comprise at least two oligonucleotides selected from one of the following sets of oligonucleotides:
SEQ ID NOS:770-777;

At least one of colorectal carcinoma tissue or colon adenomas may be distinguished from at least one tissue selected from the group consisting of inflammatory colon tissue and normal colon tissue. This is achieved by analysis of the methylation status of at least one target sequence comprising, or hybridizing under stringent conditions to at least 16 contiguous nucleotides of a SEQ ID NO:7 or a complements thereof. This is preferrably achieved by use of a set consisting of at least one oligonucleotide, and more preferably at least two selected from one of the groups consisting of, or by use of a set comprising of at least two oligonucleotides selected from one of the groups consisting of:
SEQ ID NOS:770-777

Colorectal carcinoma may be distinguished from at least one tissue selected from the group consisting of non-colon healthy tissue, peripheral blood lymphocytes and non-colon cancer. In one embodiment this is achieved by analysis of the methylation status of at least one target sequence comprising, or hybridizing under stringent conditions to at least 16 contiguous nucleotides of SEQ ID NO:7. This is preferrably achieved by use of a set consisting of at least one oligonucleotide, and more preferably at least two oligonucleotides selected from one of the groups consisting of:
SEQ ID NOS: 770-777

Colorectal carcinoma may be distinguished from at least one tissue selected from the group consisting of inflammatory colon tissue, normal colon tissue, non-colon healthy tissue, peripheral blood lymphocytes, colon adenomas and non-colon cancer tissue. This is achieved by analysis of the methylation status of at least one target sequence comprising, or hybridizing under stringent conditions to at least 16 contiguous nucleotides of a SEQ ID NO: 7 or a complements thereof. This is preferably achieved by use of a set consisting of at least one oligonucleotide, and more preferably at least two by use of a set of oligomers comprising at least two oligonucleotides selected from one of the groups consisting of:
SEQ ID NOS:770-777

At least one of colorectal carcinoma tissue, or colon adenomas may be distinguished from at least one tissue selected from the group consisting of inflammatory colon tissue and normal colon tissue. This is achieved by analysis of the methylation status of at least one target sequence comprising, or hybridizing under stringent conditions to at least 16 contiguous nucleotides of SEQ ID NO: 7 or a complements thereof. This is preferably achieved by use of a set consisting of at least one oligonucleotide, and more preferably at least two oligomers comprising at least two oligonucleotides selected from one of the groups consisting of:
SEQ ID NOS: 770-777

Colorectal carcinoma tissue may be distinguished from at least one of inflammatory colon tissue and normal colon tissue. This is achieved by analysis of the methylation status of at least one target sequence comprising, or hybridizing under stringent conditions to at least 16 contiguous nucleotides of SEQ ID NO:7 or a complements thereof. This is preferably achieved by use of a set consisting of at least one oligonucleotide, and more preferably at least two oligomers comprising at least two oligonucleotides selected from one of the groups consisting of:
SEQ ID NOS: 770-777

At least one of colorectal carcinoma tissue, or colon adenomas may be distinguished from at least one tissue selected from the group consisting of inflammatory colon tissue, normal colon tissue, non-colon healthy tissue, peripheral blood lymphocytes, and non-colon cancer tissue. This is achieved by analysis of the methylation status of at least one target sequence comprising, or hybridizing under stringent conditions to at least 16 contiguous nucleotides of SEQ ID NO:7 or a complements thereof. This is preferably achieved by use of a set consisting of at least one oligonucleotide, and more preferably at least two oligomers comprising at least two oligonucleotides selected from one of the groups consisting of:
SEQ IDNOS: 770-777

Tissues originating from the colon may be distinguished from tissues of non-colon origin by use of a set of oligomers comprising at least two oligonucleotides selected from one of the groups consisting of:
SEQ ID NOS: 770-777

Described is a method for ascertaining genetic and/or epigenetic parameters of the genomic sequences according to SEQ ID NO:7 within a subject by analyzing cytosine methylation and single nucleotide polymorphisms. Said method comprising contacting a nucleic acid comprising one or more of SEQ ID NO:7 in a biological sample obtained from said subject with at least one reagent or a series of reagents, wherein said reagent or series of reagents, distinguishes between methylated and non-methylated CpG dinucleotides within the target nucleic acid.

Preferably, said method comprises the following steps: In the *first step*, a sample of the tissue to be analysed is obtained. The source may be any suitable source, such as cell lines, histological slides, biopsies, paraffin-embedded tissue, bodily fluids, stool, colonic effluent, urine, blood plasma, blood serum, whole blood, isolated blood cells, cells isolated from the blood and all possible combinations thereof. It is preferred that said sources of DNA are stool or bodily fluids selected from the group consisting colonic effluent , urine, blood plasma, blood serum, whole blood, isolated blood cells, cells isolated from the blood.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Once the nucleic acids have been extracted, the genomic double stranded DNA is used in the analysis.

In the *second step* of the method, the genomic DNA sample is treated in such a manner that cytosine bases which are unmethylated at the 5'-position are converted to uracil, thymine, or another base which is dissimilar to cytosine in terms of hybridization behavior. This will be understood as 'pretreatment' or 'treatment' herein.

The above-described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

In the *third step* of the method, fragments of the treated DNA are amplified, using sets of primer oligonucleotides disclosed herein, and an amplification enzyme. The amplification of several DNA segments can be carried out simultaneously in one and the same reaction vessel. Typically, the amplification is carried out using a polymerase chain reaction (PCR). The set of primer oligonucleotides includes at least two oligonucleotides whose sequences are each reverse complementary, identical, or hybridize under stringent or highly stringent conditions to an at least 16-base-pair long segment of the base sequences of one of SEQ ID NOS:316, 317, 432 and 422 and sequences complementary thereto.

Alternatively the methylation status of preselected CpG positions within the nucleic acid sequences comprising SEQ ID NO:7 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridizes to a treated nucleic acid sequence according to one of SEQ ID NOS:316, 317, 432 and 422 and sequences complementary thereto, wherein the base sequence of said oligomers comprises at least one CpG dinucleotide.

A further preferred embodiment of the method comprises the use of *blocker* oligonucleotides. The use of such blocker oligonucleotides has been described by Yu et al., BioTechniques 23:714-720, 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (*e.g*., with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridizes to a treated nucleic acid sequence according to one of SEQ ID NOS:316, 317, 432 and 422 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. W here said labels are mass labels, it is preferred that the labeled amplificates have a single positive or negative net charge, allowing for better detectability in the mass spectrometer. The detection may be carried out and visualized by means of, *e.g.,* matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapour phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of hybridization-based methods such as, but not limited to, array technology and probe based technologies as well as by means of techniques such as sequencing and template directed extension.

In one embodiment of the method, the amplificates synthesised in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG, TpG or CpA dinucleotide.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within the sequence according to SEQ ID NO:7, and the equivalent positions within SEQ ID NOS: 316, 317, 432 and 422. Said oligonucleotides may also be present in the form of peptide nucleic acids. The non-hybridized amplificates are then removed. The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also see* United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a nonextendible interrogating oligonucleotide, called a TaqMan™ probe, which, in preferred embodiments, is designed to hybridize to a GpC-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, also known as the MethylLight™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

A further suitable method for the use of probe oligonucleotides for the assessment of methylation by analysis of bisulfite treated nucleic acids In a further preferred embodiment of the method, the *fifth step* of the method comprises the use of template-directed oligonucleotide extension, such as MS-SNuPE as described by Gonzalgo & Jones, Nucleic Acids Res. 25:2529-2531,1997.

In yet a further embodiment of the method, the *fifth step* of the method comprises sequencing and subsequent sequence analysis of the amplificate generated in the *third step* of the method (Sanger F., et al., Proc Natl Acad Sci USA 74:5463-5467, 1977).

### Best mode

In the most preferred embodiment of the method the genomic nucleic acids are isolated and treated according to the first three steps of the method outlined above, namely:
a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides,* and further wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NOS:316, 317,432 or 433 and sequences complementary t hereto, wherein the base sequence of said oligomers comprises at least one CpG dinucleotide.

Step e) of the method, namely the detection of the specific amplificates indicative of the methylation status of one or more CpG positions according to SEQ ID NO: 7 is carried out by means of *real-time* detection methods as described above, and wherein the sequence of said hybridization probes is selected from the sequences specified below for SEQ ID NO: 7 detection the following oligonucleotides as detailed in the sequence listing may be used for a combined MSP-RealTime analysis:
SEQ ID NO: 7
   left Primer: 4649, 4652, 4655, 4659, 4661, 4663, 4664, 4665, 4668, 4671, 4674, 4675, 4676, 4678, 4680, 4683, 4686, 4690, 4696, 4700, 4703, 4704, 4706, 4709, 4711, 4714, 4715, 4717, 4718, 4719, 4720, 4721, 4726, 4727, 4730, 4732, 4738, 4742, 4745, 4749, 4750, 4753, 4755, 4759, 4763, 4768, 4769, 4772, 4773, 4776, 4778, 4779, 4782, 4784, 4789, 4790, 4794, 4797, 4801, 4805, 4807, 4813, 4814, 4819, 4821, 4822, 4827, 4829, 4833, 4834, 4835, 4837, 4838, 4841, 4842, 4843, 4844, 4847, 4849, 4850, 4852, 4853, 4859, 4860, 4861, 4865, 4867, 4868, 4869, 4870, 4873, 4876, 4877, 4878, 4879, 4883, 4885, 4889, 4891, 4892, 4894, 4895, 4897, 4898, 4903, 4906, 4911, 4912, 4918, 4919, 4920, 4924, 4927, 4929, 4932, 4934, 4941, 4942, 4945, 4948, 4949, 4950,
   right Primer: 4650, 4653, 4656, 4658, 4660, 4662, 4666, 4667, 4669, 4670, 4672, 4677, 4681, 4684, 4685, 4687, 4688, 4689, 4691, 4693, 4694, 4695, 4697, 4699, 4701, 4702, 4705, 4707, 4708, 4716, 4722, 4724, 4728, 4731, 4733, 4735, 4736, 4737, 4739, 4741, 4744, 4746, 4748, 4751, 4754, 4756, 4758, 4760, 4761, 4764, 4766, 4767, 4770, 4771, 4774, 4777, 4780, 4783, 4785, 4786, 4787, 4788, 4791, 4792, 4793, 4795, 4798, 4800, 4802, 4804, 4806, 4808, 4810, 4811, 4812, 4815, 4816, 4817, 4818, 4820, 4823, 4824, 4825, 4828, 4830, 4832, 4836, 4839, 4840, 4845, 4846, 4848, 4851, 4854, 4855, 4856, 4857, 4858, 4862, 4864, 4871, 4872, 4874, 4875, 4881, 4882, 4884, 4886, 4887, 4890, 4893, 4896, 4899, 4900, 4901, 4902, 4904, 4905, 4907, 4908, 4909, 4910, 4913, 4915, 4917, 4921, 4922, 4923, 4925, 4926, 4328, 4930, 4935, 4936, 4937, 4938, 4940, 4943, 4944, 4946, 4947, 4951, 4952,
   Detection: 4651, 4654,4657, 4673, 4679, 4682, 4692, 4698, 4710, 4712, 4713, 4723, 4725, 4729, 4734, 4740, 4743, 4747, 4752, 4757, 4762, 4765., 4775, 4781, 4796, 4799, 4803, 4809, 4826, 4831, 4863, 4866, 4880, 4888, 4914, 4916, 4931, 4933, 4939,
SEQ ID NO: 7
   left Primer: 12006, 12011, 12015, 12016, 12017, 12021, 12022, 12023, 12024, 12025, 12026, 12027, 12031, 12034, 12042, 12043, 12045, 12049, 12050, 12051, 12054, 12062, 12063, 12064, 12066, 12067, 12068, 12070, 12072, 12076, 12082, 12085, 12086, 12089, 12090, 12093, 12094, 12095, 12096, 12097, 12098, 12101, 12103, 12107, 12108, 12111, 12112, 12114, 12118, 12119, 12120, 12128,
   right Primer: 12007, 12010, 12012, 12020, 12028, 12032, 12033, 12035, 12038, 12039, 12044, 12046, 12052, 12055, 12056, 12057, 12058, 12059, 12060, 12061, 12065, 12069, 12071, 12073, 120T7, 12080, 12081, 12084, 12087, 12088, 12091, 12099, 12100, 12102, 12104, 12109, 12110, 12113, 12115, 12121, 12122, 12123, 12124, 12125, 12127, 12129,
   Detection: 12008, 12013, 12018, 12029, 12036, 12040, 12047, 12074, 12078, 12083, 12105, 12116,
   Anchor: 12009, 12014, 12019, 12030, 12037, 12041, 12048, 12053, 12075, 12079, 12036, 12092, 12106, 12117, 12126,

In an alternative most preferred embodiment of the method, the subsequent amplification of d) is carried out in the presence of *blocking oligonucleotides,* as described above. Said *blocking oligonucleotides* comprising a sequence having a length of at least 9 nucleotide which hybridizes to a treated nucleic acid sequence according to one of SEQ ID NOS: 316, 317, 432 or 433 and sequences complementary thereto, wherein the base sequence of said oligomers comprises at least one CpG, TpG or CpA dinucleotide. Step e) of the method, namely the detection of the specific amplificates indicative of the methylation status of one or more CpG positions according to SEQ ID NO:7 is carried out by means of real-time detection methods as described above.

Step e) of the method, namely the detection of the specific amplificates indicative of the methylation status of one or more CpG positions according to SEQ ID NO: 7 is carried out by means of real-time detection methods as described above, and wherein the sequence of said hybridization probes is selected from the group consisting of SEQ ID NOS: 1308 and 1309. the following oligonucleotides as detailed in the sequence listing may be used for a combined Blocking oligonucleotide-RealTime analysis: Lightcycler dual probe detection assays:
SEQ ID NO: 7
   left Primer: 1307, 1311,
   right Primer: 1306, 1312, 1313, 1314, 1323, 1326, 1329, 1331, 1333, 1335, 1336, Blocker: 1310, 1315, 1316, 1317, 1318, 1319, 1320, 1321, 1322, 1324, 1325, 1327, 1328, 1330, 1332, 1334, 1337, 1338,
   Detection: 1308,
   Anchor: 1309,

Additional embodiments of the invention provide a method for the analysis of the methylation status of genomic DNA as described herein (SEQ ID NO: 7, and complements thereof) without the need for pretreatment.

In the *first step* of such additional embodiments, the genomic DNA sample is isolated from tissue or cellular sources. Preferably, such sources include cell lines, histological slides, body fluids, or tissue embedded in paraffin. In the *second step,* the genomic DNA is extracted. Extraction may be by means that are standard to one skilled in the art, including but not limited to the use of detergent lysates, sonification and vortexing with glass beads. Once the nucleic acids have been extracted, the genomic double-stranded DNA is used in the analysis.

The DNA may be cleaved prior to the treatment, and this may be by any means standard in the state of the art, in particular with methylation-sensitive restriction endonucleases.

In the *third step,* the DNA is then digested with one or more methylation sensitive restriction enzymes. The digestion is carried out such that hydrolysis of the DNA at the restriction site is informative of the methylation status of a specific CpG dinucleotide.

In the *fourth step,* which is optional but preferred, the restriction fragments are amplified. This is preferably carried out using a polymerase chain reaction, and said amplificates may carry suitable detectable labels as discussed above, namely fluorophore labels, radionuclides and mass labels.

In the *fifth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis.

Subsequent to the determination of the methylation state of the genomic nucleic acids the presence, absence or subclass of colorectal cell proliferative disorder is deduced basedupon the methylation state of at least one CpG dinucleotide sequence of SEQ ID NO: 7, or an average, or a value reflecting an average methylation state of a plurality of CpG dinucleotide sequences of SEQ ID NO: 7.

### Diagnostic Assays for colorectal cell proliferative disorders

Described is a diagnosis of events which are disadvantageous to patients or individuals in which important genetic and/or epigenetic parameters within SEQ ID NO:7 may be used as markers. Said parameters obtained as described herein may be compared to another set of genetic and/or epigenetic parameters, the differences serving as the basis for a diagnosis and/or prognosis of events which are disadvantageous to patients or individuals.

More specifically the screening of at-risk populations for the early detection of colorectal carcinomas is described. Further embodiments of the method may also be used as alternatives to cytological screening for the differentation of colorectal carcinomas from other colon cell proliferative disorders, e.g. colon polyps.

Specifically, diagnostic cancer assays based on measurement of differential methylation of one or more CpG dinucleotide sequences of SEQ ID NO: 7 or of subregions thereof that comprise such a CpG dinucleotide sequence are described. Typically, such assays involve obtaining a tissue sample from a test tissue, performing an assay to measure the methylation status of at least one of one or more CpG dinucleotide sequences of SEQ ID NO:7 derived from the tissue sample, relative to a control sample, or a known standard and making a diagnosis or prognosis based thereon.

In particular preferred embodiments, oligomers are used to assess the CpG dinucleotide methylation status, such as those based on SEQ ID NO: 7, 316, 317, 432 and 433, or arrays thereof, as well as in kits based thereon and useful for the diagnosis and/or prognosis of colorectal cell proliferative disorders.

### Kits

Moreover, an additional aspect of the present invention is the use of kit comprising, for example: a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID NO:7, 316, 317, 432 and 433 oligonucleotides and/or PNA-oligomers; as well as instructions for carrying out and evaluating the described method. In a further preferred embodiment, said kit may further comprise standard reagents for performing a CpG position-specific methylation analysis, wherein said analysis comprises one or more of the following techniques: MS-SNuPE, MSP, MethyLight ™, HeavyMethyl™, COBRA, and nucleic acid sequencing. However, a kit along the lines of the present invention can also contain only part of the aforementioned components.

While the present invention has been described with specificity in accordance with certain of its preferred embodiments, the following examples serve only to illustrate the invention and are not intended to limit the invention, which his defined by the appended claims.

### EXAMPLES

Samples were received either as frozen tissue or extracted genomic DNA. DNA samples were extracted using lysis buffer from Qiagen and the Roche magnetic separation kit for genomic DNA isolation. DNA samples were also extracted using Qiagen Genomic Tip-100 columns, as well as the MagnaPure device and Roche reagents. All samples were quantitated using spectrophotometric or fluorometric techniques and on agarose gels for a subset of samples.

### Bisulfite treatment and mPCR

Total genomic DNA of all samples was bisulfite treated converting unmethylated cytosines to uracil. Methylated cytosines remained conserved. Bisulfite treatment was performed with minor modifications according to the protocol described in Olek et al. (1996). In order to avoid processing all samples with the same biological background together resulting in a potential process-bias in the data later on, the samples were randomly grouped into processing batches. For bisulfite treatment we created batches of 50 samples randomized for sex, diagnosis, and tissue. Per DNA sample two independent bisulfite reactions were performed. After bisulfitation 10 ng of each DNA sample was used in subsequent mPCR reactions containing 6-8 primer pairs.

Each reaction contained the following:
0.4 mM each dNTPS
1 Unit Taq Polymerase
2.5 µl PCR buffer
3.5 mM MgCl2
80 nM Primerset (12-16 primers)
11.25 ng DNA (bisulfite treated)
Further details of the primers are shown in TABLE 1.

Forty cycles were carried out as follows: Denaturation at 95°C for 15 min, followed by annealing at 55°C for 45 sec., primer elongation at 65°C for 2 min. A final elongation at 65°C was carried out for 10 min.

### 1.1.2 Hybridization

All PCR products from each individual sample were then hybridised to glass slides carrying a pair of immobilised oligonucleotides for each CpG position under analysis. Each of these detection oligonucleotides was designed to hybridise to the bisulphite converted sequence around one CpG site which was either originally unmethylated (TG) or methylated (CG). See Table 2 for further details of all hybridisation oligonucleotides used (both informative and non-informative.) Hybridisation conditions were selected to allow the detection of the single nucleotide differences between the TG and CG variants.

5 µl volume of each multiplex PCR product was diluted in 10 x Ssarc buffer (10 x Ssarc:230 ml 20 x SSC, 180 ml sodium lauroyl sarcosinate solution 20%, dilute to 1000 ml with dH2O). The reaction mixture was then hybridised to the detection oligonucleotides as follows. Denaturation at 95°C, cooling down to 10 °C, hybridisation at 42°C overnight followed by washing with 10 x Ssarc and dH2O at 42°C.

Further details of the hybridisation oligonucleotides are shown in TABLE 2.

Fluorescent signals from each hybridised oligonucleotide were detected u sing g enepix scanner and software. Ratios for the two signals (from the CG oligonucleotide and the TG oligonucleotide used to analyse each CpG position) were calculated based on comparison of intensity of the fluorescent signals.

The samples were processed in batches of 8 0 samples randomized for sex, diagnosis, tissue, and bisulphite batch For each bisulfite treated DNA sample 2 hybridizations were performed. This means that for each sample a total number of 4 chips were processed.

### Data analysis methods

### Analysis of the chip data:

From raw hybridization intensities to methylation ratios;
The log methylation ratio (log(CG/TG)) at each CpG position is determined according to a standardized preprocessing pipeline that includes the following steps:
   For each spot the median background pixel intensity is subtracted from the median foreground pixel intensity (this gives a good estimate of background corrected hybridization intensities):
      For both CG and TG detection oligonucleotides of each CpG position the background corrected median of the 4 redundant spot intensities is taken;
      For each chip and each CpG position the log(CG/TG) ratio is calculated;
      For each sample the median of log(CG/TG) intensities over the redundant chip repetitions is taken.

This ratio has the property that the hybridization noise has approximately constant variance over the full range of possible methylation rates (Huber et al., 2002).

### Principle Component Analysis

The principle component analysis (PCA) projects measurement vectors (*e.g*. chip data, methylation profiles on several CpGs etc.) onto a new coordinate system. The new coordinate axes are referred to as principal components. The first principal component spans the direction of the largest variance of the data. Subsequent components are ordered by decreasing variance and are orthogonal to each other. Different CpG positions contribute with different weights to the extension of the data cloud along different components. PCA is an unsupervised technique, *i.e.,* it does not take into account the labels of the data points (for further details see e.g. Ripley (1996)).

PCA is typically used to project high dimensional data (in our case methylation-array data) onto lower dimensional subspaces in order to visualize or extract features with high variance from the data. In the present report we use 2 dimensional projections for statistical quality control of the data. We investigate the effect of different process parameters on the chip data and exclude that changing process parameters cause large alterations in the measurement values.

A robust version of PCA is used to detect single outlier chips and exclude them from further analysis (Model et al., 2002).

### Hypothesis testing

The main task is to identify markers that show significant differences in the average degree of methylation between two classes. A significant difference is detected when the nullhypothesis that the average methylation of the two classes is identical can be rejected with p<0.05. Because we apply this test to a whole set of potential markers we have to correct the p-values for multiple testing. This was done by applying the False Discovery Rate (FDR) method (Dudoit et al., 2002).

For testing the null hypothesis that the methylation levels in the two classes are identical we u sed the likelihood ratio test for logistic r egression models (Venables and Ripley, 2 002). The logistic regression model for a single marker is a linear combination of methylation measurements from all CpG positions in the respective genomic region of interest (ROI). A significant p-value for a marker means that this ROI has some systematic correlation to the question of interest as given by the two classes. However, at least formally it makes no statement about the actual predictive power of the marker.

### Class prediction by supervised learning

In order to give a reliable estimate of how well the CpG ensemble of a selected marker can differentiate between different tissue classes we can determine its prediction accuracy by classification. For that purpose we calculate a methylation profile based prediction function using a certain set of tissue samples with their class label. This step is called training and it exploits the prior knowledge represented by the data labels. The prediction accuracy of that function is then tested by cross-validation or on a set of independent samples. As a method of choice, we use the support vector machine (SVM) algorithm (Duda (2001), Christiannini (2000)) to learn the prediction function. If not stated otherwise, for this report the risk associated with false positive or false negative classifications are set to be equal relative to the respective class sizes. It follows that the learning algorithm obtains a class prediction function with the objective to optimize accuracy on an independent test sample set. Therefore sensitivity and specificity of the resulting classifier can be expected to be approximately equal.

### Estimating the performance of the tissue class prediction: Cross Validation

With limited sample size the cross-validation method provides an effective and reliable estimate for the prediction accuracy of a discriminator function and therefore in addition to the significance of the markers we provide cross-validation accuracy, sensitivity and specificity estimates. For each classification task, the samples were partitioned into 5 groups of approximately equal size. Then the learning algorithm was trained on 4 of these 5 sample groups. The predictor obtained by this method was then tested on the remaining group of independent test samples. The number of correct positive and negative classifications was counted over 5 runs for the learning algorithm for all possible choices of the independent test group without using any knowledge obtained from the previous runs. This procedure was repeated on up to 10 random permutations of the sample set. Note that the above-described cross-validation procedure evaluates accuracy, sensitivity and specificity using practically all possible combinations of training and independent test sets. It therefore gives a better estimate of the prediction performance than simply splitting the samples into one training sample set and one independent test set.

### Results

For the results shown in Figures 1-12, 16, 17, 19-27, 29, 31 and 35, see the description of the figures above.

### Colon Normal vs. Colorectal Cancer

In the first comparison 102 colorectal carcinoma samples were compared to 73 samples from normal colon, including colon polyps and colon inflammatory disorders.
Figure 1 shows the multivariate test results of the top 12 performing markers using the conservative Bonferroni corrected LogReg test.
Figure 2 shows the uncorrected multivariate L ogReg test results for each comparison. Each individual genomic region of interest is represented as a point, the dotted line represents the cut off point for the 25% false discovery rate.
Figure 3 shows the accuracy of the top 12 performing markers.
Figure 4 shows the accuracy of the genewise linear support vector machine cross validation for all genomic regions of each classification.

Inter alia, the genomic marker according to SEQ ID NO: 7 was evaluated as being significant.

### Other Tissues vs. Colorectal Cancer

In this classification 73 colorectal carcinoma samples were compared to an 'other tissue' class consisting of 140 samples from non-colorectal carcinomas, peripheral blood lymphocytes and other normal tissues of non-colorectal origin. These markers therefore enable the detection of colorectal carcinoma cells in', for example, body fluids such as serum.

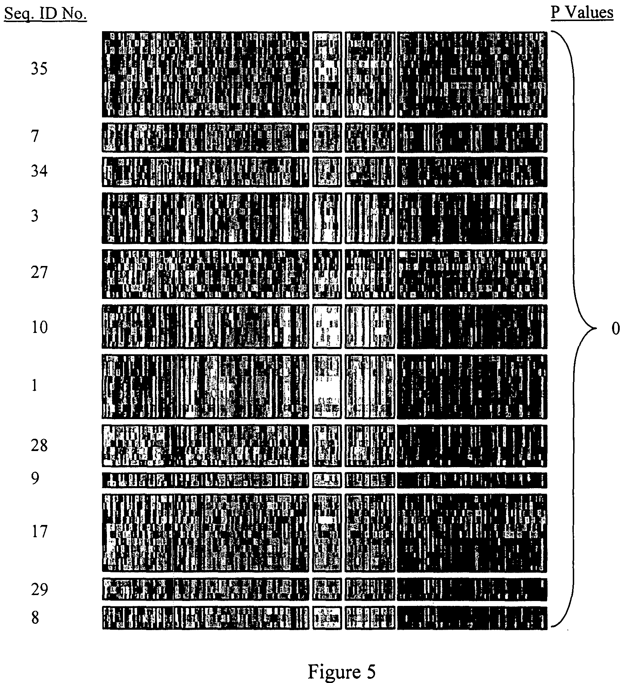

Figure 5 shows the multivariate test results of the top 12 performing markers using the conservative Bonferroni corrected LogReg test.

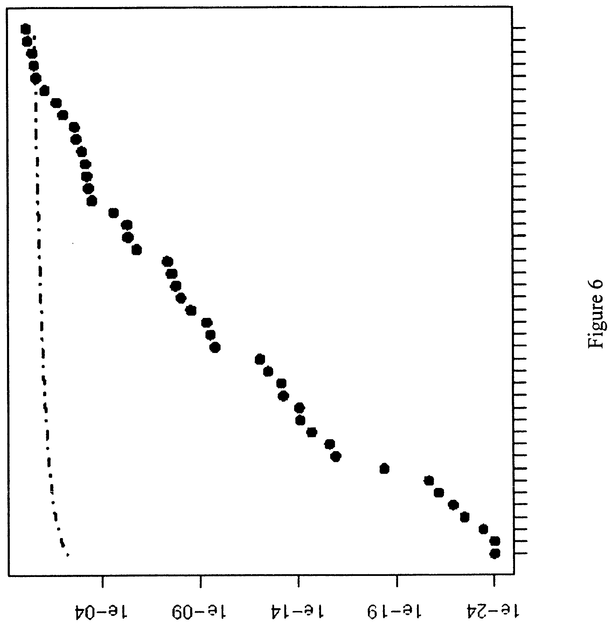

Figure 6 shows the uncorrected multivariate LogReg test results for each comparison. Each individual genomic region of interest is represented as a point, the dotted line represents the cut off point for the 25% false discovery rate.

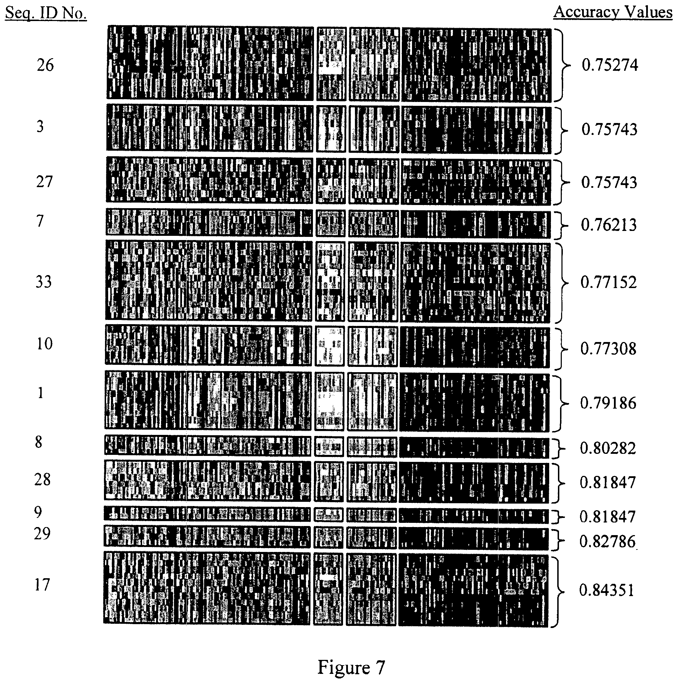

Figure 7 shows the accuracy of the top 12 performing markers.

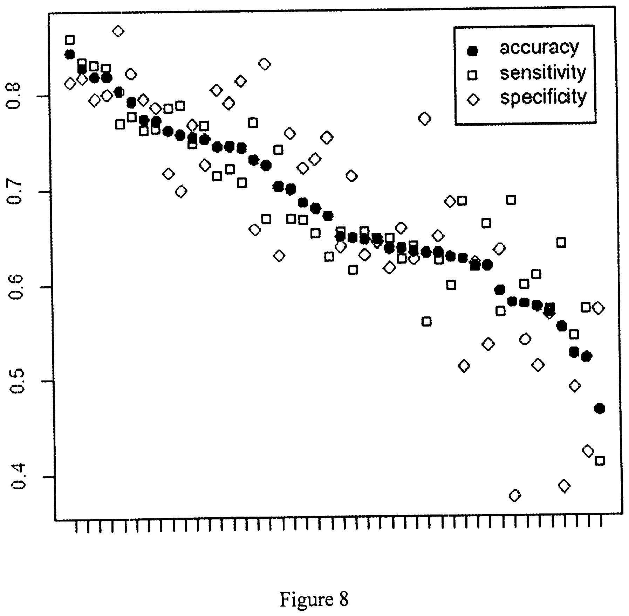

Figure 8 shows the accuracy of the genewise linear support vector machine cross validation for all genomic regions of each classification.

Inter alia, the genomic marker according to SEQ ID NO: 7 was evaluated as being significant.

### Colon Normal and Other Tissue vs. Colon Cancer

In this classification 73 colorectal carcinoma samples were compared to 242 colon normal and 'other tissue' samples. The colon normal class consisted of healthy colon, colon polyps and inflammatory disorder colon tissue samples, the 'other tissues' consisted of samples from non-colorectal carcinomas, peripheral blood lymphocytes and other normal tissues of non-colorectal origin.

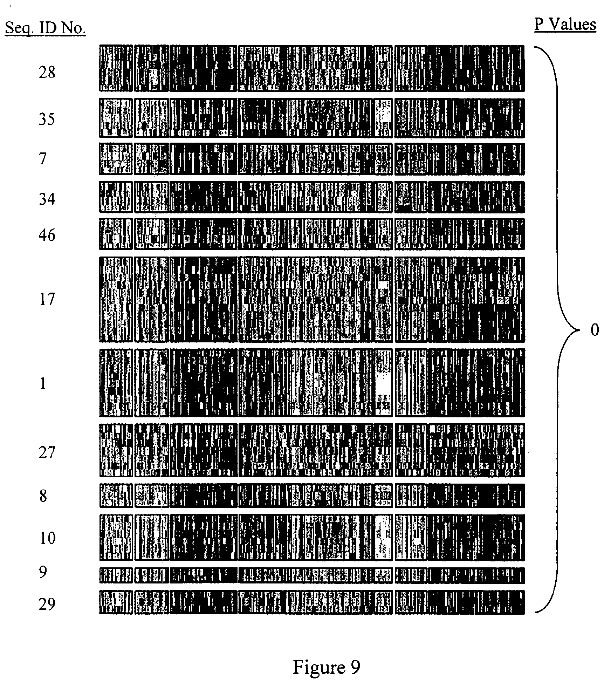

Figure 9 shows the multivariate test results of the top 12 performing markers using the conservative Bonferroni corrected LogReg test.

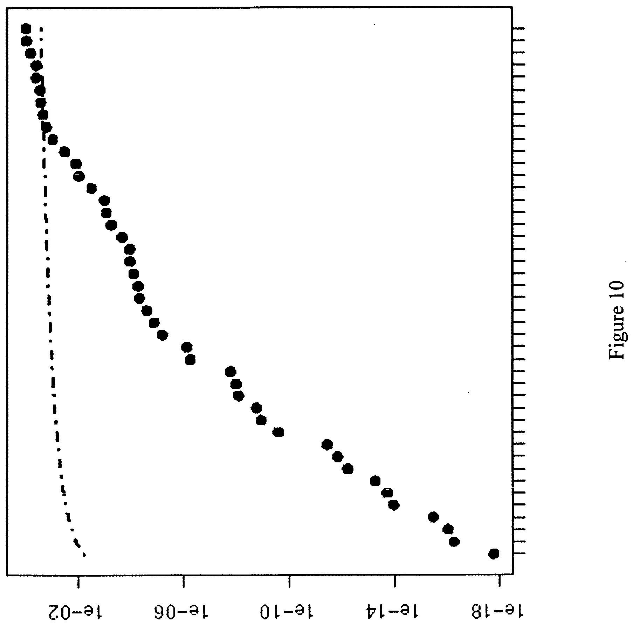

Figure 10 shows the uncorrected multivariate LogReg test results for each comparison. Each individual genomic region of interest is represented as a point, the dotted line represents the cut off point for the 25% false discovery rate.

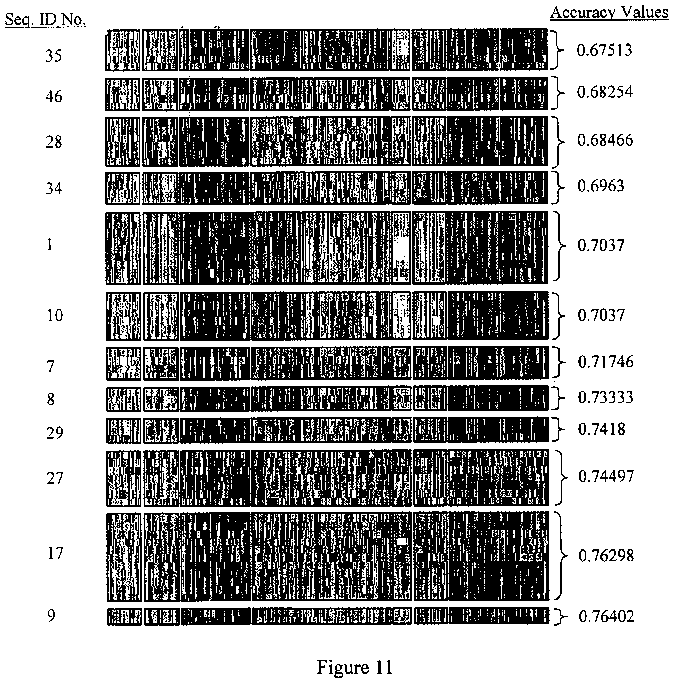

Figure 11 shows the accuracy of the top 12 performing markers.

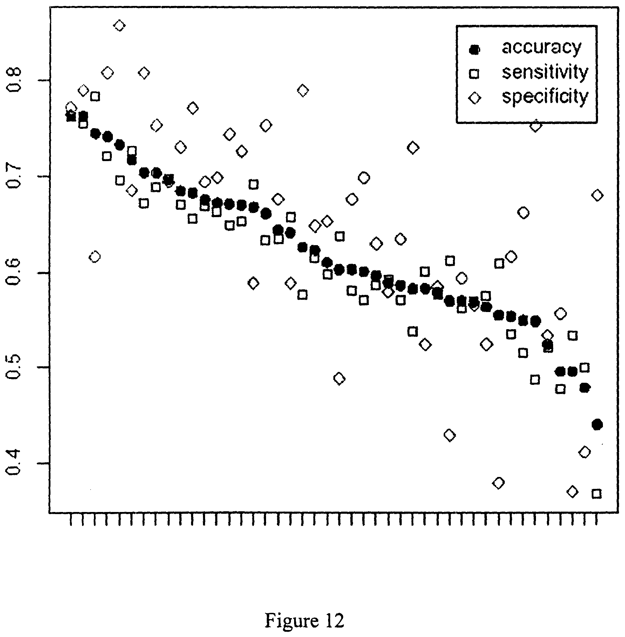

Figure 12 shows the accuracy of the genewise linear support vector machine cross validation for all genomic regions of each classification.

Inter alia, the genomic marker according to SEQ ID NO: 7 was evaluated as being significant.

Inter alia, the genomic marker according to SEQ ID NO: 7 was evaluated as being significant.

### Colon normal vs. Colorectal cell proliferative disorder

In this calssification 124 colorectal cell proliferative disorder samples (consisting of colon polyps and colorectal carcinoma) were compared to 51 'normal colon' samples consisting of both healthy colon samples and colon tissue of inflammatory disorders.

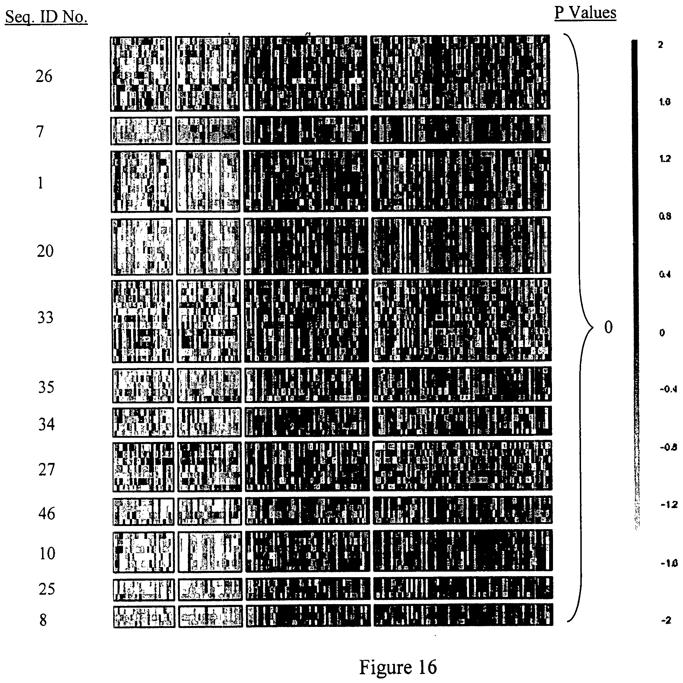

Figure 16 shows the multivariate test results of the top 12 performing markers using the conservative Bonferroni corrected LogReg test.

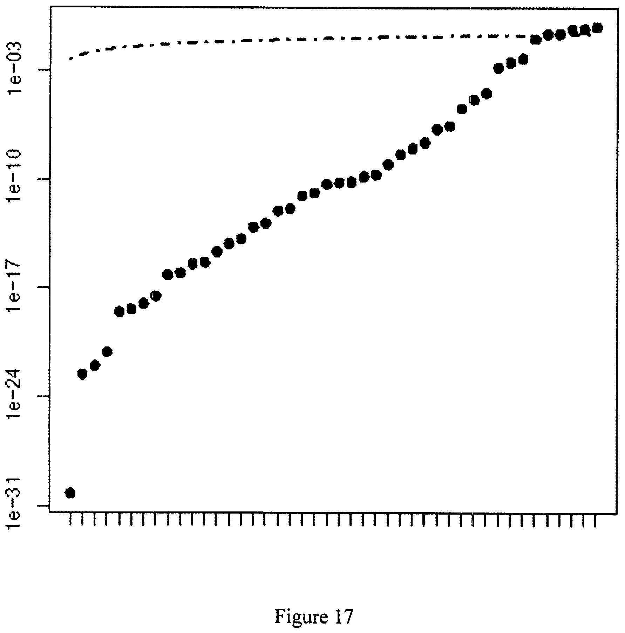

Figure 17 shows the uncorrected multivariate LogReg test results for each comparison. Each individual genomic region of interest is represented as a point, the dotted line represents the cut off point for the 25% false discovery rate.

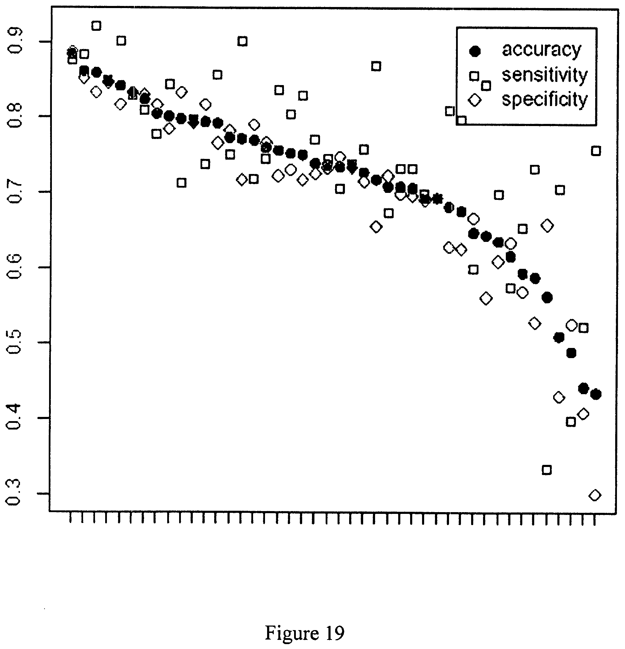

Figure 19 shows the accuracy of the genewise linear support vector machine cross validation for all genomic regions of each classification.

Inter alia, the genomic marker according to SEQ ID NO: 7 was evaluated as being significant.

### Colon Normal vs. Colorectal Cancer

In this classification 73 colorectal carcinoma samples were compared to 51 'normal colon' samples consisting of both healthy colon samples and colon tissue of inflammatory disorders.

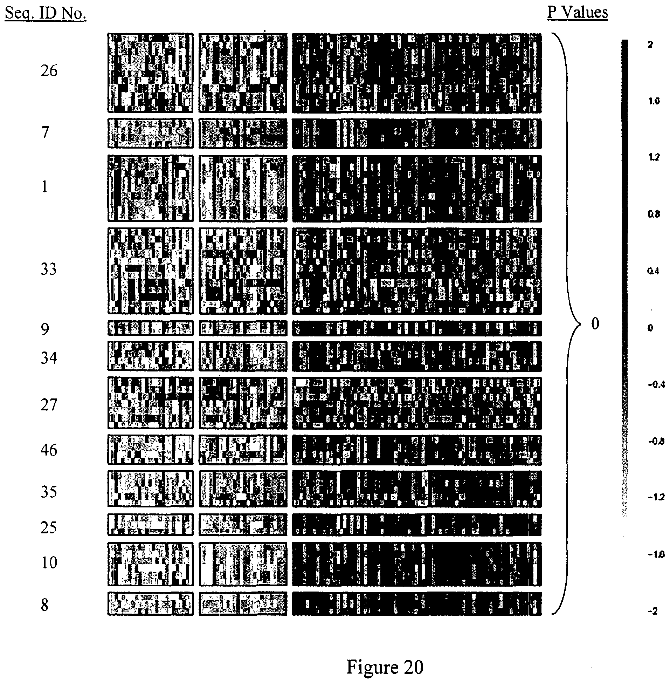

Figure 20 shows the multivariate test results of the top 12 performing markers using the conservative Bonferroni corrected LogReg test.

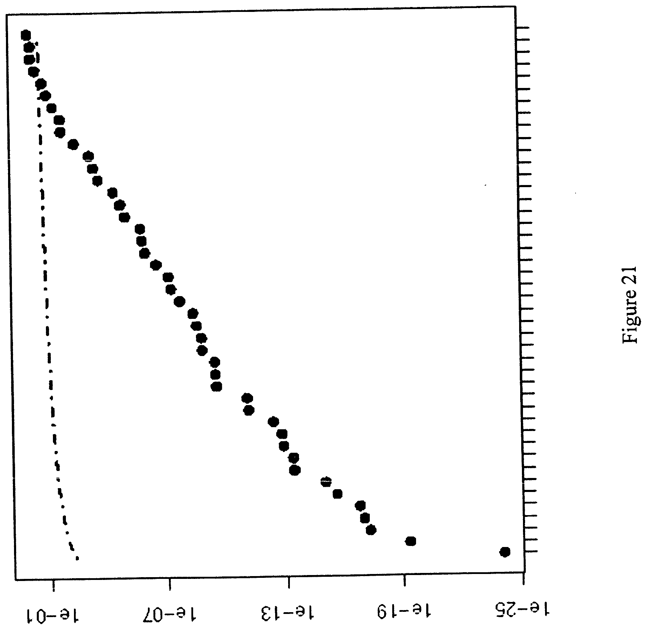

Figure 21 shows the uncorrected multivariate LogReg test results for each comparison. Each individual genomic region of interest is represented as a point, the dotted line represents the cut off point for the 25% false discovery rate.

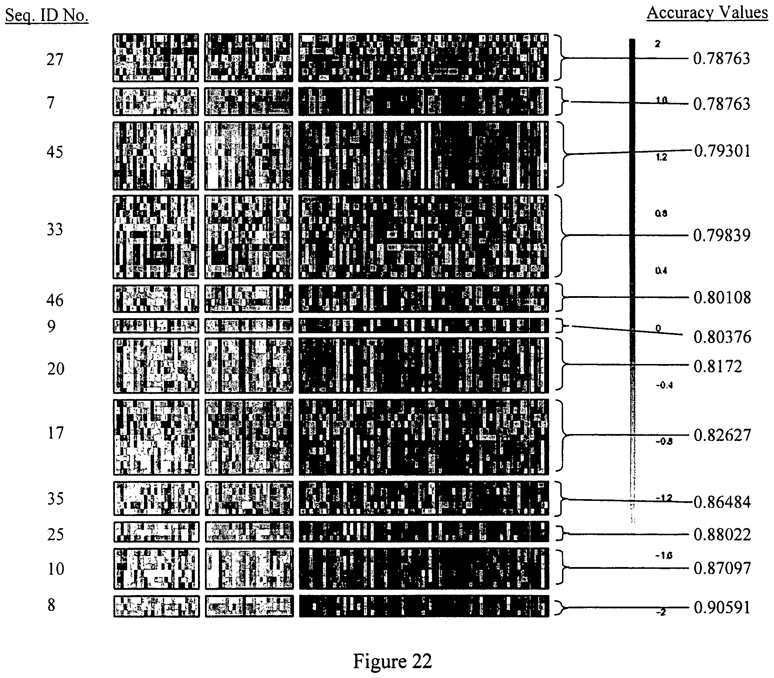

Figure 22 shows the accuracy of the top 12 performing markers.

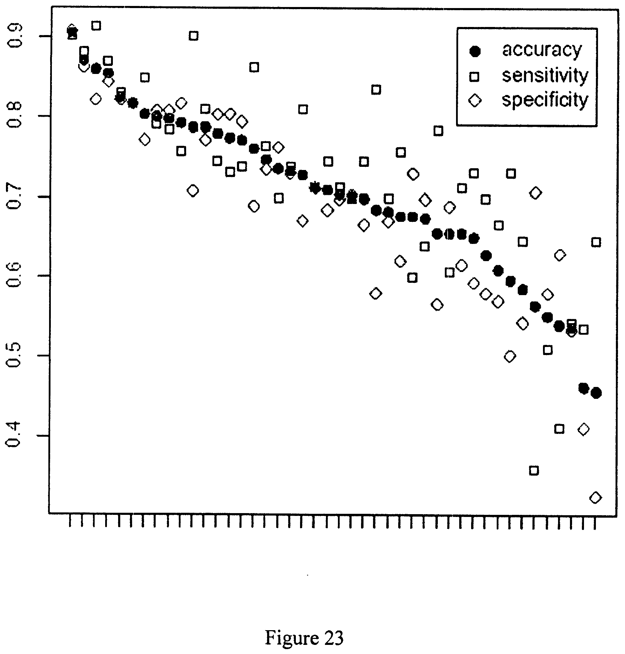

Figure 23 shows the accuracy of the genewise linear support vector machine cross validation for all genomic regions of each classification.

Inter alia, the genomic marker according to SEQ ID NO: 7 was evaluated as being significant.

### Colon Normal and Other Tissues vs. Colorectal cell proliferative Disorder

In this classification 124 colorectal cell proliferative disorder samples (consisting of colon polyps and colorectal carcinoma) were compared to a class consisting of 'colon normal' and 'other tissue' samples. The colon normal samples consisted of both healthy colon samples and colon tissue of inflammatory disorders, the 'other tissue' samples consisted of samples from non-colorectal carcinomas, peripheral blood lymphocytes and other normal tissues of non-colorectal origin.

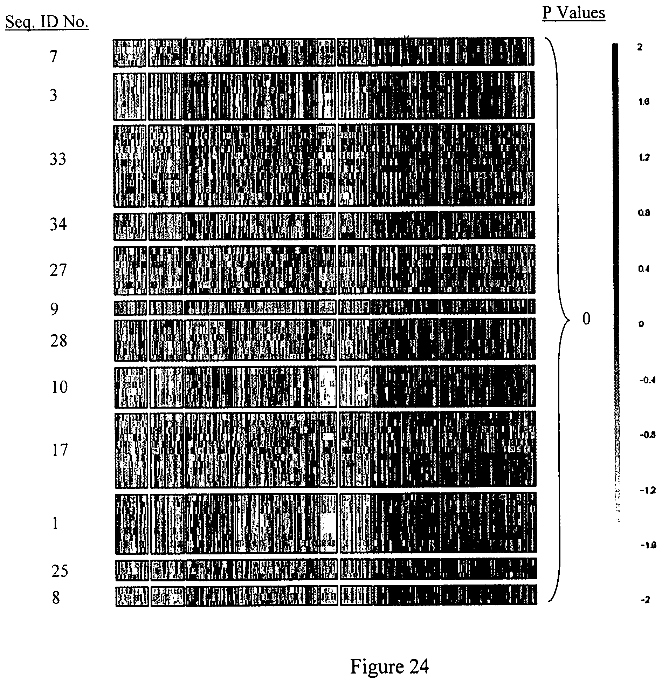

Figure 24 shows the multivariate test results of the top 12 performing markers using the conservative Bonferroni corrected LogReg test.

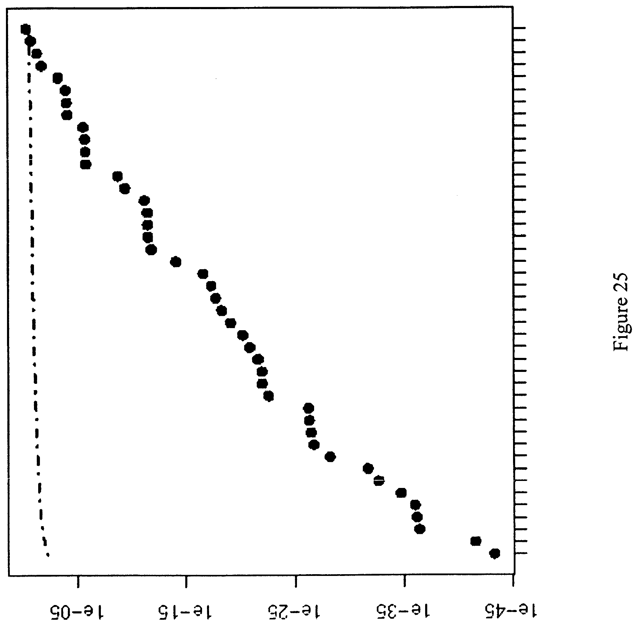

Figure 25 shows the uncorrected multivariate LogReg test results for each comparison. Each individual genomic region of interest is represented as a point, the dotted line represents the cut off point for the 25% false discovery rate.

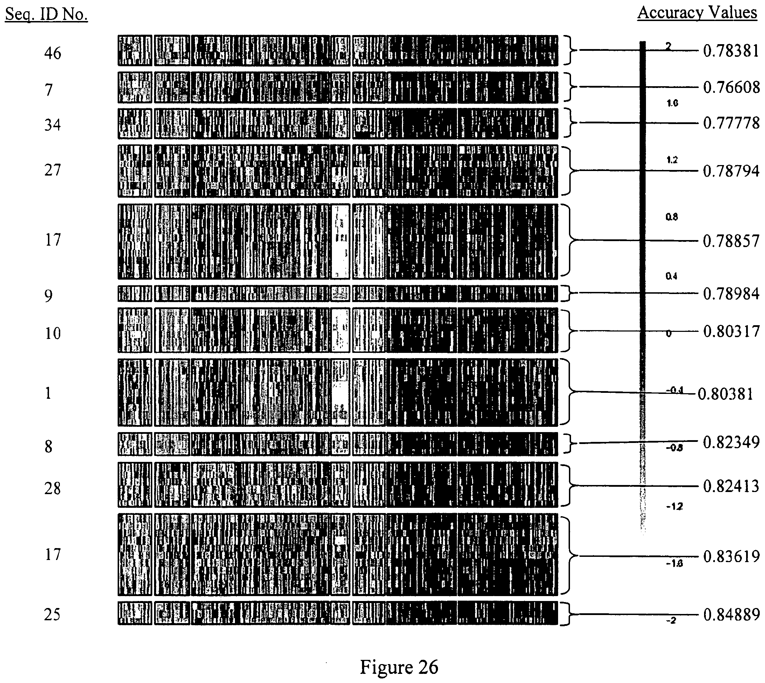

Figure 26 shows the accuracy of the top 12 performing markers.

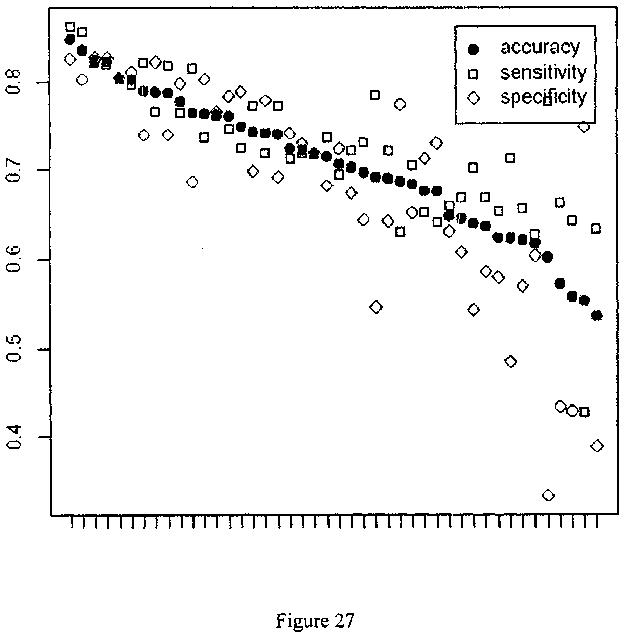

Figure 27 shows the accuracy of the genewise linear support vector machine cross validation for all genomic regions of each classification.

Inter alia, the genomic marker according to SEQ ID NO: 7 was evaluated as being significant.

### Other Tissue vs. Colon Tissue

The following comparison was carried out in order to identify markers capable of discerning elevated levels of free floating colon DNA, especially in bodily fluids as a marker of tumor progression. In this classification the 'colon tissue' class consisted of samples from colorectal carcinoma, c olon polyps, colon tissue of inflammatory disorders and healthy colon tissue. The 'other tissue' class consisted of samples from non-colorectal carcinomas, peripheral blood lymphocytes and other normal tissues of non-colorectal origin.

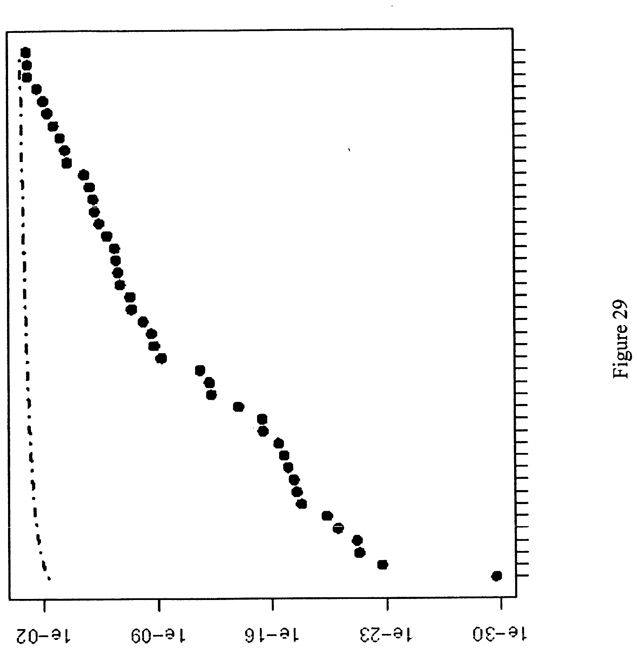

Figure 29 shows the uncorrected multivariate LogReg test results for each comparison. Each individual genomic region of interest is represented as a point, the dotted line represents the cut off point for the 25% false discovery rate.

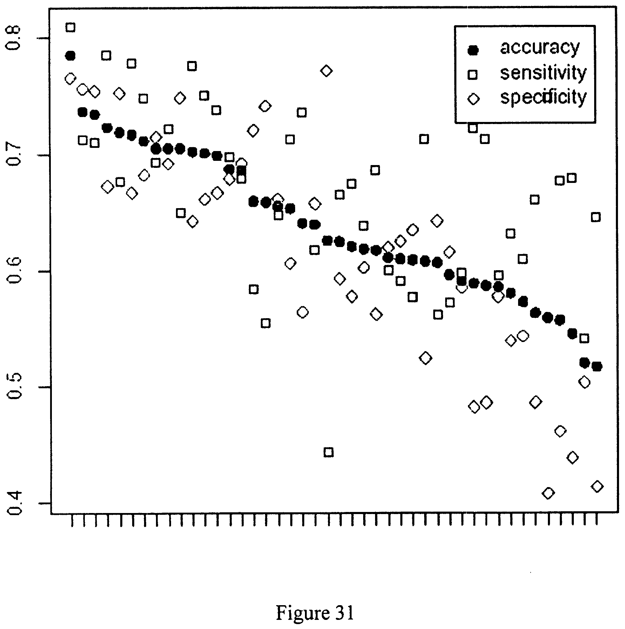

Figure 31 shows the accuracy of the genewise linear support vector machine cross validation for all genomic regions of each classification.

Inter alia, the genomic marker according to SEQ ID NO: 7 was evaluated as being significant.

### Normal Tissue vs. Cell Proliferative Disorder Tissue

In this classification the gene panel was assessed for its ability to accurately discriminate cell proliferative disorder samples from both colorectal carcinoma, colon polyps and non-colon origin cancers from 'normal tissues', namely healthy colon samples, colon tissue of inflammatory disorders, peripheral blood lymphocytes, other normal tissues of non-colorectal origin.
Figure 33 shows the uncorrected multivariate LogReg test results for each comparison. Each individual genomic region of interest is represented as a point, the dotted line represents the cut off point for the 25% false discovery rate.

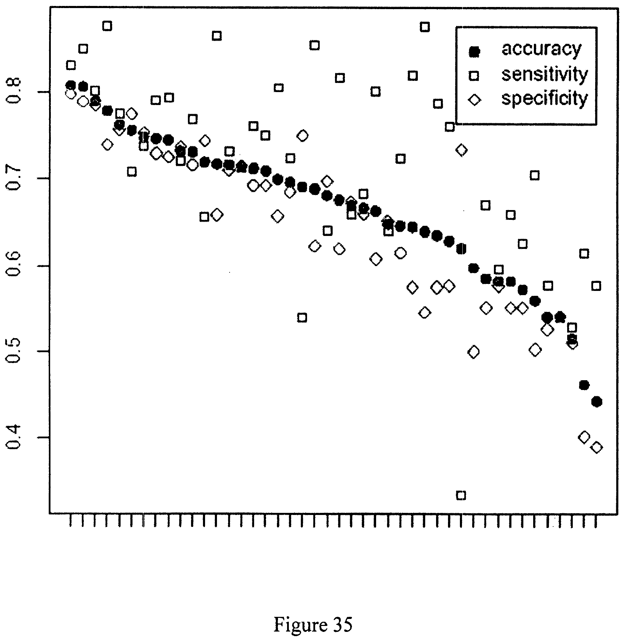

Figure 35 shows the accuracy of the genewise linear support vector machine cross validation for all genomic regions of each classification.

Inter alia, the genomic marker according to SEQ ID NO: 7 was evaluated as being significant.

### Example 14

### MSP analysis of the genes according to Table 6 (below).

In the following analysis the methylation status of a selection of the genes according to Table 3 were analysed by means of methylation specific amplification using the primers according to Table 6 (below).

The study was run on approximately 140 samples from colon, breast and liver carcinoma, normal tissue (peripheral blood lymphocytes, healthy colon and healthy tissue adjacent to colon carcinoma), colon polyps and other colon diseases. The positive class consisted of Colon Cancer and Colon polyps. The negative class was PBL (peripheral blood lymphocytes), Colon-Normal, Colon-NAT and Colon-Inflammatory. For AUC values in brackets (see table 7, below) Breast-Cancer and Liver-Cancer were also included in the negative class.

Genomic DNA was analyzed using the MSP technique after bisulfite conversion. Total genomic DNA of all samples was bisulfite treated converting unmethylated cytosines to uracil. Methylated cytosines remained conserved. Bisulfite treatment was performed with minor modifications according to the protocol described in Olek et al. (1996).

The sequence of interest was then amplified by means of methylation specific primers, the amplificate is then detected by means of methylation specific Taqman probes. //

**[Table 6 Oligonucleotides for MSP Taqman**

| 3 | Assay number |
|---|---|
| TUMOR NECROSIS FACTOR RECEPTOR SUPERFAMILY MEMBER 16 PRECURSOR | Gene/Genomic location |
| agggcgttttgttggcg | Forward primer sequence |
| atcggcgttttagcgtgcggg | Probe |
| ctacaacctaaacgacgcgct | Reverse primer sequence |
| Figure 74 | Amplification curve |

### Reaction Conditions for Taqman PCR program:

### Denaturation at 95°C for 10 minutes.

50 cycles: Denaturation at 95°C for 15 seconds.
   Annealing at 62°C for 1 minute. Except Assay15, which has an annealing temp of 60°C.

Amplification curves for each assay are shown in figures 72 to 86 (according to Table 6).

The technical performance of a series of alternative assays was validated by testing each of the following using sperm DNA as the template:

**Gene SEQ ID NO: 7 (HeavyMethyl™ assay)**

| **Primer :** | | | | |
|---|---|---|---|---|
| Tgagagagagagggttgaaa (SEQ ID NO: 14554) | | | | |
| Tctaaataacaaaatacctccatt (SEQ ID NO: 14555) | | | | |

| **probes:** | | | | |
|---|---|---|---|---|
| CGaccCGccaacCGac-fluo (SEQ ID NO: 14556) | | | | |
| LCred640-CGcCGaaaCGCGctc-p (SEQ ID NO: 14557) | | | | |

| **blocker:** | | | | |
|---|---|---|---|---|
| ccattaccaacacaacccaccaaccaa-p (SEQ ID NO: 14558) | | | | |
| | | | | |

| **LightCycler program:** | | | | |
|---|---|---|---|---|
| activation: | 95°C | 10min | | |
| | | | | |
| 55 cycles: | 95°C | 10 sec | (20°C/s) | |
| | **56°C** | 30 sec | (20°C/s) | detection |
| | 72°C | 10 sec | (20°C/s) | |
| | | | | |
| melting curve: | 95°C | 10sec | 20 | |
| | 40°C | 10sec | 20 | |
| | 70°C | 0sec | 0,1 | |
| | | | | |
| cooling: | 40°C | 5 sec | | |

### Results

Sensitivity and specificity were computed with SVM and 10 fold cross validation

**Table 7**

| Gene/Genomic location | AUC | Sensitivity | Specificity |
|---|---|---|---|
| TUMOR NECROSIS FACTOR RECEPTOR SUPERFAMILY MEMBER 16 PRECURSOR | 0.82 (0.82) | 0.74 | 0.90 |

### Example 15

### HeavyMethyl analysis.

In the following, methylation of the genes/genomic sequences inter alia according to Gene SEQ ID Nos: 7 was analysed in 15 colorectal cancer samples (referred to as 'colon' in Table 7), and eight peripheral blood lymphocyte samples referred to as 'PBL' in Table 7).

Total genomic DNA of all samples was bisulfite treated converting unmethylated cytosines to uracil. Methylated cytosines remained conserved. Bisulfite treatment was performed with minor modifications according to the protocol described in Olek et al. (1996).

The sequence of interest was then amplified by means of primers specific for bisulfite treated DNA and a blocking oligonucleotide (in order to suppress amplification of non-methylated DNA). The 3'OH terminus of the blocking oligonucleotide was "capped" by a phosphate group to prevent polymerase from extending the primer during PCR reaction. The amplificate is then detected by means of methylation specific LightCycler™ probes, wherein one probe was labelled with 5'red640 and had a capped 3'OH terminus and the other was labelled with 3'Fluorescein.
Genomic SEQ ID NO:7
Primers: ggtttgggatggttgt(SEQ ID NO: 14615) & tctaaataacaaaatacctccatt (SEQ ID NO: 14616)
Blocking oligonucleotides: ccattaccaacacaacccaccaaccaa (SEQ ID NO: 14617)
Probes: ggtcgcggtgagagagagag (Fluorescein) & (SEQ ID NO: 14618)
tgaaattagagcgcgtttcgg (red640) (SEQ ID NO: 14619)

The reactions were performed in a total volume of 20 µl using a LightCycler device (Roche Diagnostics). The real time PCR reaction mix contained 10 µl of template DNA, 2 µl of FastStart LightCycler reaction mix for hybridization probes (Roche Diagnostics, Penzberg),

**Table 7: Results of HeavyMethyl assays**

| Tissue | Crossing points | | Crossing points | | Calculated Concentratio n DNA undiluted (pg/10µl) |
|---|---|---|---|---|---|
| | Genomic SEQ ID | Genomic SEQ ID | Genomic SEQ ID NO. | Genomic SEQ ID NO. | |

| | NO. | NO. | (undiluted) | (diluted) | |
|---|---|---|---|---|---|
| Colon | 36,73 | 38,22 | 39,58 | | 5,855 |
| Colon | 33,1 | >51 | | | 386,9 |
| Colon | 39,8 | 42,84 | | | 57,5 |
| Colon | 39,74 | 38,75 | 32,54 | 33,89 | 482,6 |
| Colon | | >51 | | | 132,55 |
| Colon | | 38,82 | | | 29,955 |
| Colon | 32,08 | 33,9 | 33,43 | 35,43 | 816 |
| Colon | 37,03 | 36,91 | | | 284,35 |
| Colon | 38,07 | 46,54 | | | 0 |
| Colon | 35,22 | 37,81 | 36,8 | 37,03 | 298,35 |
| Colon | 33,66 | 36,19 | 33,23 | 34,01 | 1355 |
| Colon | 36,78 | 37,46 | 40,71 | | 879 |
| Colon | | 37,23 | | | 57,5 |
| Colon | | | | | 1669 |
| Colon | 34,88 | 34,71 | 34,21 | 34,49 | 880,5 |
| PBL | | | | | 716,5 |
| PBL | | | | | 796 |
| PBL | | | | | 584,5 |
| PBL | 38,55 | | | | 1813 |
| PBL | | | | | 5470 |
| PBL | | 49,04 | | | 761 |
| PBL | | | | | 625 |
| PBL | | 39,75 | | | 9835 |
| M 100pg | 36,34 | 37,29 | 34,95 | 34,51 | 583,5 |
| M 100pg | 36,06 | 37,86 | 34,64 | 34,09 | 428,3 |
| uMRoche 100ng | | 34,48 | | | |
| uMPhi 100ng | | | | | |
| water | | | | | |

Bold type figures indicate those classified as positive because the amplification curves were flat.

### Example 16

### Serum analysis of the genes according to SEQ ID No. 7.

The assays as described above in example 14 (MSP) & were then applied to a set of serum samples consisting 32 colorectal carcinoma serum samples, and compared to a set consisting of 54 normal serum samples, 11 serum samples from benign colon lesions and 6 serum samples from inflammatory colon disease. 130 ul of serum was used per assay and the results were quantitatively reported as positive or negative.

**Table 6**

| Marker or combination of markers | Sensitivity | Specificity |
|---|---|---|
| SEQ ID NO: 7 (MSP) | 15.6 | 98.3 |

### FURTHER TABLES

**TABLE 1.**

| ***No:*** | ***Gene*** | ***Oligo:*** |
|---|---|---|
| 99 | (SEQ ID NO: 7) | TTCGTCGGCGGTAGAG (SEQ ID NO: 770) |
| 100 | (SEQ ID NO: 7) | TAGAGTTTGTTGGTGG (SEQ ID NO: 771) |
| 101 | (SEQ ID NO: 7) | GATCGCGGGTACGTTT (SEQ ID NO: 772) |
| 102 | (SEQ ID NO: 7) | ATTGTGGGTATGTTTGT (SEQ ID NO: 773) |
| 103 | (SEQ ID NO: 7) | TTAACGTCGTTGGTTA (SEQ ID NO: 774) |
| 104 | (SEQ ID NO: 7) | TGATTAATGTTGTTGGT (SEQ ID NO: 775) |
| 105 | (SEQ ID NO:7) | TTCGCGCGAAGATTTA (SEQ ID NO: 776) |
| 106 | (SEQ ID NO: 7) | GTTTTTGTGTGAAGATT (SEQ ID NO: 777) |

**Table 3: Genes or genomic locus associated with genomic sequences according to SEQ ID NO .7**

| Genomic SEQ ID NO: | Bisulfite SEQ ID Nos: | Genbank Ref. ID No:/contig | Description |
|---|---|---|---|
| 7 | 316, 317, 432 & 433 | NM_002507 | TUMOR NECROSIS FACTOR RECEPTOR SUPERFAMILY MEMBER 16 PRECURSOR (LOW- AFFINITY NERVE GROWTH FACTOR RECEPTOR) (NGF RECEPTOR) (GP80-LNGFR) (P75 ICD) (LOW AFFINITY NEUROTROPHIN RECEPTOR P75NTR). NGFR |

## Claims

1. A method for detecting colon cell proliferative disorders in a subject comprising contacting genomic DNA isolated from a biological sample obtained from the subject with at least one reagent, or series of reagents that distinguishes between methylated and non-methylated CpG dinucleotides within at least one target region of the genomic DNA, wherein the target region comprises, or hybridizes under stringent conditions to at least 16 contiguous nucleotides of the gene or a sequence of NGFR and wherein said contiguous nucleotides comprise at least one CpG dinucleotide sequence, wherein hypermethylation indicates the presence of a colon cell proliferative disorder.

2. The method of claim 1 wherein the biological sample is blood plasma, blood serum, whole blood, isolated blood cells, cells isolated from the blood.

3. The method of claim 1, wherein at least one of colorectal carcinoma tissue, or colon adenomas is distinguished from at least one tissue selected from the group consisting of inflammatory colon tissue, normal colon tissue, non-colon healthy tissue, peripheral blood lymphocytes, and non-colon cancer tissue, and wherein the target region comprises, or hybridizes under stringent conditions to at least 16 contiguous nucleotides of the sequence SEQ ID NO: 7, and complements thereof.

4. A method for detectingcolon cell proliferative disorders in a subject comprising determining the expression level of the gene or sequence of NGFR, in a biological sample isolated from said subject,
wherein said expression level is determined by detecting the presence, absence or level of mRNA transcribed from said gene or sequence, or wherein said expression level is determined by detecting the presence, absence or level of a polypeptide encoded by said gene or sequence, or wherein said expression is determined by detecting the presence or absence of CpG methylation within said gene or sequence, wherein hypermethylation indicates the presence of a colon cell proliferative disorder.

5. A method according to any of claims 1 to 4, comprising:
a) extracting or otherwise isolating genomic DNA from a biological sample obtained from the subject
b) treating the genomic DNA of a), or a fragment thereof, with one or more reagents to convert cytosine bases that are unmethylated in the 5-position thereof to uracil;
c) contacting the treated genomic DNA, or the treated fragment thereof, with an amplification enzyme and at least two primers comprising, in each case a contiguous sequence of at least 9 nucleotides that is complementary to, or hybridizes under moderately stringent or stringent conditions to a sequence selected from the group consisting of SEQ ID NOs: 316, 317, 432, 433, and complements thereof, wherein the treated genomic DNA or the fragment thereof is either amplified to produce at least one amplificate, or is not amplified; and
d) determining, based on a presence or absence of, or on a property of said amplificate, the methylation state of at least one CpG dinucleotide of the sequence SEQ ID NO: 7, or an average, or a value reflecting an average methylation state of a plurality of CpG dinucleotides of the sequence of SEQ ID NO: 7.

6. The method of claim 5, wherein treating the genomic DNA, or the fragment thereof in b), comprises use of a reagent selected from the group consisting of bisulfite, hydrogen sulfite, disulfite, and combinations thereof.

7. The method of claim 6, further comprising in step d) the use of at least one nucleic acid molecule or peptide nucleic acid molecule comprising in each case a contiguous sequence at least 9 nucleotides in length that is complementary to, or hybridizes under moderately stringent or stringent conditions to a sequence selected from the group consisting of SEQ ID NOs: 316, 317, 432, 433, and complements thereof, wherein said nucleic acid molecule or peptide nucleic acid molecule suppresses amplification of the nucleic acid to which it is hybridized.

8. The method of claim 6, wherein determining in d) comprises hybridization of at least one nucleic acid molecule or peptide nucleic acid molecule in each case comprising a contiguous sequence at least 9 nucleotides in length that is complementary to, or hybridizes under moderately stringent or stringent conditions to a sequence selected from the group consisting of SEQ ID NOs: 316, 317, 432, 433, and complements thereof.

9. A method for detecting colon cell proliferative disorders in a subject, comprising:
a) providing a biological sample that has been obtained from a subject and contains subject genomic DNA;
b) extracting, or otherwise isolating the genomic DNA;
c) contacting the genomic DNA of b), or a fragment thereof, comprising at least 16, contiguous nucleotides of a sequence of SEQ ID NO: 7, and sequences that hybridize under stringent conditions thereto, with one or more methylation-sensitive restriction enzymes, wherein the genomic DNA is, with respect to each cleavage recognition motif thereof, either cleaved thereby to produce cleavage fragments, or not cleaved thereby; and
d) determining, based on a presence or absence of, or on property of at least one such cleavage fragment, the methylation state of at least one CpG dinucleotide of the sequence of SEQ ID NO: 7, or an average, or a value reflecting an average methylation state of a plurality of CpG dinucleotides of the sequence of SEQ ID NO: 7,
wherein hypermethylation indicates the presence of a colon cell proliferative disorder or allows distinguishing between or among colon cell proliferative disorders.

10. Use of a nucleic acid derived from SEQ ID NO: 7 in detecting colon cell proliferative disorders, wherein at least one unmethylated cytosine base of the genomic DNA sequence is converted to uracil.

11. The use of claim 10, wherein the nucleic acid comprises at least 16 contiguous nucleotides of a DNA sequence selected from the group consisting of SEQ ID NOs: 316, 317, 432, 433, and sequences complementary thereto.

12. The use of claim 10, wherein the nucleic acid comprises a sequence of at least 9 contiguous nucleotides that is complementary to, or hybridizes under moderately stringent or stringent conditions to a DNA sequence selected from the group consisting of SEQ ID NOs: 316, 317, 432, 433.

13. The use of claims 10 to 12, wherein the contiguous base sequence of the nucleic acid comprises at least one CpG, TpG or CpA dinucleotide sequence.

14. Use of kit for detecting colon cell proliferative disorders of a subject, the kit comprising:
- a methylation-sensitive restriction enzyme; and
- at least one nucleic acid molecule or peptide nucleic acid molecule, comprising a contiguous sequence of at least 16, nucleotides that is complementary to, or hybridizes under moderately stringent or stringent conditions to the sequence of SEQ ID NO: 7, and complements thereof.

15. Use of a kit useful for detecting colon cell proliferative disorders of a subject, the kit comprising:
- a bisulfite reagent; and
- at least one nucleic acid molecule or peptide nucleic acid molecule, comprising a contiguous sequence of at least 16, nucleotides that is complementary to, or hybridizes under moderately stringent or stringent conditions to the sequence selected from the group consisting of SEQ ID NOs: 316, 317, 432, 433, and complements thereof.

## Patentansprüche

1. Verfahren zur Detektion von Kolon-Zellproliferationsstörungen in einem Subjekt, umfassend das Inkontaktbringen genomischer DNA, welche aus einer von einem Subjekt erhaltenen biologischen Probe isoliert wurde, mit mindestens einem Reagenz oder einer Serie von Reagenzien, die zwischen methylierten und nicht-methylierten CpG Dinukleotiden innerhalb mindestens einer Targetregion der genomischen DNA unterscheiden, wobei die Targetregion mindestens 16 benachbarte Nukleotide des Gens oder einer NGFR Sequenz umfasst oder unter stringenten Bedingungen damit hybridisiert, und wobei besagte benachbarte Nukleotide mindestens eine CpG Dinukleotid Sequenz umfassen, wobei Hypermethylierung die Anwesenheit einer Kolon-Zellproliferationsstörung anzeigt.

2. Verfahren gemäß Anspruch 1, wobei die biologische Probe Blutplasma, Blutserum, Vollblut, isolierte Blutzellen, aus Blut isolierte Zellen sind.

3. Verfahren gemäß Anspruch 1, wobei mindestens eines aus Kolorektalkarzinom Gewebe oder Kolonadenomen von mindestens einem Gewebe ausgewählt aus der Gruppe bestehend aus entzündliches Kolongewebe, normales Kolongewebe, gesundes nicht-Kolon Gewebe, periphere Blutlymphozyten und nicht-Kolon Krebsgewebe unterschieden wird, und wobei die Targetregion mindestens 16 benachbarte Nukleotide der Sequenz der SEQ ID NO: 7 und Komplementen dieser umfasst oder unter stringenten Bedingungen damit hybridisiert.

4. Verfahren zur Detektion von Kolon-Zellproliferationsstörungen in einem Subjekt, welches die Bestimmung des Expressionslevels des Gens oder der NGFR Sequenz in einer von besagtem Subjekt isolierten biologischen Probe umfasst,
wobei besagtes Expressionslevel durch Detektion der Anwesenheit, Abwesenheit oder des Levels transkribierter mRNA von besagtem Gen oder Gensequenz bestimmt wird, oder wobei besagtes Expressionslevel durch Detektion der Anwesenheit, Abwesenheit oder des Levels des kodierten Polypeptids von besagtem Gen oder Gensequenz bestimmt wird, oder wobei besagte Expression durch Detektion der Anwesenheit oder Abwesenheit von CpG Methylierung innerhalb des besagten Gens oder Sequenz bestimmt wird, wobei Hypermehtylierung die Anwesenheit einer Kolon-Zellproliferationsstörung anzeigt.

5. Verfahren gemäß einem der Ansprüche 1-4, umfassend
(a) Extrahieren oder in anderer Weise Isolieren genomischer DNA aus einer biologischen vom Subjekt erhaltenen Probe;
(b) Behandeln der genomischen DNA aus Schritt (a), oder eines Fragmentes davon, mit einem oder mehreren Reagenzien, um unmethylierte Cytosinbasen in der 5' Position in Uracil umzuwandeln;
(c) Inkontaktbringen der behandelten genomischer DNA oder eines behandelten Fragments dieser, mit einem Amplifikationsenzym und mindestens zwei Primem, welche in jedem Fall eine benachbarte Sequenz von mindestens 9 Nukleotiden umfasst, welche komplementär zu ist oder unter moderat stringenten oder stringenten Bedingungen an eine Sequenz ausgewählt aus der Gruppe bestehend aus SEQ ID NO: 316, 317, 432, 433 und Komplementen dieser hybridisiert, wobei die behandelte genomische DNA oder das Fragment davon entweder amplifiziert wird, um mindestens ein Amplifikat zu bilden, oder nicht amplifiziert wird; und
(d) Bestimmen, des Methylierungsstatus von mindestens einem CpG Dinukleotid der SEQ ID NO: 7 oder eines Mittelwertes oder eines einen Mittleren Methylierungsstatus einer Vielzahl von CpG Dinukleotiden der Sequenz von SEQ ID NO: 7 reflektierenden Wertes, basierend auf der Anwesenheit oder Abwesenheit von, oder einer Eigenschaft des besagten Amplifikates,

6. Verfahren gemäß Anspruch 5, wobei Behandeln der genomischen DNA oder des Fragments davon in Schritt (b) die Verwendung eines Reagenz ausgewählt aus der Gruppe bestehend aus Bisulfit, Hydrogensulfit, Disulfit und Kombinationen dieser, umfasst.

7. Verfahren gemäß Anspruch 6, weiterhin umfassend in Schritt (d) die Verwendung von mindestens einem Nukleinsäuremolekül oder Peptid-Nukleinsäuremolekül, in jedem Fall eine benachbarte Sequenz von mindestens 9 Nukleotiden Länge umfassend, welche komplementär zu oder unter moderat stringenten oder stringenten Bedingungen mit einer Sequenz ausgewählt aus der Gruppe bestehend aus SEQ ID NO: 316, 317, 432, 433 und Komplementen dieser hybdrisiert, wobei besagtes Nukleinsäuremolekül oder Peptid-Nukleinsäuremolekül die Amplifikation der Nukleinsäure, mit der es hybridisiert, unterdrückt.

8. Verfahren gemäß Anspruch 6, wobei Bestimmen in Schritt (d) die Hybridisierung von mindestens einem Nukleinsäuremolekül oder Peptid-Nukleinsäuremolekül, umfasst, in jedem Fall eine benachbarte Sequenz von mindestens 9 Nukleotiden Länge umfassend, welche komplementär zu oder unter moderat stringenten oder stringenten Bedingungen mit einer Sequenz ausgewählt aus der Gruppe bestehend aus SEQ ID NO: 316, 317, 432, 433 und Komplementen dieser hybridisiert.

9. Verfahren zur Detektion von Kolon-Zellproliferationsstörugen in einem Subjekt, umfassend:
(a) Bereitstellen einer biologischen Probe, die von einem Subjekt erhalten worden ist und genomische DNA des Subjekts enthält;
(b) Extrahieren oder auf andere Weise Isolieren der genomischen DNA;
(c) Inkontaktbringen der genomischen DNA aus Schritt (b) oder eines Fragmentes davon, umfassend mindestens 16 benachbarte Nukleotide einer Sequenz der SEQ ID NO: 7, und Sequenzen, die unter stringenten Bedingungen damit hybridisieren, mit einer oder mehreren methylierungssensitiven Restriktionsenzymen, wobei die genomische DNA im Hinblick auf jedes Spalterkennungsmotiv, entweder dadurch gespalten wird, um Spaltfragmente zu produzieren oder nicht dadurch gespalten wird; und
(d) Bestimmen, des Methylierungsstatus von mindestens einem CpG Dinukleotid der Sequenz der SEQ ID NO: 7, oder eines Mittelwertes oder eines einen Mittleren Methylierungsstatus einer Vielzahl von CpG Dinukleotiden der Sequenz von SEQ ID NO: 7 reflektierenden Wertes, basierend auf der Anwesenheit oder Abwesenheit von oder auf einer Eigenschaft von mindestens einem solchen Spaltfragment,
wobei Hypermethylierung die Anwesenheit einer Kolon-Zellproliferationsstörung anzeigt oder zwischen oder unter Kolon-Zellproliferationsstörungen zu unterscheiden erlaubt

10. Verwendung einer von SEQ ID NO: 7 stammenden Nukleinsäure, um eine Kolon-Zellproliferationsstörung zu detektieren, wobei mindestens eine unmethylierte Cytosinbase der genomischen DNA Sequenz in Uracil umgewandelt ist.

11. Verwendung gemäß Anspruch 10, wobei die Nukleinsäure mindestens 16 benachbarte Nukleotide einer DNA Sequenz ausgewählt aus der Gruppe bestehend aus SEQ ID NO: 316, 317, 432, 433 und dazu komplementären Sequenzen umfasst.

12. Verwendung gemäß Anspruch 10, wobei die Nukleinsäure eine Sequenz von mindestens 9 benachbarten Nukleotiden, die komplementär zu ist, oder unter moderat stringenten oder stringenten Bedingungen mit einer DNA Sequenz ausgewählt aus der Gruppe bestehend aus SEQ ID NO: 316, 317, 432, 433 hybridisert.

13. Verwendung gemäß den Ansprüchen 10-12, wobei die benachbarte Basensequenz der Nukleinsäure mindestens eine CpG, TpG oder CpA Dinukleotid Sequenz umfasst.

14. Verwendung eines Kits zum Detektieren von Kolon-Zellproliferationsstörungen eines Subjekts, das Kit umfassend:
- ein methylierungssensitives Restriktionsenzym; und
- mindestens ein Nukleinsäuremolekül oder Peptid-Nukleinsäuremolekül, welches eine benachbarte Sequenz von mindestens 16 Nukleotiden umfasst, die komplementär zu ist oder unter moderat stringenten oder stringenten Bedingungen mit der Sequenz der SEQ ID NO: 7 und Komplementen dieser hybridisiert.

15. Verwendung eines Kits zum Detektieren von Kolon-Zellproliferationsstörungen eines Subjekts, das Kit umfassend:
- ein Bisulfit Reagenz; und
- mindestens ein Nukleinsäuremolekül oder Peptid-Nukleinsäuremolekül, welches eine benachbarte Sequenz von mindestens 16 Nukleotiden umfasst, die komplementär zu ist, oder unter moderat stringenten oder stringenten Bedingungen mit einer DNA Sequenz ausgewählt aus der Gruppe bestehend aus SEQ ID NO: 316, 317, 432, 433 und Komplementen dieser hybridisiert.

## Revendications

1. Procédé pour la détection des troubles de prolifération de cellules du côlon chez un sujet, comprenant la mise en contact d'ADN génomique isolé à partir d'un échantillon biologique obtenu à partir d'un sujet avec au moins un réactif, ou des séries de réactifs qui font la distinction entre dinucléotides CpG méthylés et non-méthylés dans au moins une région cible de l'ADN génomique, tandis que la région cible comprend, ou hybride dans des conditions stringentes à au moins 16 nucléotides contigus du gène ou une séquence de NGFR, et tandis que lesdits nucléotides contigus comprennent au moins une séquence de dinucléotides CpG, tandis qu'une hyperméthylation indique la présence d'un trouble de prolifération de cellules du côlon.

2. Procédé selon la revendication 1 dans lequel l'échantillon biologique est du plasma sanguin, du sérum sanguin, du sang total, des cellules sanguines isolées, des cellules isolées à partir du sang.

3. Procédé selon la revendication 1, dans lequel au moins l'un parmi tissu de carcinome colorectal, ou adénome du côlon est distingué d'au moins un tissu choisi dans le groupe consistant en tissu du côlon inflammatoire, tissu du côlon normal, tissu sain n'appartenant pas au côlon, lymphocytes du sang périphérique, et tissu cancéreux qui n'est pas du côlon, et où la région cible comprend, ou hybride dans des conditions stringentes à au moins 16 nucléotides contigus de la séquence SEQ ID NO: 7, et ses compléments.

4. Procédé pour la détection de troubles de prolifération de cellules du côlon chez un sujet comprenant la détermination du niveau d'expression du gène ou de la séquence de NGFR, dans un échantillon biologique isolé à partir dudit sujet,
dans lequel ledit niveau d'expression est déterminé par détection de la présence, l'absence ou le niveau d'ARNm transcrit à partir dudit gène ou de ladite séquence, ou dans lequel ledit niveau d'expression est déterminé par détection de la présence, l'absence ou le niveau d'un polypeptide codé par ledit gène ou ladite séquence, ou dans lequel ladite expression est déterminée par détection de la présence ou l'absence d'une méthylation de CpG dans ledit gène ou ladite séquence, tandis qu'une hyperméthylation indique la présence d'un trouble de prolifération de cellules du côlon.

5. Procédé selon l'une quelconque des revendications 1 à 4, comprenant:
a) l'extraction ou l'isolement d'une autre manière d'ADN génomique provenant d'un échantillon biologique obtenu à partir du sujet;
b) le traitement de l'ADN génomique de a), ou d'un fragment de celui-ci, avec un ou plusieurs réactifs pour convertir les bases cytosine qui sont non-méthylées sur leur position 5 en uracile;
c) la mise en contact de l'ADN génomique traité, ou du fragment traité de celui-ci, avec une enzyme d'amplification et au moins deux amorces comprenant, dans chaque cas une séquence contiguë d'au moins 9 nucléotides qui est complémentaire de, ou qui hybride dans des conditions modérément stringentes ou stringentes avec une séquence choisie dans le groupe consistant en SEQ ID NOs: 316, 317, 432, 433, et leurs compléments, tandis que l'ADN génomique traité ou son fragment soit est amplifié pour donner au moins un produit d'amplification, soit n'est pas amplifié; et
d) la détermination, fondée sur une présence ou absence, ou sur une propriété dudit produit d'amplification, de l'état de méthylation d'au moins un dinucléotide CpG de la séquence SEQ ID NO: 7, ou d'une moyenne, ou d'une valeur reflétant un état de méthylation moyen d'une pluralité de dinucléotides CpG de la séquence selon SEQ ID NO: 7.

6. Procédé selon la revendication 5, dans lequel le traitement de l'ADN génomique, ou du fragment de celui-ci selon b), comprend l'utilisation d'un réactif choisi dans le groupe consistant en bisulfite, hydrogéno sulfite, disulfite, et des combinaisons de ceux-ci.

7. Procédé selon la revendication 6, comprenant en outre dans l'étape d) l'utilisation d'au moins une molécule d'acide nucléique ou une molécule d'acide nucléique peptidique comprenant dans chaque cas une séquence contiguë d'au moins 9 nucléotides de long qui est complémentaire de, ou hybride dans des conditions modérément stringentes ou stringentes à une séquence choisie dans le groupe consistant en SEQ ID NOs: 316, 317, 432, 433, et leurs compléments, tandis que ladite molécule d'acide nucléique ou ladite molécule d'acide nucléique peptidique supprime l'amplification de l'acide nucléique auquel elle est hybridée.

8. Procédé selon la revendication 6, dans lequel la détermination sous d) comprend l'hybridation d'au moins une molécule d'acide nucléique ou d'au moins une molécule d'acide nucléique peptidique comprenant dans chaque cas une séquence contiguë ayant au moins 9 nucléotides de long, qui est complémentaire de, ou hybride dans des conditions modérément stringentes ou stringentes à une séquence choisie dans le groupe consistant en SEQ ID NOs: 316, 317, 432, 433, et leurs compléments.

9. Procédé pour la détection de troubles de prolifération de cellules du côlon, comprenant:
a) la fourniture d'un échantillon biologique qui a été obtenu à partir d'un sujet et contient de l'ADN génomique du sujet;
b) l'extraction, ou l'isolement d'une autre manière de l'ADN génomique;
c) la mise en contact de l'ADN génomique de b) ou d'un fragment de celui-ci, comprenant au moins 16 nucléotides contigus d'une séquence selon SEQ ID NO: 7, et des séquences qui hybrident dans des conditions stringentes, avec une ou plusieurs enzymes de restriction sensibles à la méthylation, tandis que l'ADN génomique est, en ce qui concerne chacun de ses motifs de reconnaissance de clivage, soit clivé ainsi pour produire des fragments de clivage, soit non clivé de la sorte; et
d) la détermination, fondée sur une présence ou absence, ou sur une propriété d'au moins l'un d'un tel fragment de clivage, de l'état de méthylation d'au moins un dinucléotide CpG de la séquence SEQ ID NO: 7, ou d'une moyenne, ou d'une valeur reflétant un état de methylation moyen d'une pluralité de dinucléotides CpG de la séquence selon SEQ ID NO: 7,
tandis que l'hyperméthylation indique la présence d'un trouble proliférant de cellules du côlon ou permet de faire la distinction entre ou parmi les troubles de prolifération de cellules du côlon.

10. Utilisation d'un acide nucléique dérivé de SEQ ID NO: 7 dans la détection de troubles de prolifération de cellules du côlon, tandis qu'au moins une base cytosine non-méthylée de la séquence d'ADN génomique est convertie en uracile.

11. Utilisation selon la revendication 10, dans laquelle l'acide nucléique comprend au moins 16 nucléotides contigus d'une séquence d'ADN choisie dans le groupe consistant en SEQ ID NOs: 316, 317, 432, 433, et les séquences qui leur sont complémentaires.

12. Utilisation selon la revendication 10, dans laquelle l'acide nucléique comprend une séquence d'au moins 9 nucléotides contigus qui est complémentaire de, ou hybride dans des conditions modérément stringentes ou stringentes avec une séquence d'ADN choisie dans le groupe consistant en SEQ ID NOs: 316, 317, 432, 433.

13. Utilisation selon les revendications 10 à 12, dans laquelle la séquence de bases contiguës de l'acide nucléique comprend au moins une séquence dinucléotidique CpG, TpG ou CpA.

14. Utilisation d'un kit pour la détection de troubles de prolifération de cellules du côlon d'un sujet, le kit comprenant:
- une enzyme de restriction sensible à la méthylation; et
- au moins une molécule d'acide nucléique ou molécule d'acide nucléique peptidique, comprenant une séquence contiguë d'au moins 16 nucléotides qui est complémentaire de, ou hybride dans des conditions modérément stringentes ou stringentes avec la séquence selon SEQ ID NO: 7, et ses compléments.

15. Utilisation d'un kit utile pour la détection de troubles de prolifération de cellules du côlon d'un sujet, le kit comprenant:
- un réactif au bisulfite; et
- au moins une molécule d'acide nucléique ou molécule d'acide nucléique peptidique, comprenant une séquence contiguë d'au moins 16 nucléotides qui est complémentaire de, ou hybride dans des conditions modérément stringentes ou stringentes avec la séquence choisie dans le groupe consistant en SEQ ID NOs: 316, 317, 432, 433, et leurs compléments.
